# EUROPÄISCHE PATENTSCHRIFT

(11) **EP 0 915 837 B1**
(45) Veröffentlichungstag und Bekanntmachung des Hinweises auf die Patenterteilung: **03.07.2002**
(21) Anmeldenummer: 97930516.6
(22) Anmeldetag: 09.07.1997
(51) Int. Cl.: C07C 251/66, C07C 251/62, C07C 251/38, C07C 327/44, C07D 307/81, A01N 37/50, A01N 43/00

(54) **GLYOXYLSÄURE-DERIVATE**
GLYOXYLIC ACID DERIVATIVES
DERIVES D'ACIDE GLYOXYLIQUE

(30) Priorität: 22.07.1996 DE 19629466; 22.07.1996 DE 19629463; 22.07.1996 DE 19629464; 22.07.1996 DE 19629465
(43) Veröffentlichungstag der Anmeldung: 19.05.1999
(73) Patentinhaber: BAYER AG, 51368 Leverkusen (DE)
(72) Erfinder: SEITZ, Thomas, D-40764 Langenfeld (DE); STENZEL, Klaus, D-40595 Düsseldorf (DE); WACHENDORFF-NEUMANN, Ulrike, D-56566 Neuwied (DE)
(86) Internationale Anmeldenummer: EP9703644
(87) Internationale Veröffentlichungsnummer: WO9803474

(56) Entgegenhaltungen:
- EP-A- 0 658 549
- WO-A-94/26700
- WO-A-96/23763

## Beschreibung

Die Erfindung betrifft neue Glyoxylsäurederivate, ein Verfahren zu ihrer Herstellung und ihre Verwendung als Schädlingsbekämpfungsmittel.

In WO 94/26700 werden strukturell ähnliche o-Benzyloximether-Derivate und ihre-Verwendungals Pestizide beschrieben.

In EP-A1- 0 658 541 werden strukturell ähnliche Pyrimidinderivate und ihre Verwendung als Herbizide beschrieben.

Es wurden neue Verbindungen der allgemeinen Formel gefunden, 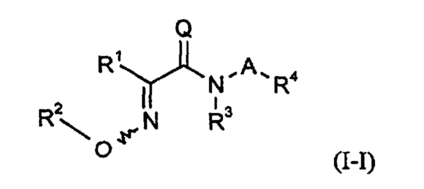 in welcher
- A: für eine Einfachbindung oder für gegebenenfalls einfach oder mehrfach, gleich oder verschieden substituiertes Alkylen mit 1 bis 6 Kohlenstoffatomen steht, wobei die möglichen Substituenten aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen, substituiertes Aryl oder Heterocyclyl,
- Q: für Sauerstoff oder Schwefel steht,
- R¹: für jeweils gegebenenfalls einfach oder mehrfach, gleich oder verschieden substituiertes Aryl, Cycloalkyl oder Cycloalkenyl mit 3 bis 7 Kohlenstoffatomen, oder Heterocyclyl mit 3 bis 12 Ringgliedern steht, wobei die möglichen Substituenten aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 8 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 8 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
substituiertes Aryl, Aryloxy, Arylthio, Arylalkyl, Arylalkyloxy, Arylalkylthio, Heterocyclyl, Heterocyclyloxy, Heterocyclylthio, Heterocyclylalkyl, Heterocyclylalkyloxy oder Heterocyclylalkylthio.
- R²: für eine der folgenden Gruppierungen steht: 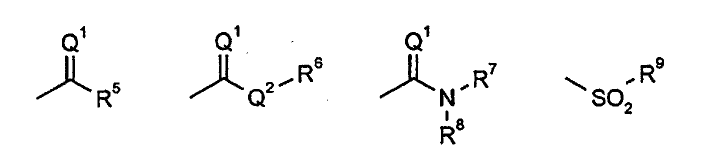 worin
Q¹ und Q² unabhängig voneinander für Sauerstoff oder Schwefel stehen,
R⁵ für Wasserstoff oder Alkyl mit 1 bis 6 Kohlenstoffatomen oder für jeweils gegebenenfalls im Arylteil durch Halogen, Cyano, Nitro, Alkyl oder Alkoxy mit jeweils 1 bis 4 Kohlenstoffatomen substituiertes Aryl oder Arylalkyl mit 1 bis 4 Kohlenstoffatomen im Alkylteil steht,
R⁶ für Alkyl mit 1 bis 6 Kohlenstoffatomen oder für jeweils gegebenenfalls im Arylteil durch Halogen, Cyano, Nitro, Alkyl oder Alkoxy mit jeweils 1 bis 4 Kohlenstoffatomen substituiertes Aryl oder Arylalkyl mit 1 bis 4 Kohlenstoffatomen im Alkylteil steht,
R⁷ für Wasserstoff, Alkyl mit 1 bis 6 Kohlenstoffatomen oder für jeweils gegebenenfalls im Arylteil durch Halogen, Cyano, Nitro, Alkyl oder Alkoxy mit jeweils 1 bis 4 Kohlenstoffatomen substituiertes Aryl oder Arylalkyl mit 1 bis 4 Kohlenstoffatomen im Alkylteil steht,
R⁸ für Alkyl mit 1 bis 6 Kohlenstoffatomen oder für jeweils gegebenenfalls im Arylteil durch Halogen, Cyano, Nitro, Alkyl oder Alkoxy mit jeweils 1 bis 4 Kohlenstoffatomen substituiertes Aryl oder Arylalkyl mit 1 bis 4 Kohlenstoffatomen im Alkylteil steht, oder
R⁷ und R⁸ gemeinsam mit dem Stickstoffatom, an das sie gebunden sind, für einen gegebenenfalls durch Methyl substituierten, heterocyclischen Ring stehen,
R⁹ für Alkyl mit 1 bis 6 Kohlenstoffatomen, Dialkylamino mit jeweils 1 bis 4 Kohlenstoffatomen in den einzelnen Alkylteilen, über Stickstoff gebundenes Heterocyclyl mit 3 bis 7 Ringgliedern oder für jeweils gegebenenfalls im Arylteil durch Halogen, Cyano, Nitro, Alkyl oder Alkoxy mit jeweils 1 bis 4 Kohlenstoffatomen substituiertes Aryl oder Arylalkyl mit 1 bis 4 Kohlenstoffatomen im Alkylteil steht,
- R³: für Wasserstoff oder Alkyl mit 1 bis 4 Kohlenstoffatomen steht,
- R⁴: für jeweils gegebenenfalls einfach oder mehrfach, gleich oder verschieden substituiertes Aryl, Cycloalkyl oder Cycloalkenyl mit 3 bis 8 Kohlenstoffatomen, oder Heterocyclyl mit 3 bis 12 Ringgliedern steht, wobei die möglichen Substituenten aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
substituiertes Aryl, Aryloxy, Arylthio, Arylalkyl, Arylalkyloxy, Arylalkylthio, Heterocyclyl, Heterocyclyloxy, Heterocyclylthio, Heterocyclylalkyl, Heterocyclylalkyloxy oder Heterocyclylalkylthio.

Die Erfindung betrifft insbesondere Verbindungen der Formel (I-I), in welcher
- A: für eine Einfachbindung oder für jeweils gegebenenfalls einfach oder mehrfach, gleich oder verschieden durch Fluor, Chlor, Cyano oder Methoxy substituiertes Methylen, 1,1-Ethylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methyl-propylen) steht,
- Q: für Sauerstoff oder Schwefel steht,
- R¹: für jeweils gegebenenfalls einfach bis dreifach substituiertes Cyclopropyl, Cyclobutyl, Cyclopentyl oder Cyclohexyl, Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinoethyl oder Ethoximinoethyl;
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy;
Cyclopropyl, Cyclobutyl, Cyclopentyl oder Cyclohexyl;
gegebenenfalls durch die obengenannten Substituenten substituiertes Phenyl, Benzyl, Phenoxy, Benzyloxy,
- R²: für eine der folgenden Gruppierungen steht: 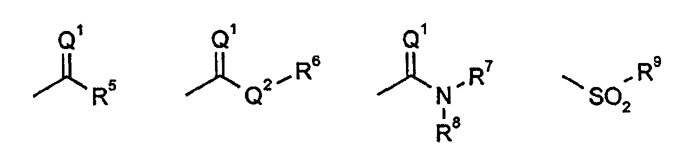 worin
Q¹ und Q² unabhängig voneinander für Sauerstoff oder Schwefel stehen,
R⁵ für Wasserstoff, Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁶ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁷ für Wasserstoff, Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁸ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht, oder
R⁷ und R⁸ gemeinsam mit dem Stickstoffatom, an das sie gebunden sind, für einen gegebenenfalls durch Methyl substituierten, heterocyclischen Ring stehen,
R⁹ für Methyl, Ethyl, n- oder i-Propyl, n-, i-, s- oder t-Butyl, Dimethylamino, Diethylamino, Pyrrolidin-1-yl, Morpholin-N-yl, Piperidin-1-yl, oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
- R³: für Wasserstoff oder für Methyl oder Ethyl steht,
- R⁴: für jeweils gegebenenfalls einfach bis dreifach substituiertes Cyclopropyl, Cyclobutyl, Cyclopentyl oder Cyclohexyl, Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl.

Besonders bevorzugt sind Verbindungen der Formel (I-I), in welcher
- A: für eine Einfachbindung oder für gegebenenfalls einfach oder mehrfach, gleich oder verschieden durch Fluor, Chlor, Cyano, Methoxy substituiertes Methylen, 1,1-Ethylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methyl-propylen) steht,
- Q: für Sauerstoff oder Schwefel steht,
- R¹: für jeweils gegebenenfalls einfach bis sechsfach substituiertes Cyclobutyl, Cyclopentyl oder Cyclohexyl, wobei als Substituenten die unten genannten bevorzugt sind;
für jeweils gegebenenfalls einfach bis dreifach substituiertes Phenyl, Naphthyl, Furyl, Benzofuranyl, Thienyl, Benzothienyl, Pyridyl, Chinolyl, Tetrahydrofuryl oder Perhydropyranyl steht, wobei die möglichen Substituenten aus der nachstehenden Aufzählung ausgewählt sind:
Fluor, Chlor, Brom, Cyano, Nitro, Methyl, Ethyl, n- oder i-Propyl, n-, i-, s-oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Acetyl, Propionyl, Acetyloxy, Methoxycarbonyl, Ethoxycarbonyl, Methylsulfonyloxy, Ethylsulfonyloxy,
Hydroximinomethyl, Hydroximinoethyl, Methoximinoethyl oder Ethoximinoethyl;
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy; Cyclopropyl, Cyclopentyl, Cyclohexyl; gegebenenfalls durch die oben genannten Substituenten substituierter Phenyl, Phenoxy, Benzyloxy;
- R²: für eine der folgenden Gruppierungen steht: 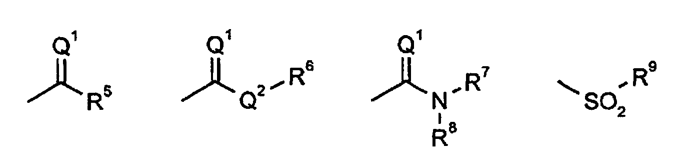 worin
Q¹ und Q² unabhängig voneinander für Sauerstoff oder Schwefel stehen,
R⁵ für Wasserstoff, Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁶ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁷ für Wasserstoff, Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁸ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht, oder
R⁷ und R⁸ gemeinsam mit dem Stickstoffatom, an das sie gebunden sind, für einen gegebenenfalls durch Methyl substituierten, heterocyclischen Ring stehen,
R⁹ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
- R³: für Wasserstoff oder für Methyl steht,
- R⁴: für jeweils gegebenenfalls einfach bis sechsfach substituiertes Cyclopropyl, Cyclobutyl, Cyclopentyl oder Cyclohexyl steht, wobei als Substituenten die untengenannten bevorzugt sind;
für jeweils gegebenenfalls einfach bis dreifach substituiertes Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinoethyl oder Ethoximinoethyl, jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl.

Eine ganz besonders bevorzugte Gruppe erfindungsgemäßer Verbindungen sind diejenigen Verbindungen der Formel (I-I), in welcher
- A: für eine Einfachbindung oder für Methylen, 1,1-Ethylen, 1,2-Ethylen, 1,1-Propylen, 1,2-Propylen, 1,3-Propylen oder 2,2-Propylen steht,
- Q: für Sauerstoff steht,
- R¹: für gegebenenfalls durch Brom, Chlor, Fluor, Nitro, Methylsulfonyl, Phenyl, Phenyloxy, Benzyloxy, Cyclopropyl, Cyclohexyl, Methyl, Ethyl, n- oder i-Propyl, n-, i-, s- oder t-Butyl, n-Pentyl, n-Hexyl, n-Heptyl, Methoxy, Ethoxy, Trifluormethyl, Trifluormethoxy und/oder Methylthio einfach oder zweifach substituiertes Phenyl, Thienyl oder Furanyl steht oder für jeweils gegebenenfalls durch Fluor substituiertes 3,4-Methylen- und Ethylendioxo, Propan-1,3-diyl und Butan-1,4-diyl substituiertes Phenyl steht oder für Naphthyl, Benzofuranyl oder Benzothienyl steht,
- R²: für eine der folgenden Gruppierungen steht: 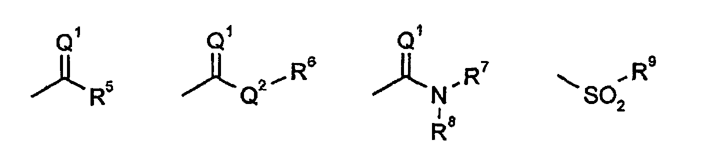 worin
Q¹ und Q² für Sauerstoff stehen,
R⁵ für Wasserstoff, Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁶ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁷ für Wasserstoff, Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁸ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht, oder
R⁷ und R⁸ gemeinsam mit dem Stickstoffatom, an das sie gebunden sind, für einen gegebenenfalls durch Methyl substituierten, heterocyclischen Ring stehen,
R⁹ für Methyl, Ethyl, n- oder i-Propyl, n-, i-, s- oder t-Butyl, Dimethylamino, Diethylamino, Pyrrolidin-1-yl, Morpholin-N-yl, Piperidin-1-yl, oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
- R³: für Wasserstoff oder Methyl steht,
- R⁴: für Cyclohexyl oder gegebenenfalls einfach bis dreifach substituiertes Phenyl, Thienyl, Furyl, Benzofuryl, Benzothienyl, Pyridyl, Pyrimidinyl, Naphthyl, Chinolyl steht, wobei die möglichen Substituenten aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy steht.

Von ganz besonderem Interesse sind Verbindungen der Formel (I-I), in welcher der Rest R¹ für unsubstituiertes oder in 3- und/oder 4-Stellung substituiertes Phenyl, oder für unsubstituiertes oder in 4- und/oder 5-Stellung substituiertes Furanyl oder Thienyl steht, wobei als Substituenten die oben genannten und insbesondere Chlor, Brom, Fluor, Nitro, Methylsulfonyl, Phenyl, Phenoxy, Benzyloxy, Cyclopropyl, Cyclohexyl, Methyl, Ethyl, n-, i-Propyl, n-, i-, s-, t-Butyl, n-Pentyl, n-Hexyl, n Heptyl, Methoxy, Ethoxy, Methylthio, Trifluormethyl und Trifluormethoxy ausgewählt sind oder jeweils gegebenenfalls durch Fluor substituiertes 3,4-Methylen- und Ethylendioxo, Propan-1,3-diyl und Butan-1,4-diyl substituiertes Phenyl oder in 2-Stellung substituiertes Naphthyl, Benzofuranyl oder Benzothienyl bedeuten.

Insbesondere sind auch Verbindungen der Formel (I-I) bevorzugt, in denen
- R³: für Wasserstoff steht.

Insbesondere sind auch Verbindungen der Formel (I-I) bevorzugt, in denen R⁴ für in 3- und 4-Stellung durch Methoxy substituiertes Phenyl steht.

In einer weiterhin besonders bevorzugten Gruppe von Verbindungen steht A für -CH₂-CH₂-.

Die in den jeweiligen Kombinationen bzw. bevorzugten Kombinationen von Resten im einzelnen für diese Reste angegebenen Restedefinitionen werden unabhängig von der jeweilig angegebenen Kombination, beliebig auch durch Restedefinitionen anderer Vorzugsbereiche ersetzt.

Die oben aufgeführten allgemeinen oder in Vorzugsbereichen angegebenen Restedefinitionen gelten sowohl für die Endprodukte der Formel (I-I) als auch entsprechend für die jeweils zur Herstellung benötigten Ausgangsstoffe bzw. Zwischenprodukte.

Bevorzugte Einzelverbindungen können den folgenden Tabellen entnommen werden:

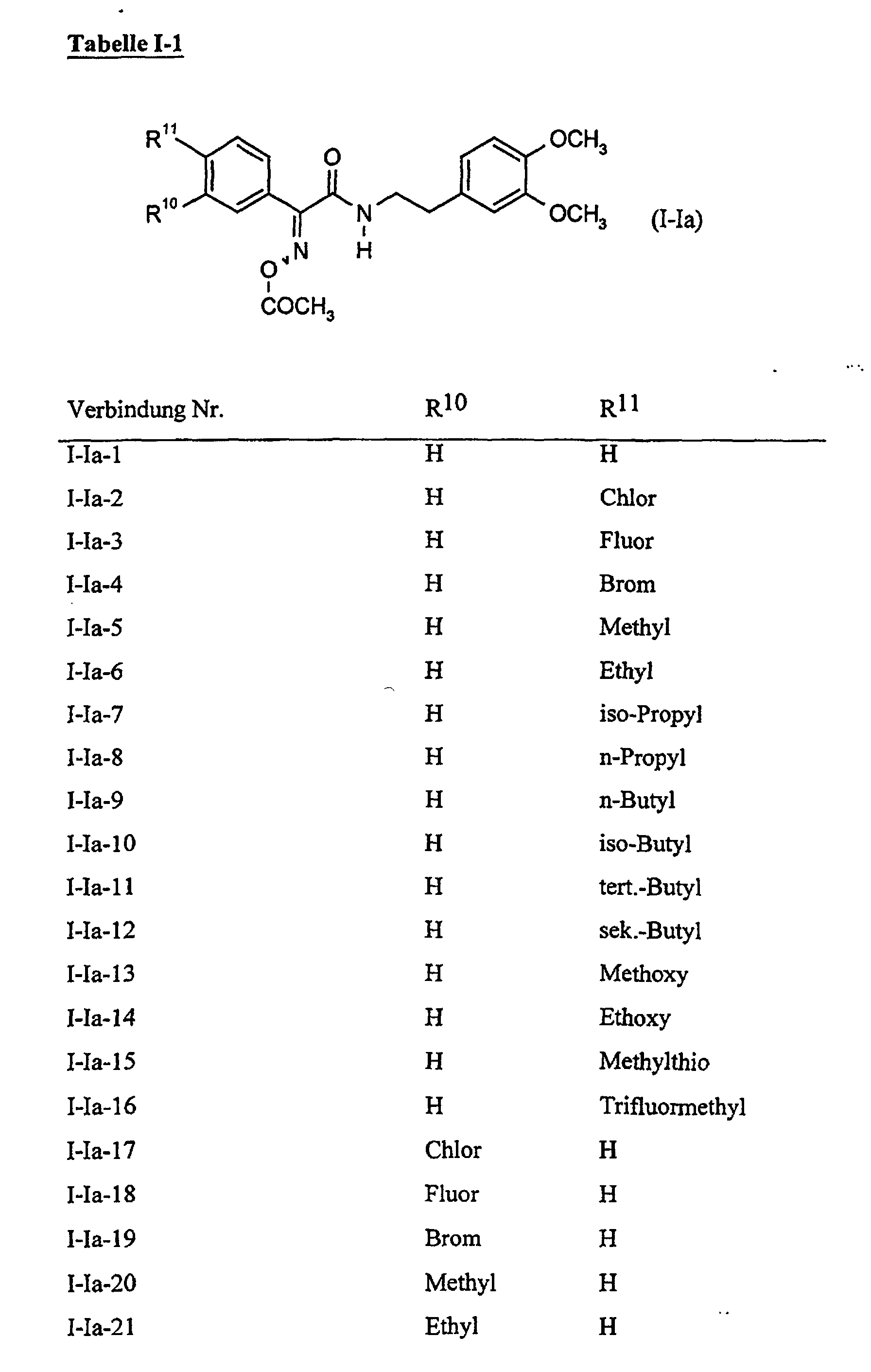

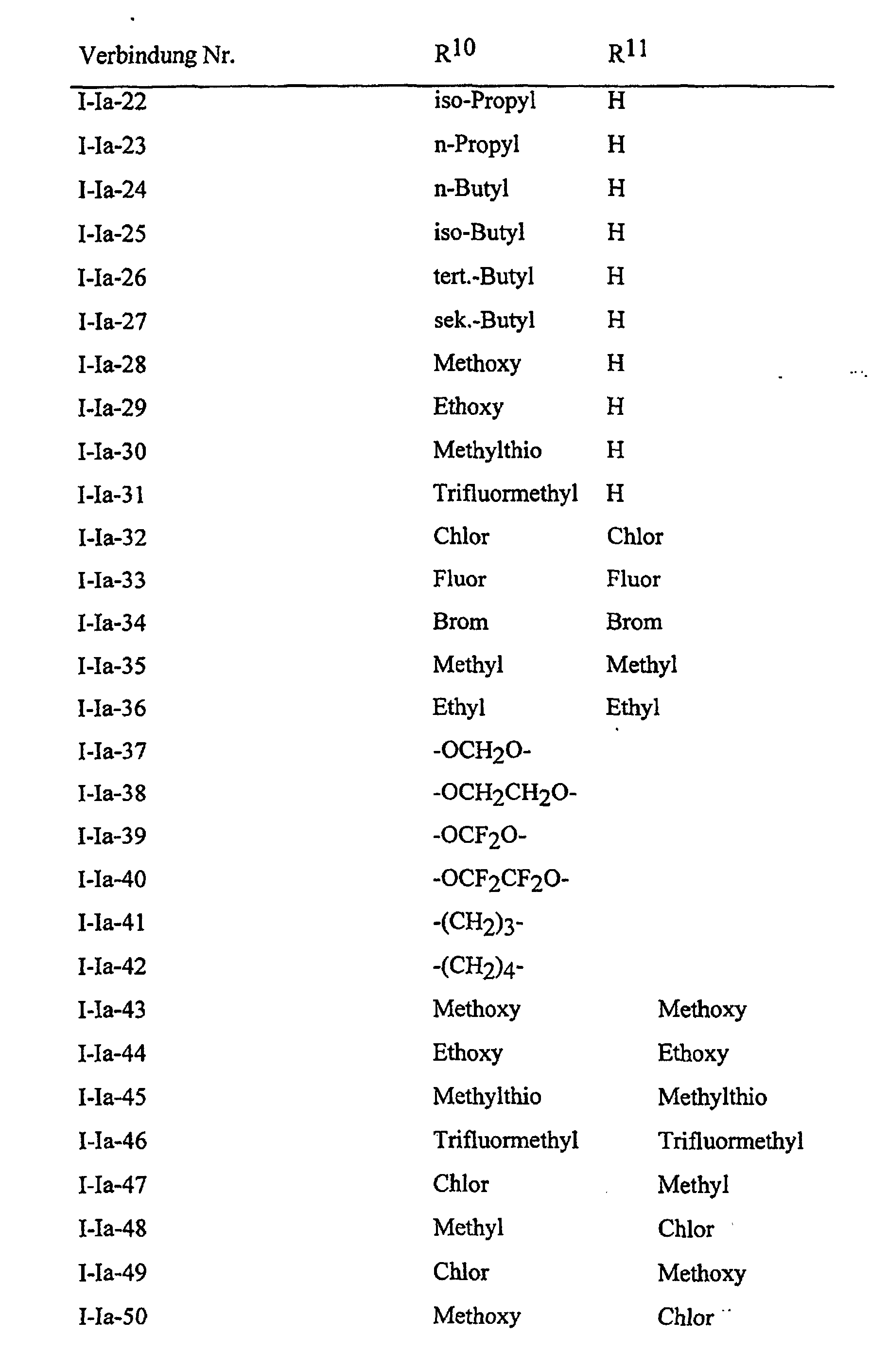

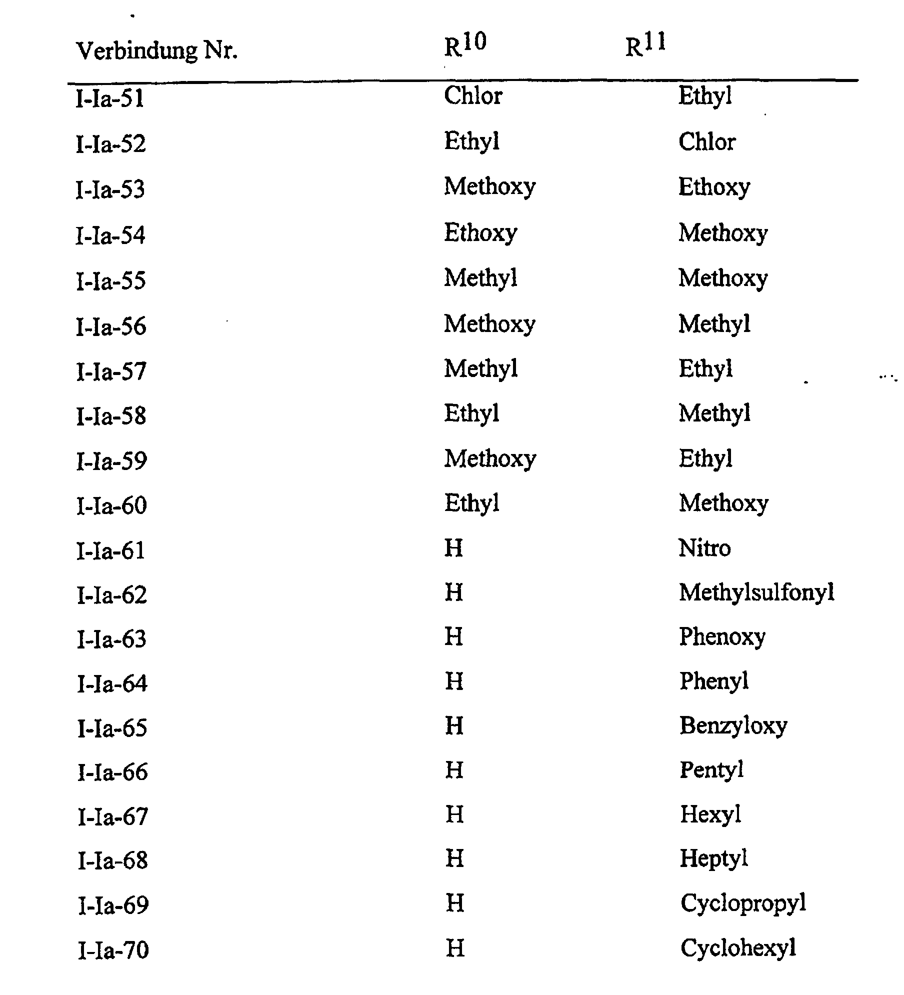

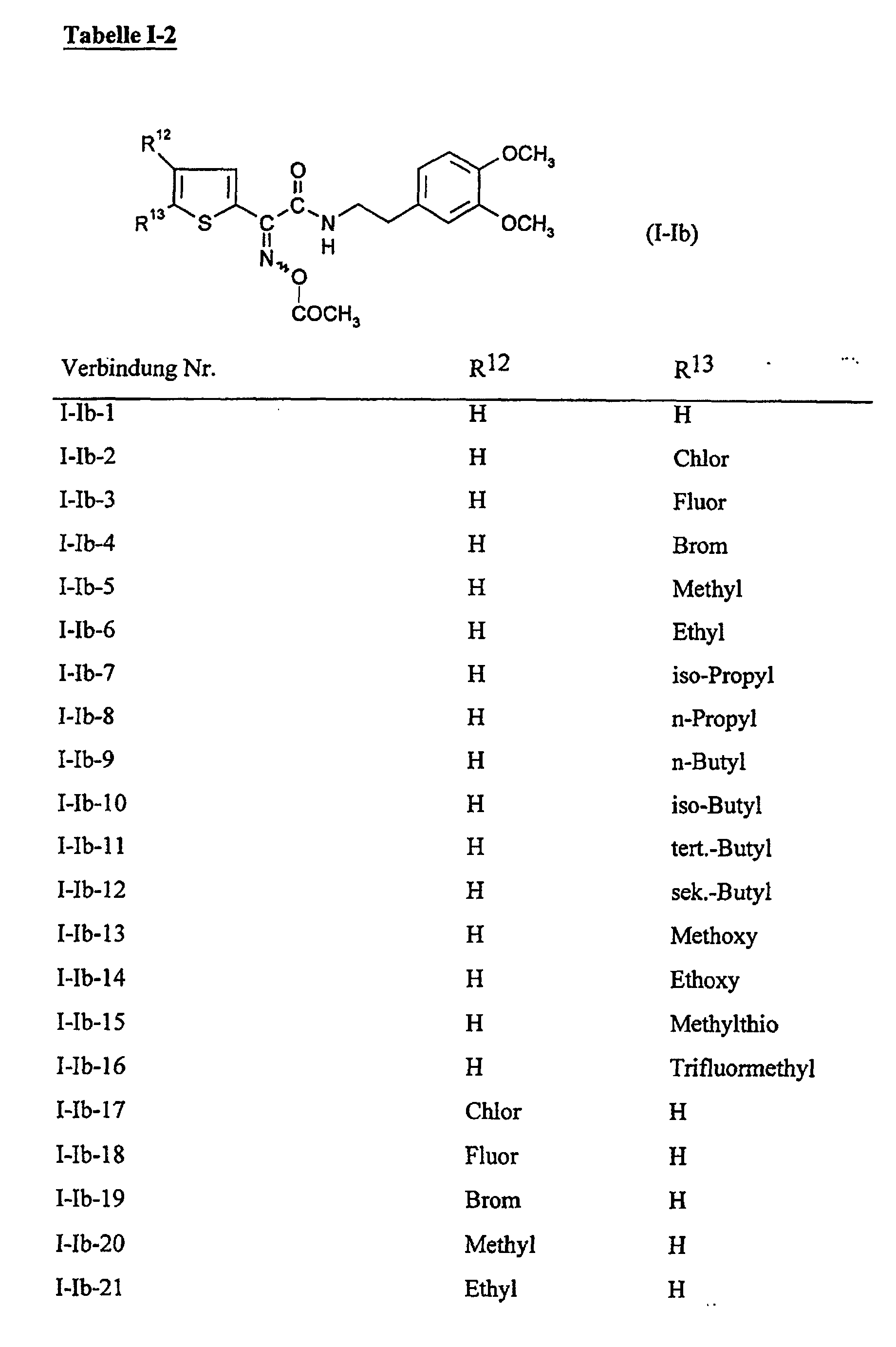

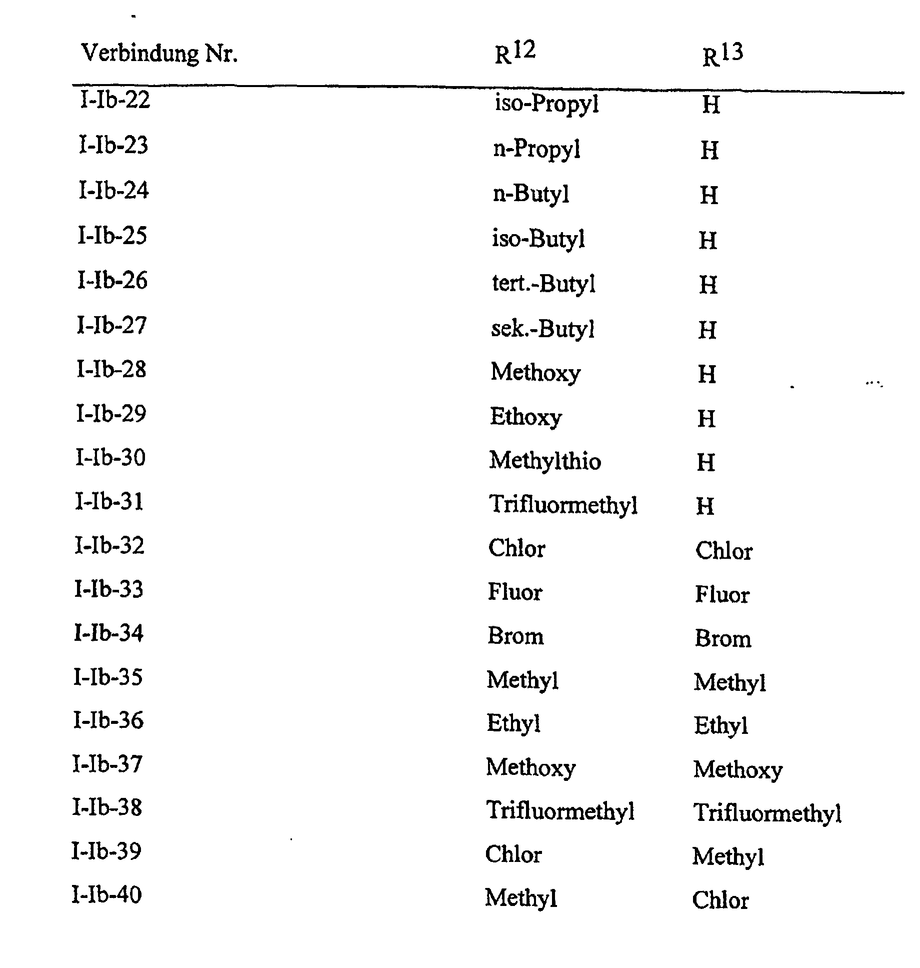

### Tabelle I-3

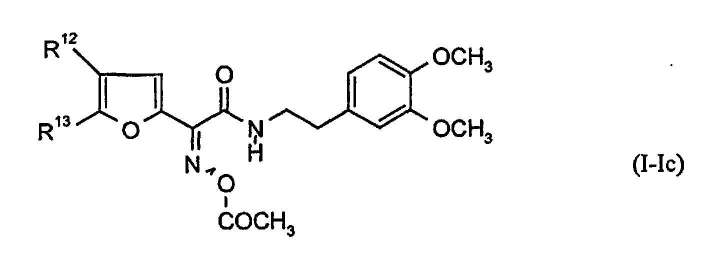

wobei R¹² und R¹³ für die in Tabelle I-2 genannten Substituenten stehen.

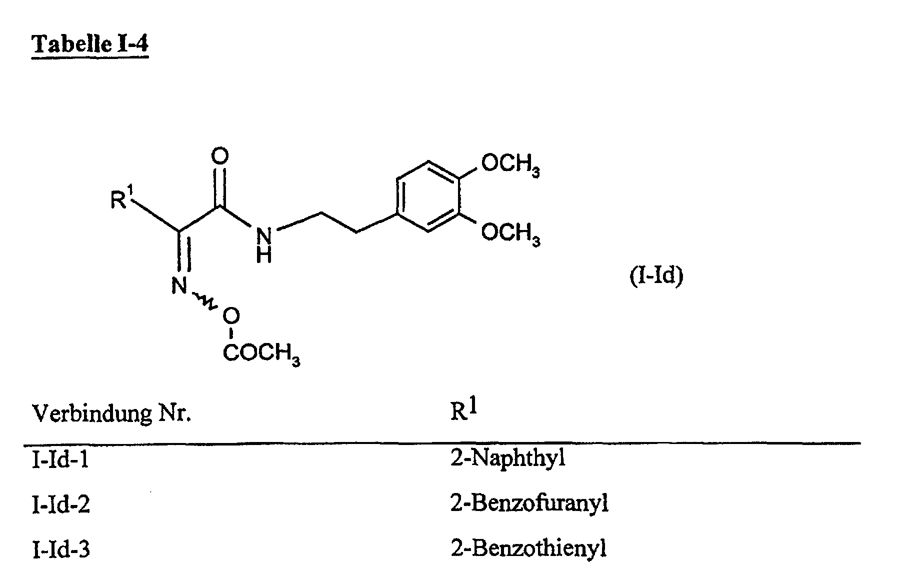

### Tabelle I-5

Verbindungen I-Ia-1 bis I-Id-3 entsprechend den Formeln I-Ia, I-Ib, I-Ic und I-Id, wobei an Stelle der 3,4-Dimethoxyphenyl-Gruppe (im allgemeinen bezeichnet als R⁴) ein Phenylrest steht, der die in den Verbindungen I-Ia-1 bis I-Ia-70 als R¹⁰ und R¹¹ jeweils angegebenen Substituenten trägt.

### Tabelle I-6

Verbindungen I-Ia-1 bis I-Id-3 entsprechend den Formeln I-Ia, I-Ib, I-Ic und I-Id, wobei an Stelle der 3,4-Dimethoxyphenyl-Gruppe (im allgemeinen bezeichnet als R⁴) einer der folgenden dreifach substituierten Phenylreste steht: 3,4,5-Trimethoxyphenyl; 3,4,5-Trichlorphenyl; 3,4,5-Trimethylphenyl.

### Tabelle I-7

Verbindungen I-Ia-1 bis I-Id-3 entsprechend den Formeln I-Ia, I-Ib, I-Ic und I-Id, wobei anstelle der Acetylgruppe-Gruppe (im allgemeinen bezeichnet als R²) eine Methoxycarbonyl-Gruppe steht.

Weiterhin wurde gefunden, daß man die neuen Acylglyoxylsäureoxime der allgemeinen Formel (I-I) erhält, wenn man Glyoxylsäureoxime der Formel (I-II) 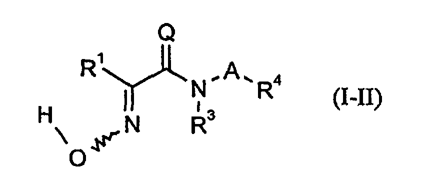 in welcher
A, R¹, R³ und R⁴ die in Formel (I-I) angegebenen Bedeutungen haben, mit einem aktivierten Säurederivat aus einer der Formeln (I-III) bis (I-X), 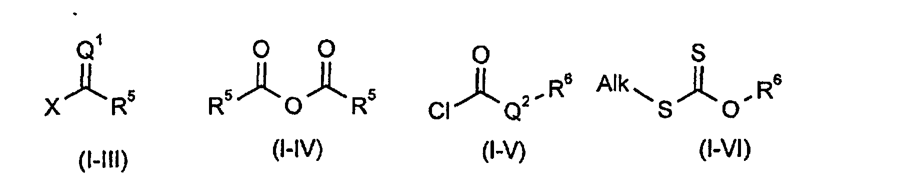 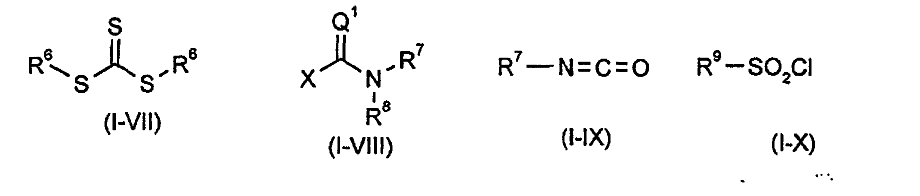 in welchen
R⁵, R⁶, R⁷, R⁸ und R⁹ die in Formel (I-I) angegebenen Bedeutungen haben,
gegebenenfalls in Gegenwart eines Verdünnungsmittels und gegebenenfalls in Gegenwart einer Base, umsetzt.

Die zur Durchführung des erfindungsgemäßen Verfahrens zur Herstellung der erfindungsgemäßen Verbindungen der Formel (I-I) als Ausgangsstoffe benötigten Glyoxylsäureoxime sind durch die Formel (I-II) allgemein definiert. In dieser Formel (I-II) haben A, R¹, R³ und R⁴ vorzugsweise diejenige Bedeutungen, die bereits im Zusammenhang mit der Beschreibung der erfindungsgemäßen Verbindungen der Formel (I-I) als bevorzugt bzw. als insbesondere bevorzugt für A, R¹, R³ und R⁴ angegeben wurden.

Die Ausgangsstoffe der Formel (I-II) werden erhalten, wenn man Carbonsäurederivate der allgemeinen Formel (I-XI) 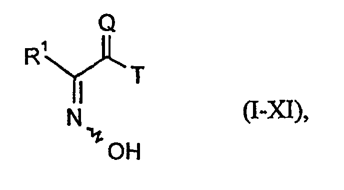 in welcher
- R¹ und Q: die in Formel (I-I) angegebenen Bedeutungen haben und
- T: für Hydroxy, Halogen oder Alkoxy steht,
mit einem Amin der allgemeinen Formel (I-XII) 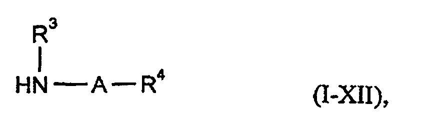 in welcher
R³, R⁴ und A die in Formel (I-I) angegebenen Bedeutungen haben,
- oder mit einem Hydrogenhalogenid hiervon ―
gegebenenfalls in Gegenwart eines Säureakzeptors, wie beispielsweise Triethylamin, gegebenenfalls in Gegenwart eines Kondensationsmittels, wie beispielsweise Dicyclohexylcarbodiimid, und gegebenenfalls in Gegenwart eines Verdünnungsmittels, wie beispielsweise Toluol, umsetzt.

Die zur Herstellung der Glyoxylsäureoxime der Formel (I-II) als Ausgangsstoffe benötigten Carbonsäurederivate sind durch die Formel (I-XI) allgemein definiert. In dieser Formel (I-XI) haben Q und R¹ vorzugsweise diejenigen Bedeutungen, die bereits im Zusammenhang mit der Beschreibung der erfindungsgemäßen Verbindungen der Formel (I-I) als bevorzugt bzw. als insbesondere bevorzugt für Q und R¹ angegeben wurden; T steht vorzugsweise für Alkoxy mit 1 bis 4 Kohlenstoffatomen, insbesondere für Methoxy oder Ethoxy, für Hydroxy oder Chlor.

Die Ausgangsstoffe der Formel (I-XI) sind bekannt und/oder können nach an sich bekannten Verfahren hergestellt werden (vgl. EP-A 178 826, EP-A 242 081, EP-A 382 375, EP-A 493 711, EP-A 432 503, DE-A 3 938 054).

Die weiterhin zur Herstellung der Glyoxylsäureoxime der Formel (I-II) als Ausgangsstoffe benötigten Amine sind durch die Formel (I-XII) allgemein definiert. In dieser Formel (I-XII) haben R³, R⁴ und A vorzugsweise diejenigen Bedeutungen, die bereits im Zusammenhang mit der Beschreibung der erfindungsgemäßen Verbindungen der Formel (I-I) als bevorzugt bzw. als insbesondere bevorzugt für R³, R⁴ und A angegeben wurden.

Die Amine der Formel (I-XII) sind bekannte organische Synthesechemikalien und/oder können nach an sich bekannten Verfahren hergestellt werden.

Die weiterhin zur Durchführung des erfindungsgemäßen Verfahrens zur Herstellung der erfindungsgemäßen Verbindungen der Formel (I-I) benötigten aktivierten Säurederivate sind durch die Formeln (I-III) bis (I-X) allgemein definiert. In diesen Formeln (I-III) bis (I-X) haben R⁵, R⁶, R⁷, R⁸ und R⁹ vorzugsweise diejenigen Bedeutungen, die bereits im Zusammenhang mit der Beschreibung der erfindungsgemäßen Verbindungen der Formel (I-I) als bevorzugt bzw. als insbesondere bevorzugt für R⁵, R⁶, R⁷, R⁸ und R⁹ angegeben wurden.

Die aktivierten Säurederivate der Formeln (I-III) bis (I-X) sind bekannte organische Synthesechemikalien und/oder können nach an sich bekannten Verfahren hergestellt werden.

Als Verdünnungsmittel zur Durchführung des erfindungsgemäßen Verfahrens zur Herstellung der erfindungsgemäßen Verbindungen der Formel (I-I) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; oder Sulfone, wie Sulfolan.

Das erfindungsgemäße Verfahren zur Herstellung der erfindungsgemäßen Verbindungen der Formel (I-I) wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens zur Herstellung der erfindungsgemäßen Verbindungen der Formel (I-I) in einem größeren Bereich variiert werden. Im allgemeinen arbeitet man bei Temperaturen von -30°C bis 150°C, vorzugsweise bei Temperaturen von -10°C bis 80°C.

Zur Durchführung des erfindungsgemäßen Verfahrens zur Herstellung der Verbindungen der Formel (I-I) setzt man pro Mol des Glyoxylsäureoxims der Formel (I-II) im allgemeinen 1 bis 15 Mol, vorzugsweise 2 bis 8 Mol aktiviertes Säurederivat aus einer der Formeln (I-IV) bis (I-X) ein.

Das erfindungsgemäße Verfahren zur Herstellung der erfindungsgemäßen Verbindungen der Formel (I-I) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Die Reaktionsdurchführung, Aufarbeitung und Isolierung der Reaktionsprodukte erfolgt nach bekannten Verfahren (vergleiche auch die Herstellungsbeispiele).

Bevorzugt sind auch Verbindungen der allgemeinen Formel (I-I)' 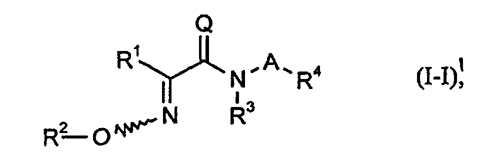 in welcher
- A: für eine Einfachbindung oder für gegebenenfalls einfach oder mehrfach, gleich oder verschieden substituiertes Alkylen mit 1 bis 6 Kohlenstoffatomen steht, wobei die möglichen Substituenten aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen, substituiertes Aryl oder Heterocyclyl,
- Q: für Sauerstoff oder Schwefel steht,
- R¹: für gegebenenfalls einfach oder mehrfach, gleich oder verschieden substituiertes, am Benzolring verknüpftes, Benzoheterocyclyl mit 3 bis 12 Ringgliedem im Heterocyclylteil und einem, zwei oder drei Heteroatomen (wobei jedoch höchstens ein Heteroatom für Sauerstoff steht) steht, wobei die möglichen Substituenten aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 8 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 8 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
substituiertes Aryl, Aryloxy, Arylthio, Arylalkyl, Arylalkyloxy, Arylalkylthio, Heterocyclyl, Heterocyclyloxy, Heterocyclylthio, Heterocyclylalkyl, Heterocyclylalkyloxy oder Heterocyclylalkylthio.
- R²: für eine der folgenden Gruppierungen steht: 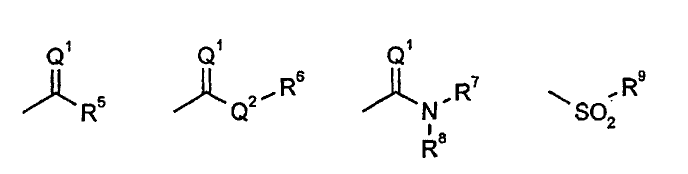 worin
Q¹ und Q² unabhängig voneinander für Sauerstoff oder Schwefel stehen,
R⁵ für Wasserstoff oder Alkyl mit 1 bis 6 Kohlenstoffatomen oder für jeweils gegebenenfalls im Arylteil durch Halogen, Cyano, Nitro, Alkyl oder Alkoxy mit jeweils 1 bis 4 Kohlenstoffatomen substituiertes Aryl oder Arylalkyl mit 1 bis 4 Kohlenstoffatomen im Alkylteil steht,
R⁶ für Alkyl mit 1 bis 6 Kohlenstoffatomen oder für jeweils gegebenenfalls im Arylteil durch Halogen, Cyano, Nitro, Alkyl oder Alkoxy mit jeweils 1 bis 4 Kohlenstoffatomen substituiertes Aryl oder Arylalkyl mit 1 bis 4 Kohlenstoffatomen im Alkylteil steht,
R⁷ für Wasserstoff, Alkyl mit 1 bis 6 Kohlenstoffatomen oder für jeweils gegebenenfalls im Arylteil durch Halogen, Cyano, Nitro, Alkyl oder Alkoxy mit jeweils 1 bis 4 Kohlenstoffatomen substituiertes Aryl oder Arylalkyl mit 1 bis 4 Kohlenstoffatomen im Alkylteil steht,
R⁸ für Alkyl mit 1 bis 6 Kohlenstoffatomen oder für jeweils gegebenenfalls im Arylteil durch Halogen, Cyano, Nitro, Alkyl oder Alkoxy mit jeweils 1 bis 4 Kohlenstoffatomen substituiertes Aryl oder Arylalkyl mit 1 bis 4 Kohlenstoffatomen im Alkylteil steht, oder
R⁷ und R⁸ gemeinsam mit dem Stickstoffatom, an das sie gebunden sind, für einen gegebenenfalls durch Methyl substituierten, heterocyclischen Ring stehen,
R⁹ für Alkyl mit 1 bis 6 Kohlenstoffatomen, Dialkylamino mit jeweils 1 bis 4 Kohlenstoffatomen in den einzelnen Alkylteilen, über Stickstoff gebundenes Heterocyclyl mit 3 bis 7 Ringgliedem oder für jeweils gegebenenfalls im Arylteil durch Halogen, Cyano, Nitro, Alkyl oder Alkoxy mit jeweils 1 bis 4 Kohlenstoffatomen substituiertes Aryl oder Arylalkyl mit 1 bis 4 Kohlenstoffatomen im Alkylteil steht,
- R³: für Wasserstoff oder Alkyl mit 1 bis 4 Kohlenstoffatomen steht,
- R⁴: für jeweils gegebenenfalls einfach oder mehrfach, gleich oder verschieden substituiertes Aryl, Cycloalkyl oder Cycloalkenyl mit 3 bis 8 Kohlenstoffatomen, oder Heterocyclyl mit 3 bis 12 Ringgliedern steht, wobei die möglichen Substituenten aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
substituiertes Aryl, Aryloxy, Arylthio, Arylalkyl, Arylalkyloxy, Arylalkylthio, Heterocyclyl, Heterocyclyloxy, Heterocyclylthio, Heterocyclylalkyl, Heterocyclylalkyloxy oder Heterocyclylalkylthio.

Die Erfindung betrifft insbesondere Verbindungen der Formel (I-I)', in welcher
- A: für eine Einfachbindung oder für jeweils gegebenenfalls einfach oder mehrfach, gleich oder verschieden durch Fluor, Chlor, Cyano oder Methoxy substituiertes Methylen, 1,1-Ethylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methyl-propylen) steht,
- Q: für Sauerstoff oder Schwefel steht,
- R¹: für eine der nachstehenden Gruppierungen 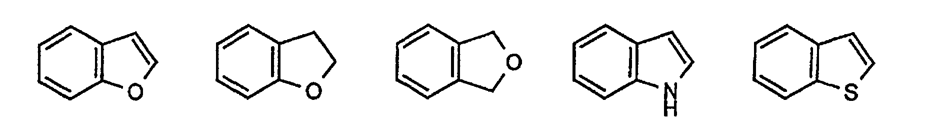 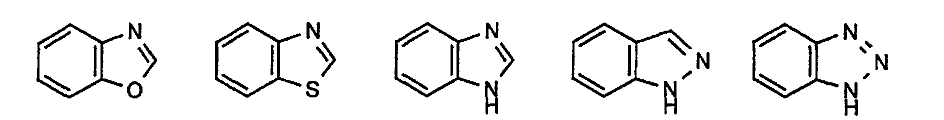 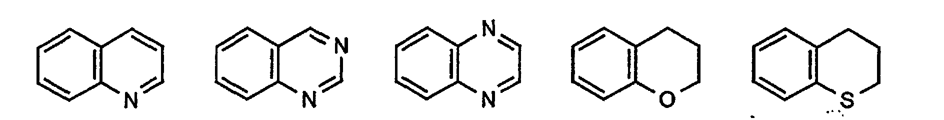 steht, die jeweils über ein Kohlenstoffatom des jeweiligen Benzolringes verbunden sind und die jeweils gegebenenfalls einfach bis dreifach, gleich oder verschieden durch die nachfolgend aufgezählten Substituenten substituiert sein können:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinoethyl oder Ethoximinoethyl;
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy;
Cyclopropyl, Cyclobutyl, Cyclopentyl oder Cyclohexyl;
gegebenenfalls durch die oben genannten Substituenten substituiertes Phenyl, Benzyl, Phenoxy oder Benzyloxy,
- R²: für eine der folgenden Gruppierungen steht: 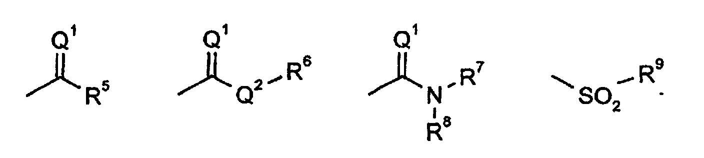 worin
Q¹ und Q² unabhängig voneinander für Sauerstoff oder Schwefel stehen,
R⁵ für Wasserstoff oder Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s-oder t-Butyl, Methoxy oder Ethoxy substituiertes Phenyl, Benzyl, 1-Phenylethyl oder 2-Phenylethyl steht,
R⁶ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propy], n-, i-, s- oder t-Butyl, Methoxy oder Ethoxy substituiertes Phenyl, Benzyl, 1-Phenylethyl oder 2-Phenylethyl steht,
R⁷ für Wasserstoff, Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁸ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht, oder
R⁷ und R⁸ gemeinsam mit dem Stickstoffatom, an das sie gebunden sind, für einen gegebenenfalls durch Methyl substituierten, heterocyclischen Ring stehen,
R⁹ für Methyl, Ethyl, n- oder i-Propyl, n-, i-, s- oder t-Butyl, Dimethylamino, Diethylamino, Pyrrolidin-1-yl, Morpholin-N-yl, Piperidin-1-yl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
- R³: für Wasserstoff oder für Methyl oder Ethyl steht,
- R⁴: für jeweils gegebenenfalls einfach bis dreifach substituiertes Cyclopropyl, Cyclobutyl, Cyclopentyl oder Cyclohexyl, Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy, Cyclopropyl, Cyclobutyl, Cyclopentyl oder Cyclohexyl.

Besonders bevorzugt sind Verbindungen der Formel (I-I)', in welcher
- A: für eine Einfachbindung oder für gegebenenfalls einfach oder mehrfach, gleich oder verschieden durch Fluor, Chlor, Cyano, Methoxy substituiertes Methylen, 1,1-Ethylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methyl-propylen) steht,
- Q: für Sauerstoff oder Schwefel steht,
- R¹: für eine der nachstehenden Gruppierungen 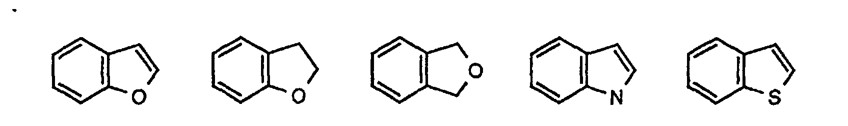 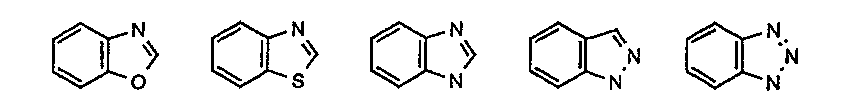 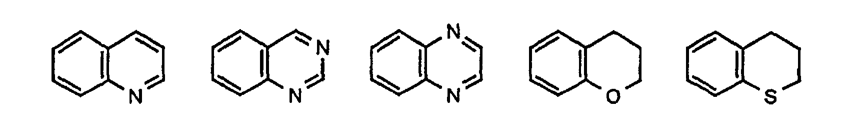 steht, die jeweils über ein Kohlenstoffatom des jeweiligen Benzolringes verbunden sind und die jeweils gegebenenfalls einfach bis dreifach, gleich oder verschieden durch die nachfolgend aufgezählten Substituenten substituiert sein können:
Fluor, Chlor, Brom, Cyano, Nitro, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinoethyl oder Ethoximinoethyl;
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy; Cyclopropyl, Cyclopentyl, Cyclohexyl; gegebenenfalls durch die obengenannten Substituenten substituiertes Phenyl, Phenoxy, Benzyloxy;
- R²: für eine der folgenden Gruppierungen steht: 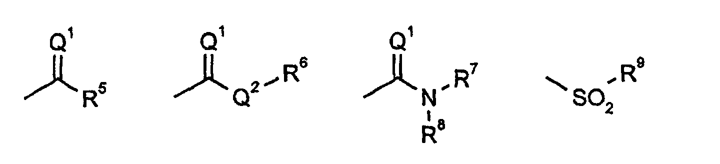 worin
Q¹ und Q² unabhängig voneinander für Sauerstoff oder Schwefel stehen,
R⁵ für Wasserstoff oder Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s-oder t-Butyl, Methoxy oder Ethoxy substituiertes Phenyl, Benzyl, 1-Phenylethyl oder 2-Phenylethyl steht,
R⁶ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁷ für Wasserstoff, Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁸ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht, oder
R⁷ und R⁸ gemeinsam mit dem Stickstoffatom, an das sie gebunden sind, für einen gegebenenfalls durch Methyl substituierten, heterocyclischen Ring stehen,
R⁹ für Methyl, Ethyl, n- oder i-Propyl, n-, i-, s- oder t-Butyl, Dimethylamino, Diethylamino, Pyrrolidin-1-yl, Morpholin-N-yl, Piperidin-1-yl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R³ für Wasserstoff oder für Methyl steht,
R⁴ für jeweils gegebenenfalls einfach bis sechsfach substituiertes Cyclopropyl, Cyclobutyl, Cyclopentyl oder Cyclohexyl steht, wobei als Substituenten die unten genannten bevorzugt sind;
für jeweils gegebenenfalls einfach bis dreifach substituiertes Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl, jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl.

Eine ganz besonders bevorzugte Gruppe erfindungsgemäßer Verbindungen sind diejenigen Verbindungen der Formel (I-I)', in welcher
- A: für eine Einfachbindung oder für Methylen, 1,1-Ethylen, 1,2-Ethylen, 1,1-Propylen, 1,2-Propylen, 1,3-Propylen oder 2,2-Propylen steht,
- Q: für Sauerstoff steht,
- R¹: für eine der nachstehenden Gruppierungen 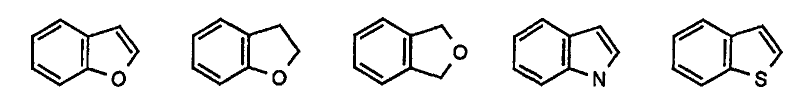 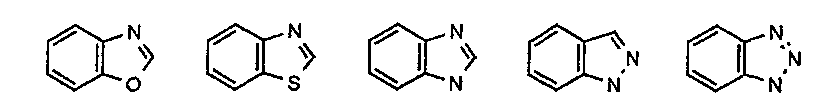 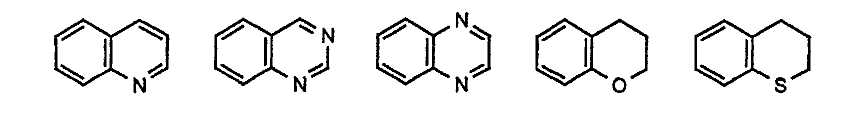 steht, die jeweils über ein Kohlenstoffatom des jeweiligen Benzolringes verbunden sind und die jeweils gegebenenfalls einfach bis dreifach, gleich oder verschieden durch die nachfolgend aufgezählten Substituenten substituiert sein können:
Brom, Chlor, Fluor, Nitro, Methylsulfonyl, Phenyl, Phenyloxy, Benzyloxy, Cyclopropyl, Cyclohexyl, Methyl, Ethyl, n- oder i-Propyl, n-, i-, s- oder t-Butyl, n-Pentyl, n-Hexyl, n-Heptyl, Methoxy, Ethoxy, Trifluormethyl, Trifluormethoxy und/oder Methylthio,
- R²: für eine der folgenden Gruppierungen steht: 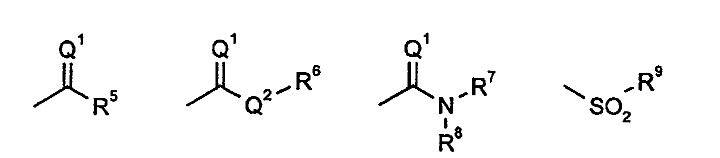 worin
Q¹ und Q² für Sauerstoff stehen,
R⁵ für Wasserstoff oder Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s-oder t-Butyl, Methoxy oder Ethoxy substituiertes Phenyl, Benzyl, 1-Phenylethyl oder 2-Phenylethyl steht,
R⁶ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁷ für Wasserstoff, Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁸ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht, oder
R⁷ und R⁸ gemeinsam mit dem Stickstoffatom, an das sie gebunden sind, für einen gegebenenfalls durch Methyl substituierten, heterocyclischen Ring stehen,
R⁹ für Methyl, Ethyl, n- oder i-Propyl, n-, i-, s- oder t-Butyl, Dimethylamino, Diethylamino, Pyrrolidin-1-yl, Morpholin-N-yl, Piperidin-1-yl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
- R³: für Wasserstoff oder Methyl steht,
- R⁴: für Cyclohexyl oder gegebenenfalls einfach bis dreifach substituiertes Phenyl, Thienyl, Furyl, Benzofuryl, Benzothienyl, Pyridyl, Pyrimidinyl, Naphthyl, Chinolyl steht, wobei die möglichen Substituenten aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy steht.

Insbesondere sind auch Verbindungen der Formel (I-I)' bevorzugt, in denen
- R²: für Methoxy, Fluormethoxy, Ethoxy, Methylamino oder Dimethylamino steht.

Insbesondere sind auch Verbindungen der Formel (I-I)' bevorzugt, in denen
- R³: für Wasserstoff steht.

Insbesondere sind auch Verbindungen der Formel (I-I)' bevorzugt, in denen R⁴ für in 3- und 4-Stellung durch Methoxy substituiertes Phenyl steht.

In einer weiterhin besonders bevorzugten Gruppe von Verbindungen steht A für -CH₂-CH₂-.

Die oben aufgeführten allgemeinen oder in Vorzugsbereichen angegebenen Restedefinitionen gelten sowohl für die Endprodukte der Formel (I-I)' als auch entsprechend für die jeweils zur Herstellung benötigten Ausgangsstoffe bzw. Zwischenprodukte.

Diese Restedefinitionen können untereinander, also auch zwischen den angegebenen Bereichen bevorzugter Verbindungen, beliebig kombiniert werden.

Weiterhin wurde gefunden, daß man die neuen Benzoheterocyclylglyoxylsäureamide der allgemeinen Formel (I-I)' erhält, wenn man
Verfahren III-a) Carbonsäurederivate der allgemeinen Formel (III-II) 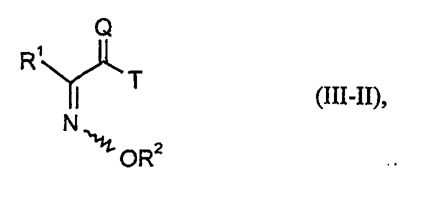 in welcher
   - R¹, R² und Q: die in Formel (I-I)' angegebenen Bedeutungen haben und
   - T: für Hydroxy, Halogen oder Alkoxy steht,
   mit einem Amin der allgemeinen Formel (III-III) 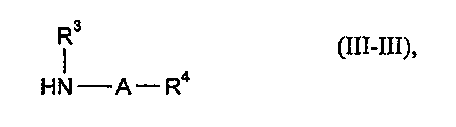 in welcher
   R³, R⁴ und A die in der Formel (I-I)' angegebenen Bedeutungen haben,
   - oder mit einem Hydrogenhalogenid hiervon ―
   gegebenenfalls in Gegenwart eines Säureakzeptors, gegebenenfalls in Gegenwart eines Kondensationsmittels und gegebenenfalls in Gegenwart eines Verdünnungsmittels umsetzt, oder wenn man
Verfahren III-b) Glyoxylsäureamide der allgemeinen Formel (III-IV) 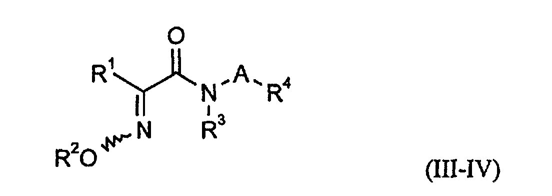 in welcher
   A, R¹, R², R³ und R⁴ die in der Formel (I-I)' angegebenen Bedeutungen haben, mit einem Schwefelungsreagenz, gegebenenfalls in Gegenwart eines Verdünnungsmittels, umsetzt, oder wenn man
Verfahren III-c) Glyoxylsäurederivate der Formel (III-V) 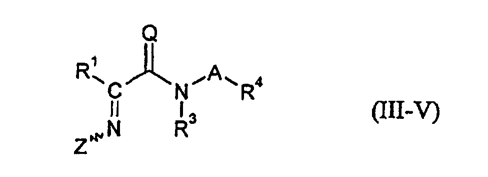 in welcher
   - A, R¹, R³ und R⁴: die in der Formel (I-I)' angegebenen Bedeutungen haben, und
   - Z: für Hydroxy steht,
   mit einem aktivierten Säurederivat aus einer der Formeln (III-VI) bis (III-XIII), 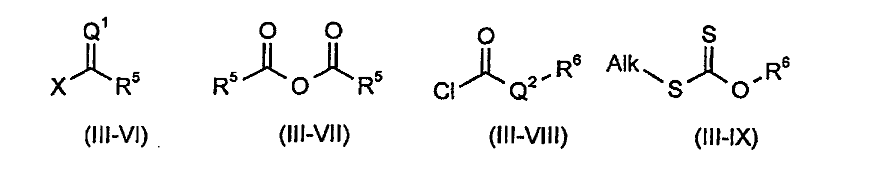 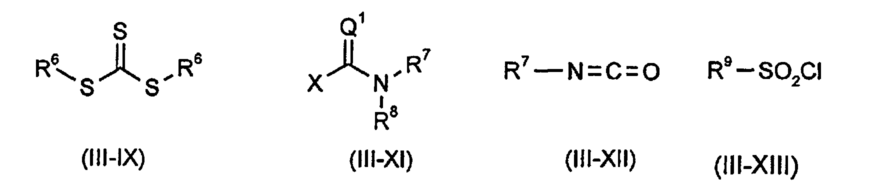 in welchen
   - R⁵, R⁶, R⁷, R⁸ und R⁹: die in der Formel (I-I)' angegebenen Bedeutungen haben,
   gegebenenfalls in Gegenwart eines Verdünnungsmittels und gegebenenfalls in Gegenwart einer Base, umsetzt.

Die zur Durchführung des erfindungsgemäßen Verfahrens III-a) als Ausgangsstoffe benötigten Carbonsäurederivate sind durch die Formel (III-II) allgemein definiert. In dieser Formel (III-II) haben Q und R¹ vorzugsweise diejenigen Bedeutungen, die bereits im Zusammenhang mit der Beschreibung der erfindungsgemäßen Verbindungen der Formel (I-I)' als bevorzugt bzw. als insbesondere bevorzugt für Q und R¹ angegeben wurden; T steht vorzugsweise für Alkoxy mit 1 bis 4 Kohlenstoffatomen, insbesondere für Methoxy oder Ethoxy, für Hydroxy oder Chlor.
Die Ausgangsstoffe der Formel (III-II) sind bekannt und/oder können nach an sich bekannten Verfahren hergestellt werden (vgl. EP-A 178 826, EP-A 242 081, EP-A 382 375, EP-A 493 711, EP-A 432 503, DE-A 3 938 054, J. Heterocycl. Chem. (1990), 27(3), 487-95, Farmaco, Ed. Sci. (1980), 35(5), 394-404, Justus Liebigs Ann. Chem. (1969), 722, 38-44, Justus Liebigs Ann. Chem. (1969), 722, 29-37, Tetrahedron 1971, 3431-6).

Die weiterhin zur Durchführung des erfindungsgemäßen Verfahrens III-a) als Ausgangsstoffe benötigten Amine sind durch die Formel (III-III) allgemein definiert. In dieser Formel (III-III) haben R³, R⁴ und A vorzugsweise diejenigen Bedeutungen, die bereits im Zusammenhang mit der Beschreibung der erfindungsgemäßen Verbindungen der Formel (I-I)' als bevorzugt bzw. als insbesondere bevorzugt für R³, R⁴ und A angegeben wurden.

Die Amine der Formel (III-III) sind bekannte organische Synthesechemikalien und/oder können nach an sich bekannten Verfahren hergestellt werden.

Die zur Durchführung des erfindungsgemäßen Verfahrens III-b) als Ausgangsstoffe benötigten Glyoxylsäureamide sind durch die Formel (III-IV) allgemein definiert. In dieser Formel (III-IV) haben A, R¹, R², R³ und R⁴ vorzugsweise diejenigen Bedeutungen, die bereits im Zusammenhang mit der Beschreibung der erfindungsgemäßen Verbindungen der Formel (I-I)' als bevorzugt bzw. als insbesondere bevorzugt für A, R¹, R², R³ und R⁴ angegeben wurden.

Die Glyoxylsäureamide der allgemeinen Formel (III-IV) sind erfindungsgemäße Verbindungen und können nach den erfindungsgemäßen Verfahren III-a) oder III-c) erhalten werden.

Als Schwefelungsreagenz zur Durchführung des erfindungsgemäßen Verfahrens III-b) kommen alle Reagenzien infrage, die in der Lage sind, an Kohlenstoff gebundene Sauerstoffatome gegen Schwefelatome auszutauschen, wie z. B. Schwefelwasserstoff, Phosphorpentasulfid oder Lawesson's Reagenz.

Die zur Durchführung des erfindungsgemäßen Verfahrens III-c) als Ausgangsstoffe benötigten Glyoxylsäurederivate sind durch die Formel (III-V) allgemein definiert. In dieser Formel (III-V) haben A, R¹, R³ und R⁴ die vorzugsweise insbesondere diejenigen Bedeutungen, die bereits im Zusammenhang mit der Beschreibung der erfindungsgemäßen Verbindungen der Formel (I-I)' als bevorzugt bzw. als insbesondere bevorzugt für A, R¹, R³ und R⁴ angegeben wurden. Z steht für Hydroxy oder Amino.

Die Ausgangsstoffe der Formel (III-V) sind erfindungsgemäße Verbindungen und können nach den erfindungsgemäßen Verfahren III-a) oder III-b) erhalten werden.

Die weiterhin zur Durchführung des erfindungsgemäßen Verfahrens III-c) zur Herstellung der erfindungsgemäßen Verbindungen der Formel (I-I)' benötigten aktivierten Säurederivate sind durch die Formeln (III-VI) bis (III-XIII) allgemein definiert. In diesen Formeln (III-VI) bis (III-XIII) haben R⁵, R⁶, R⁷, R⁸ und R⁹ vorzugsweise, bzw. insbesondere diejenigen Bedeutungen, die bereits im Zusammenhang mit der Beschreibung der erfindungsgemäßen Verbindungen der Formel (I-I)' als bevorzugt bzw. als insbesondere bevorzugt für R⁵, R⁶, R⁷, R⁸ und R⁹ angegeben wurden.

Die aktivierten Säurederivate der Formeln (III-VI) bis (III-XII) sind bekannte organische Synthesechemikalien und/oder können nach an sich bekannten Verfahren hergestellt werden.

Das erfindungsgemäße Verfahren III-a) wird gegebenenfalls in Gegenwart eines Verdünnungsmittels durchgeführt. Als Verdünnungsmittel zur Durchführung des erfindungsgemäßen Verfahrens kommen Wasser und organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykol-dimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, Ethylenglykol, Ethylenglykolmonomethylether, Ethylenglykolmonoethylether, Diethylenglykolmonomethylether, Diethylenglykolmonoethylether, deren Gemische mit Wasser oder reines Wasser.

Das erfindungsgemäße Verfahren III-a) wird gegebenenfalls in Gegenwart eines geeigneten Säureakzeptors durchgeführt. Als solche kommen alle üblichen anorganischen oder organischen Basen in Frage. Hierzu gehören beispielsweise Erdalkalimetall- oder Alkalimetallhydride, hydroxide, amide, alkoholate, acetate, carbonate oder hydrogencarbonate, wie beispielsweise Natriumhydrid, Natriumamid, Natrium-methylat, Natrium-ethylat, Kalium-tert.-butylat, Natriumhydroxid, Kaliumhydioxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethylbenzylamin, Pyridin, N-Methylpiperidin, N,N-Dimethylaminopyridin, Diazabicyclooctan (DABCO), Diazabicyclononen (DBN) oder Diazabicycloundecen (DBU).

Das erfindungsgemäße Verfahren III-a) wird gegebenenfalls in Gegenwart eines geeigneten Kondensationsmittels durchgeführt. Als solche kommen alle üblicherweise für derartige Amidierungsreaktionen verwendbaren Kondensationsmittel infrage. Beispielhaft genannt seien Säurehalogenidbildner wie Phosgen, Phosphortribromid, Phosphortrichlorid, Phosphorpentachlorid, Phosphoroxychlorid oder Thionylchlorid; Anhydridbildner wie Chlorameisensäureethylester, Chlorameisensäuremethylester oder Methansulfonylchlorid; Carbodiimide, wie N,N'-Dicyclohexylcarbodiimid (DCC) oder andere übliche Kondensationsmittel, wie Phosphorpentoxid, Polyphosphorsäure, N,N'-Carbonyldiimidazol, 2-Ethoxy-N-ethoxycarbonyl-1,2-dihydrochinolin (EEDQ) oder Triphenylphosphin/Tetrachlorkohlenstoff.

Das erfindungsgemäße Verfahren III-a) wird gegebenenfalls in Gegenwart eines Katalysators durchgeführt. Beispielsweise genannt seien 4-Dimethylaminopyridin, 1-Hydroxy-benzotriazol oder Dimethylformamid.

Die Reaktionstemperaturen können bei der Durchführung des erfindungsgemäßen Verfahrens III-a) in einem größeren Bereich variiert werden. Im allgemeinen arbeitet man bei Temperaturen zwischen -20°C und +200°C, vorzugsweise bei Temperaturen zwischen 0°C und 150°C.

Zur Durchführung des erfindungsgemäßen Verfahrens III-a) setzt man je Mol an Carbonsäurederivat der Formel (III-II) im allgemeinen 1 bis 5 Mol, vorzugsweise 1,0 bis 2,5 Mol an Amin ein.

Die Reaktionsdurchführung, Aufarbeitung und Isolierung der Reaktionsprodukte erfolgt nach bekannten Verfahren (vgl. die Herstellungsbeispiele).

Das erfindungsgemäße Verfahren III-a) kann auch als zweistufiger Prozess durchgeführt werden. Dabei werden die Carbonsäurederivate der allgemeinen Formel (III-II) zunächst in eine aktivierte Form überführt und in einem anschließenden Schritt mit den Aminen der allgemeinen Formel (III-III) zu den erfindungsgemäßen Alkoximinoessigsäurederivaten der allgemeinen Formel (I-I)' umgesetzt.

Als aktivierte Form der Carbonsäurederivate der Formel (III-II) kommen alle Carboxy-aktivierten Derivate infrage, wie z.B. Säurehalogenide, bevorzugt Säurechloride, Säureazide, ferner symmetrische und gemischte Anhydride, wie beispielsweise die gemischten O-Alkylkohlensäureanhydride, weiterhin aktivierte Ester, wie z.B. p-Nitrophenylester oder N-Hydroxisuccinimidester sowie Addukte mit Kondensationsmitteln, wie z.B. Dicyclohexylcarbodiimid oder in situ erzeugte aktivierte Formen der Carbonsäuren.

Als Verdünnungsmittel zur Durchführung des erfindungsgemäßen Verfahrens III-b) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol.

Die Reaktionstemperaturen können bei der Durchführung des erfindungsgemäßen Verfahrens III-b) in einem größeren Bereich variiert werden. Im allgemeinen arbeitet man bei Temperaturen von 0°C bis 150°C, vorzugsweise bei Temperaturen von 0°C bis 80°C.

Zur Durchführung des erfindungsgemäßen Verfahrens III-b) zur Herstellung der Verbindungen der Formel (I-I)' setzt man pro Mol des Glyoxylsäureamides der Formel (III-IV) im allgemeinen 0,1 bis 15 Mol, vorzugsweise 0,5 bis 8 Mol Schwefelungsreagenz ein.

Als Verdünnungsmittel zur Durchführung des erfindungsgemäßen Verfahrens III-c) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; oder Sulfone, wie Sulfolan.

Das erfindungsgemäße Verfahren III-c) wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetalloder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens III-c) in einem größeren Bereich variiert werden. Im allgemeinen arbeitet man bei Temperaturen von 0°C bis 150°C, vorzugsweise bei Temperaturen von 0°C bis 80°C.

Zur Durchführung des erfindungsgemäßen Verfahrens III-c) zur Herstellung der Verbindungen der Formel (I-I)' setzt man pro Mol des Glyoxylsäurederivates der Formel (III-V) im allgemeinen 1 bis 15 Mol, vorzugsweise 2 bis 8 Mol aktiviertes Säurederivat aus einer der Formeln (III-VI) bis (III-XII) ein.

Bevorzugt sind auch Verbindungen der allgemeinen Formel (I-I)", 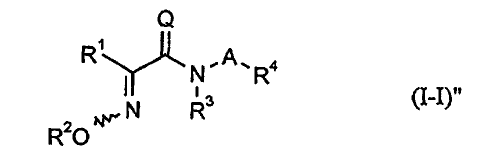 in welcher
- A: für eine Einfachbindung oder für gegebenenfalls einfach oder mehrfach, gleich oder verschieden substituiertes Alkylen mit 1 bis 6 Kohlenstoffatomen steht, wobei die möglichen Substituenten aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes, Alkoxy, Alkylthio, Alkylsulfinyl oder
Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen, substituiertes Aryl oder Heterocyclyl,
- Q: für Sauerstoff oder Schwefel steht,
- R¹: für einen Tricyclus 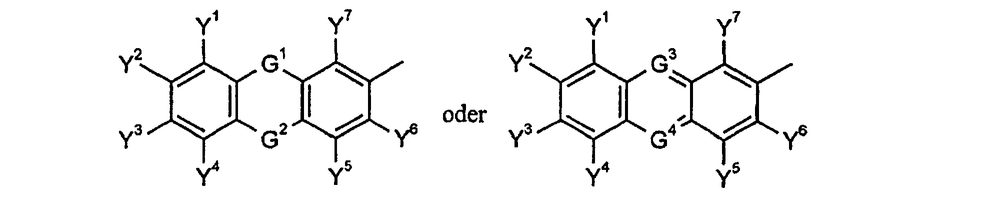 steht, worin
- G¹ und G²: unabhängig voneinander für eine Einfachbindung, Alkandiyl, Alkendiyl, Sauerstoff, Schwefel, -NH-, -N(Alkyl)- oder Carbonyl stehen,
- G³ und G⁴: unabhängig voneinander für Stickstoff oder eine Gruppierung 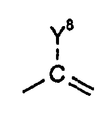 stehen und
- Y¹, Y², Y³, Y⁴, Y⁵, Y⁶, Y⁷ und Y⁸: unabhängig voneinander für Wasserstoff,Halogen, Cyano, Nitro;
Alkyl, Alkoxy, Alkylamino, Dialkylamino, Alkylthio, Alkylsulfinyl oder Alkylsulfonyl mit jeweils 1 bis 8 Kohlenstoffatomen in den einzelnen Alkylteilen;
Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen oder
Cycloalkyl mit 3 bis 6 Kohlenstoffatomen stehen,
- R²: für eine der folgenden Gruppierungen steht: 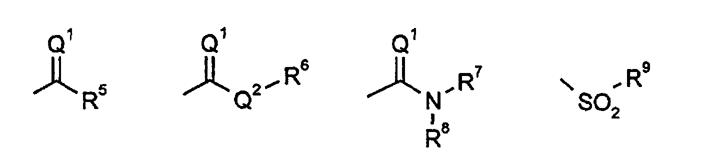 worin
Q¹ und Q² unabhängig voneinander für Sauerstoff oder Schwefel stehen,
R⁵ für Wasserstoff oder Alkyl mit 1 bis 6 Kohlenstoffatomen oder für jeweils gegebenenfalls im Arylteil durch Halogen, Cyano, Nitro, Alkyl oder Alkoxy mit jeweils 1 bis 4 Kohlenstoffatomen substituiertes Aryl oder Arylalkyl mit 1 bis 4 Kohlenstoffatomen im Alkylteil steht,
R⁶ für Alkyl mit 1 bis 6 Kohlenstoffatomen oder für jeweils gegebenenfalls im Arylteil durch Halogen, Cyano, Nitro, Alkyl oder Alkoxy mit jeweils 1 bis 4 Kohlenstoffatomen substituiertes Aryl oder Arylalkyl mit 1 bis 4 Kohlenstoffatomen im Alkylteil steht,
R⁷ für Wasserstoff, Alkyl mit 1 bis 6 Kohlenstoffatomen oder für jeweils gegebenenfalls im Arylteil durch Halogen, Cyano, Nitro, Alkyl oder Alkoxy mit jeweils 1 bis 4 Kohlenstoffatomen substituiertes Aryl oder Arylalkyl mit 1 bis 4 Kohlenstoffatomen im Alkylteil steht,
R⁸ für Alkyl mit 1 bis 6 Kohlenstoffatomen oder für jeweils gegebenenfalls im Arylteil durch Halogen, Cyano, Nitro, Alkyl oder Alkoxy mit jeweils 1 bis 4 Kohlenstoffatomen substituiertes Aryl oder Arylalkyl mit 1 bis 4 Kohlenstoffatomen im Alkylteil steht, oder
R⁷ und R⁸ gemeinsam mit dem Stickstoffatom, an das sie gebunden sind, für einen gegebenenfalls durch Methyl substituierten, heterocyclischen Ring stehen,
R⁹ für Alkyl mit 1 bis 6 Kohlenstoffatomen, Dialkylamino mit jeweils 1 bis 4 Kohlenstoffatomen in den einzelnen Alkylteilen, über Stickstoff gebundenes Heterocyclyl mit 3 bis 7 Ringgliedern oder für jeweils gegebenenfalls im Arylteil durch Halogen, Cyano, Nitro, Alkyl oder Alkoxy mit jeweils 1 bis 4 Kohlenstoffatomen substituiertes Aryl oder Arylalkyl mit 1 bis 4 Kohlenstoffatomen im Alkylteil steht,
- R³: für Wasserstoff oder Alkyl mit 1 bis 4 Kohlenstoffatomen steht,
- R⁴: für jeweils gegebenenfalls einfach oder mehrfach, gleich oder verschieden substituiertes Aryl, Cycloalkyl oder Cycloalkenyl mit 3 bis 8 Kohlenstoffatomen, oder Heterocyclyl mit 3 bis 12 Ringgliedern steht, wobei die möglichen Substituenten aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4
Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
substituiertes Aryl, Aryloxy, Arylthio, Arylalkyl, Arylalkyloxy, Arylalkylthio, Heterocyclyl, Heterocyclyloxy, Heterocyclylthio, Heterocyclylalkyl, Heterocyclylalkyloxy oder Heterocyclylalkylthio.

Die Erfindung betrifft insbesondere Verbindungen der Formel (I-I)", in welcher
- A: für eine Einfachbindung oder für jeweils gegebenenfalls einfach oder mehrfach, gleich oder verschieden durch Fluor, Chlor, Cyano oder Methoxy substituiertes Methylen, 1,1-Ethylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methyl-propylen) steht,
- Q: für Sauerstoff oder Schwefel steht,
- R¹: für einen Tricyclus 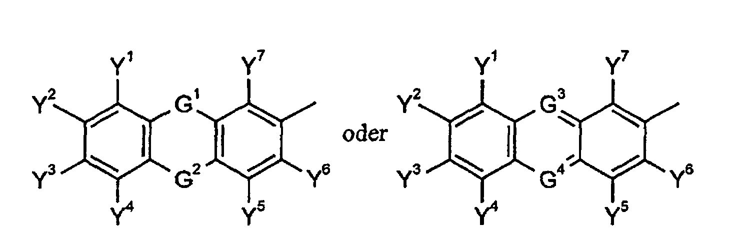 steht, worin
G¹ und G² unabhängig voneinander für eine Einfachbindung, Alkandiyl, Alkendiyl, Sauerstoff, Schwefel, -NH-, -N(Alkyl)- oder Carbonyl stehen und
G³ und G⁴ unabhängig voneinander für Stickstoff oder eine Gruppierung 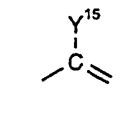 stehen und
Y¹, Y², Y³, Y⁴, Y⁵, Y⁶, Y⁷ und Y⁸ unabhängig voneinander für Wasserstoff, Fluor, Chlor, Brom, Cyano, Nitro, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n-oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Cyclopropyl, Cyclobutyl, Cyclopentyl oder Cyclohexyl stehen,
- R²: für eine der folgenden Gruppierungen steht: 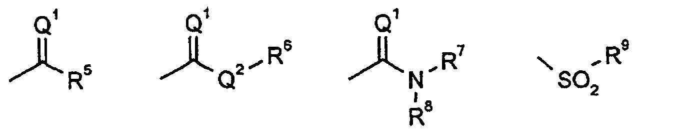 worin
Q¹ und Q² unabhängig voneinander für Sauerstoff oder Schwefel stehen,
R⁵ für Wasserstoff oder Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s-oder t-Butyl, Methoxy oder Ethoxy substituiertes Phenyl, Benzyl, 1-Phenylethyl oder 2-Phenylethyl steht,
R⁶ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁷ für Wasserstoff, Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁸ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht, oder
R⁷ und R⁸ gemeinsam mit dem Stickstoffatom, an das sie gebunden sind, für einen gegebenenfalls durch Methyl substituierten, heterocyclischen Ring stehen,
R⁹ für Methyl, Ethyl, n- oder i-Propyl, n-, i-, s- oder t-Butyl, Dimethylamino, Diethylamino, Pyrrolidin-1-yl, Morpholin-N-yl, Piperidin-1-yl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
- R³: für Wasserstoff oder für Methyl oder Ethyl steht,
- R⁴: für jeweils gegebenenfalls einfach bis dreifach substituiertes Cyclopropyl, Cyclobutyl, Cyclopentyl oder Cyclohexyl, Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl.

Besonders bevorzugt sind Verbindungen der Formel (I-I)", in welcher
- A: für eine Einfachbindung oder für gegebenenfalls einfach oder mehrfach, gleich oder verschieden durch Fluor, Chlor, Cyano, Methoxy substituiertes Methylen, 1,1-Ethylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methyl-propylen) steht,
- Q: für Sauerstoff oder Schwefel steht,
- R¹: für einen Tricyclus 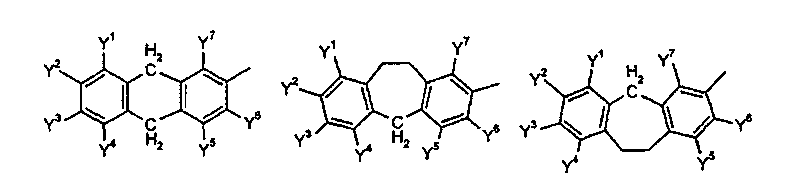 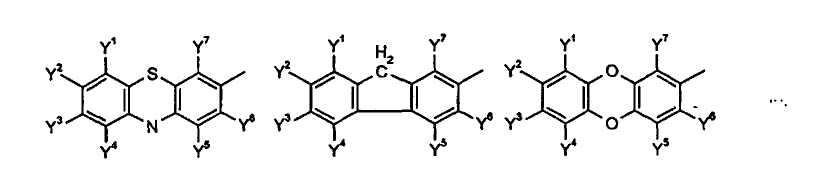 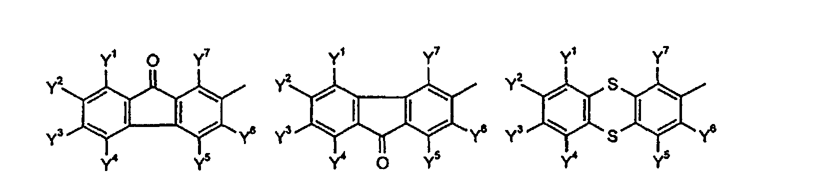 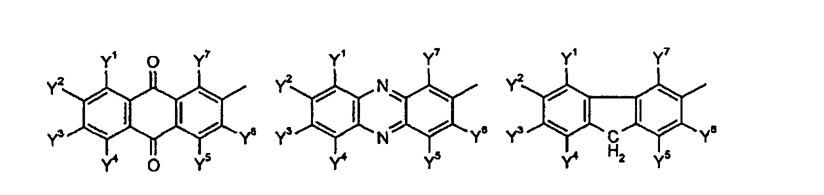 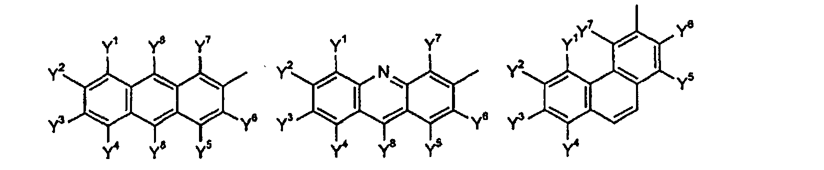 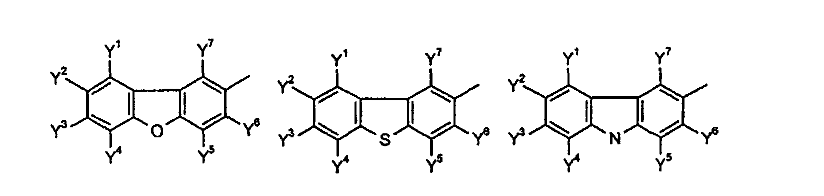 steht, worin
- Y¹, Y², Y³, Y⁴, Y⁵, Y⁶, Y⁷ und Y⁸: unabhängig voneinander für Wasserstoff, Fluor, Chlor, Brom, Cyano, Nitro, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- o-der i-Propylamino, Dimethylamino, Diethylamino, Cyclopropyl, Cyclobutyl, Cyclopentyl oder Cyclohexyl stehen,
- R²: für eine der folgenden Gruppierungen steht: 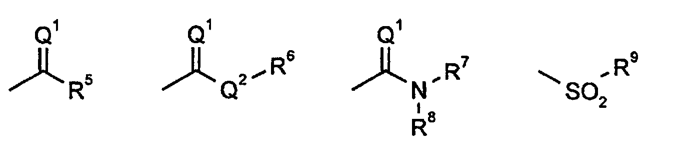 worin
Q¹ und Q² unabhängig voneinander für Sauerstoff oder Schwefel stehen,
R⁵ für Wasserstoff oder Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s-oder t-Butyl, Methoxy oder Ethoxy substituiertes Phenyl, Benzyl, 1-Phenylethyl oder 2-Phenylethyl steht,
R⁶ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁷ für Wasserstoff, Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁸ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht, oder
R⁷ und R⁸ gemeinsam mit dem Stickstoffatom, an das sie gebunden sind, für einen gegebenenfalls durch Methyl substituierten, heterocyclischen Ring stehen,
R⁹ für Methyl, Ethyl, n- oder i-Propyl, n-, i-, s- oder t-Butyl, Dimethylamino, Diethylamino, Pyrrolidin-1-yl, Morpholin-N-yl, Piperidin-1-yl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R³ für Wasserstoff oder für Methyl steht,
R⁴ für jeweils gegebenenfalls einfach bis sechsfach substituiertes Cyclopropyl, Cyclobutyl, Cyclopentyl oder Cyclohexyl steht, wobei als Substituenten die untengenannten bevorzugt sind; für jeweils gegebenenfalls einfach bis dreifach substituiertes Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl, jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl.

Eine ganz besonders bevorzugte Gruppe erfindungsgemäßer Verbindungen sind diejenigen Verbindungen der Formel (I-I)", in welcher
- A: für eine Einfachbindung oder für Methylen, 1,1-Ethylen, 1,2-Ethylen, 1,1-Propylen, 1,2-Propylen, 1,3-Propylen oder 2,2-Propylen steht,
- Q: für Sauerstoff steht,
- R¹: für einen Tricyclus 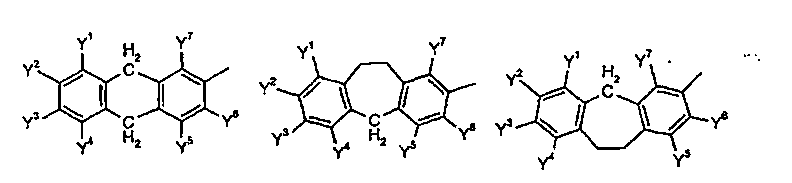 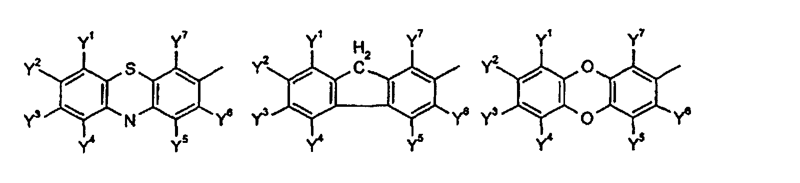 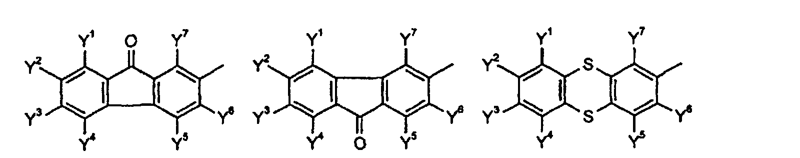 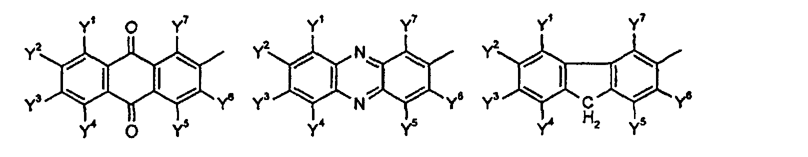 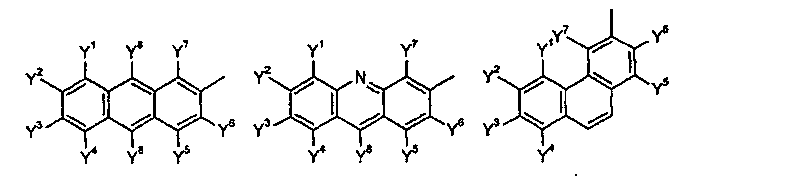 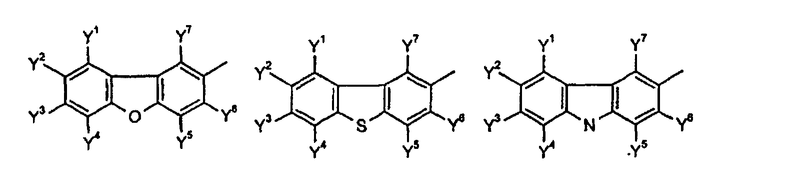 steht, worin
- Y¹, Y², Y³, Y⁴, Y⁵, Y⁶, Y⁷ und Y⁸: unabhängig voneinander für Wasserstoff, Fluor, Chlor, Brom, Cyano, Nitro, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Cyclopropyl, Cyclobutyl, Cyclopentyl oder Cyclohexyl stehen,
- R²: für eine der folgenden Gruppierungen steht: 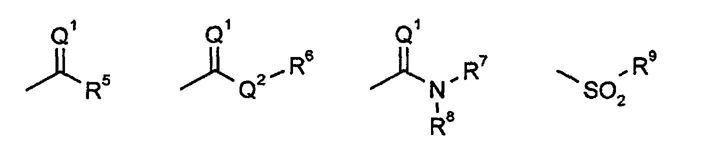 worin
Q¹ und Q² für Sauerstoff stehen,
R⁵ für Wasserstoff oder Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s-oder t-Butyl, Methoxy oder Ethoxy substituiertes Phenyl, Benzyl, 1-Phenylethyl oder 2-Phenylethyl steht,
R⁶ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁷ für Wasserstoff, Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁸ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht, oder
R⁷ und R⁸ gemeinsam mit dem Stickstoffatom, an das sie gebunden sind, für einen gegebenenfalls durch Methyl substituierten, heterocyclischen Ring stehen,
R⁹ für Methyl, Ethyl, n- oder i-Propyl, n-, i-, s- oder t-Butyl, Dimethylamino, Diethylamino, Pyrrolidin-1-yl, Morpholin-N-yl, Piperidin-1-yl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
- R³: für Wasserstoff oder Methyl steht,
- R⁴: für Cyclohexyl oder gegebenenfalls einfach bis dreifach substituiertes Phenyl, Thienyl, Furyl, Benzofuryl, Benzothienyl, Pyridyl, Pyrimidinyl, Naphthyl, Chinolyl steht, wobei die möglichen Substituenten aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy steht.

Insbesondere sind auch Verbindungen der Formel (I-I)" bevorzugt, in denen
- R²: für Methoxy, Fluormethoxy, Ethoxy, Methylamino oder Dimethylamino steht.

Insbesondere sind auch Verbindungen der Formel (I-I)" bevorzugt, in denen
- R³: für Wasserstoff steht.

Insbesondere sind auch Verbindungen der Formel (I-I)" bevorzugt, in denen
- R⁴: für in 3- und 4-Stellung durch Methoxy substituiertes Phenyl steht.

In einer weiterhin besonders bevorzugten Gruppe von Verbindungen steht A für -CH₂-CH₂-.

Die oben aufgeführten allgemeinen oder in Vorzugsbereichen angegebenen Restedefinitionen gelten sowohl für die Endprodukte der Formel (I-I)" als auch entsprechend für die jeweils zur Herstellung benötigten Ausgangsstoffe bzw. Zwischenprodukte.

Diese Restedefinitionen können untereinander, also auch zwischen den angegebenen Bereichen bevorzugter Verbindungen, beliebig kombiniert werden.

Weiterhin wurde gefunden, daß man die neuen Arylglyoxylsäureamide der allgemeinen Formel (I-I)" erhält, wenn man
Verfahren IV-a) Carbonsäurederivate der allgemeinen Formel (IV-II) 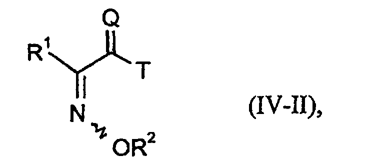 in welcher
   - R¹, R² und Q: die in Formel (I-I)" angegebenen Bedeutungen haben und
   - T: für Hydroxy, Halogen oder Alkoxy steht,
   mit einem Amin der allgemeinen Formel (IV-III) 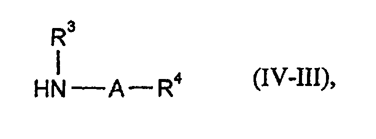 in welcher
   R³, R⁴ und A die oben angegebenen Bedeutungen haben,
   - oder mit einem Hydrogenhalogenid hiervon -
   gegebenenfalls in Gegenwart eines Säureakzeptors, gegebenenfalls in Gegenwart eines Kondensationsmittels und gegebenenfalls in Gegenwart eines Verdünnungsmittels umsetzt, oder wenn man
Verfahren IV-b) Glyoxylsäureamide der allgemeinen Formel (IV-IV) 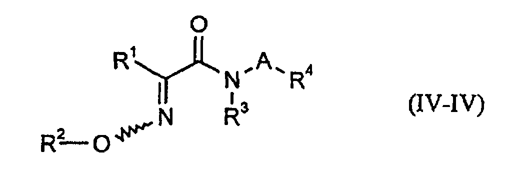 in welcher
   A, R¹, R², R³ und R⁴ die in Formel (I-I)" angegebenen Bedeutungen haben,
   mit einem Schwefelungsreagenz, gegebenenfalls in Gegenwart eines Verdünnungsmittels, umsetzt, oder wenn man
Verfahren IV-c) Glyoxylsäurederivate der Formel (IV-V) 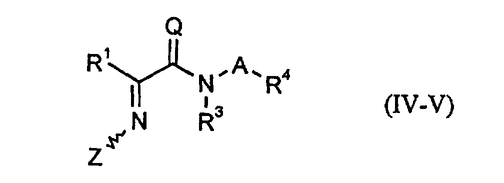 in welcher
   - A, R¹, R³ und R⁴: die in Formel (I-I)" angegebenen Bedeutungen haben, und
   - Z: für Hydroxy steht,
   mit einem aktivierten Säurederivat aus einer der Formeln ((IV-VI) bis ((IV-XIII), 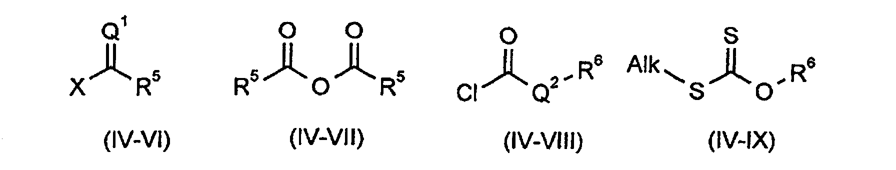 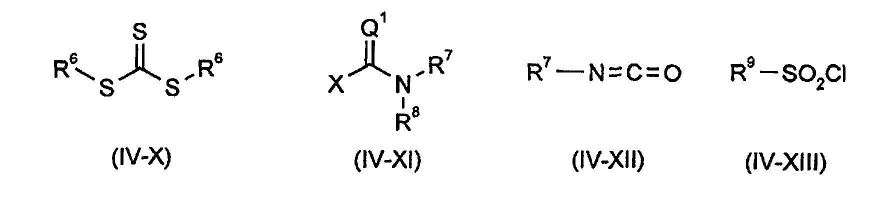 in welchen
   R⁵, R⁶, R⁷, R⁸ und R⁹ die in Formel (I-I)" angegebenen Bedeutungen haben,
   gegebenenfalls in Gegenwart eines Verdünnungsmittels und gegebenenfalls in Gegenwart einer Base, umsetzt.

Die zur Durchführung des erfindungsgemäßen Verfahrens IV-a) als Ausgangsstoffe benötigten Carbonsäurederivate sind durch die Formel (IV-II) allgemein definiert. In dieser Formel (IV-II) haben Q und R¹ vorzugsweise diejenigen Bedeutungen, die bereits im Zusammenhang mit der Beschreibung der erfindungsgemäßen Verbindungen der Formel (I-I)" als bevorzugt bzw. als insbesondere bevorzugt für Q und R¹ angegeben wurden; T steht vorzugsweise für Alkoxy mit 1 bis 4 Kohlenstoffatomen, insbesondere für Methoxy oder Ethoxy, für Hydroxy oder Chlor.

Die Ausgangsstoffe der Formel (IV-II) sind bekannt und/oder können nach an sich bekannten Verfahren hergestellt werden (vgl. EP-A 178 826, EP-A 242 081, EP-A 382 375, EP-A 493 711, EP-A 432 503, DE-A 3 938 054, J. Heterocycl. Chem. (1990), 27(3), 487-95, Farmaco, Ed. Sci. (1980), 35(5), 394-404, Justus Liebigs Ann. Chem. (1969), 722, 38-44, Justus Liebigs Ann. Chem. (1969), 722, 29-37, Tetrahedron 1971, 3431-6).

Die weiterhin zur Durchführung des erfindungsgemäßen Verfahrens IV-a) als Ausgangsstoffe benötigten Amine sind durch die Formel (IV-III) allgemein definiert. In dieser Formel (IV-III) haben R³, R⁴ und A vorzugsweise diejenigen Bedeutungen, die bereits im Zusammenhang mit der Beschreibung der erfindungsgemäßen Verbindungen der Formel (I-I)" als bevorzugt bzw. als insbesondere bevorzugt für R³, R⁴ und A angegeben wurden.

Die Amine der Formel (IV-III) sind bekannte organische Synthesechemikalien und/oder können nach an sich bekannten Verfahren hergestellt werden.

Die zur Durchführung des erfindungsgemäßen Verfahrens IV-b) als Ausgangsstoffe benötigten Glyoxylsäureamide sind durch die Formel (IV-IV) allgemein definiert. In dieser Formel (IV-IV) haben A, R¹, R², R³ und R⁴ vorzugsweise diejenigen Bedeutungen, die bereits im Zusammenhang mit der Beschreibung der erfindungsgemäßen Verbindungen der Formel (I-I)" als bevorzugt bzw. als insbesondere bevorzugt für A, R¹, R², R³ und R⁴ angegeben wurden.

Die Glyoxylsäureamide der allgemeinen Formel (IV-IV) sind erfindungsgemäße Verbindungen und können nach den erfindungsgemäßen Verfahren IV-a) oder IV-c) erhalten werden.

Als Schwefelungsreagenz zur Durchführung des erfindungsgemäßen Verfahrens IVb) kommen alle Reagenzien infrage, die in der Lage sind, an Kohlenstoff gebundene Sauerstoffatome gegen Schwefelatome auszutauschen, wie z. B. Schwefelwasserstoff, Phosphorpentasulfid oder Lawesson's Reagenz.

Die zur Durchführung des erfindungsgemäßen Verfahrens IV-c) als Ausgangsstoffe benötigten Glyoxylsäurederivate sind durch die Formel (IV-V) allgemein definiert. In dieser Formel (IV-V) haben A, R¹, R³ und R⁴ die vorzugsweise insbesondere diejenigen Bedeutungen, die bereits im Zusammenhang mit der Beschreibung der erfindungsgemäßen Verbindungen der Formel (I-I)" als bevorzugt bzw. als insbesondere bevorzugt für A, R¹, R³ und R⁴ angegeben wurden. Z steht für Hydroxy.

Die Ausgangsstoffe der Formel (IV-V) sind erfindungsgemäße Verbindungen und können nach den erfindungsgemäßen Verfahren IV-a) oder IV-b) erhalten werden. Die weiterhin zur Durchführung des erfindungsgemäßen Verfahrens IV-c) zur Herstellung der erfindungsgemäßen Verbindungen der Formel (I-I)" benötigten aktivierten Säurederivate sind durch die Formeln (IV-VI) bis (IV-XIII) allgemein definiert. In diesen Formeln (IV-VI) bis (IV-XIII) haben R⁵, R⁶, R⁷, R⁸ und R⁹ vorzugsweise diejenigen Bedeutungen, die bereits im Zusammenhang mit der Beschreibung der erfindungsgemäßen Verbindungen der Formel (I-I)" als bevorzugt bzw. als insbesondere bevorzugt für R⁵, R⁶, R⁷, R⁸ und R⁹ angegeben wurden.

Die aktivierten Säurederivate der Formeln (IV-VI) bis (IV-XIII) sind bekannte organische Synthesechemikalien und/oder können nach an sich bekannten Verfahren hergestellt werden.

Das erfindungsgemäße Verfahren IV-a) wird gegebenenfalls in Gegenwart eines Verdünnungsmittels durchgeführt. Als Verdünnungsmittel zur Durchführung des erfindungsgemäßen Verfahrens kommen Wasser und organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykol-dimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, Ethylenglykol, Ethylenglykolmonomethylether, Ethylenglykolmonoethylether, Diethylenglykolmonomethylether, Diethylenglykolmonoethylether, deren Gemische mit Wasser oder reines Wasser.

Das erfindungsgemäße Verfahren IV-a) wird gegebenenfalls in Gegenwart eines geeigneten Säureakzeptors durchgeführt. Als solche kommen alle üblichen anorganischen oder organischen Basen in Frage. Hierzu gehören beispielsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethylbenzylamin, Pyridin, N-Methylpiperidin, N,N-Dimethylaminopyridin, Diazabicyclooctan (DABCO), Diazabicyclononen (DBN) oder Diazabicycloundecen (DBU). Das erfindungsgemäße Verfahren IV-a) wird gegebenenfalls in Gegenwart eines geeigneten Kondensationsmittels durchgeführt. Als solche kommen alle üblicherweise für derartige Amidierungsreaktionen verwendbaren Kondensationsmittel in Frage. Beispielhaft genannt seien Säurehalogenidbildner wie Phosgen, Phosphortribromid, Phosphortrichlorid, Phosphorpentachlorid, Phosphoroxychlorid oder Thionylchlorid; Anhydridbildner wie Chlorameisensäureethylester, Chlorameisensäuremethylester oder Methansulfonylchlorid; Carbodiimide, wie N,N'-Dicyclohexylcarbodiimid (DCC) oder andere übliche Kondensationsmittel, wie Phosphorpentoxid, Polyphosphorsäure, N,N'-Carbonyldiimidazol, 2-Ethoxy-N-ethoxycarbonyl-1,2-dihydrochinolin (EEDQ) oder Triphenylphosphin/Tetrachlorkohlenstoff.

Das erfindungsgemäße Verfahren IV-a) wird gegebenenfalls in Gegenwart eines Katalysators durchgeführt. Beispielsweise genannt seien 4-Dimethylaminopyridin, 1-Hydroxy-benzotriazol oder Dimethylformamid.

Die Reaktionstemperaturen können bei der Durchführung des erfindungsgemäßen Verfahrens IV-a) in einem größeren Bereich variiert werden. Im allgemeinen arbeitet man bei Temperaturen zwischen -20°C und +200°C, vorzugsweise bei Temperaturen zwischen 0°C und 150°C.

Zur Durchführung des erfindungsgemäßen Verfahrens IV-a) setzt man je Mol an Carbonsäurederivat der Formel (IV-II) im allgemeinen 1 bis 5 Mol, vorzugsweise 1,0 bis 2,5 Mol an Amin ein.

Die Reaktionsdurchführung, Aufarbeitung und Isolierung der Reaktionsprodukte erfolgt nach bekannten Verfahren (vgl. die Herstellungsbeispiele).

Das erfindungsgemäße Verfahren IV-a) kann auch als zweistufiger Prozess durchgeführt werden. Dabei werden die Carbonsäurederivate der allgemeinen Formel (IV-II) zunächst in eine aktivierte Form überführt und in einem anschließenden Schritt mit den Aminen der allgemeinen Formel (IV-III) zu den erfindungsgemäßen Alkoximinoessigsäurederivaten der allgemeinen Formel (I-I)" umgesetzt.

Als aktivierte Form der Carbonsäurederivate der Formel (IV-II) kommen alle Carboxy-aktivierten Derivate in Frage, wie z.B. Säurehalogenide, bevorzugt Säurechloride, Säureazide, ferner symmetrische und gemischte Anhydride, wie beispielsweise die gemischten O-Alkylkohlensäureanhydride, weiterhin aktivierte Ester, wie z.B. p-Nitrophenylester oder N-Hydroxisuccinimidester sowie Addukte mit Kondensationsmitteln, wie z.B. Dicyclohexylcarbodiimid oder in situ erzeugte aktivierte Formen der Carbonsäuren.

Als Verdünnungsmittel zur Durchführung des erfindungsgemäßen Verfahrens IV-b) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol.

Die Reaktionstemperaturen können bei der Durchführung des erfindungsgemäßen Verfahrens IV-b) in einem größeren Bereich variiert werden. Im allgemeinen arbeitet man bei Temperaturen von 0°C bis 150°C, vorzugsweise bei Temperaturen von 0°C bis 80°C.

Zur Durchführung des erfindungsgemäßen Verfahrens IV-b) zur Herstellung der Verbindungen der Formel (I-I)" setzt man pro Mol des Glyoxylsäureamides der Formel (IV-IV) im allgemeinen 0,1 bis 15 Mol, vorzugsweise 0,5 bis 8 Mol Schwefelungsreagenz ein.

Als Verdünnungsmittel zur Durchführung des erfindungsgemäßen Verfahrens IV-c) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2- Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; oder Sulfone, wie Sulfolan.

Das erfindungsgemäße Verfahren IV-c) wird gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetalloder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens IV-c) in einem größeren Bereich variiert werden. Im allgemeinen arbeitet man bei Temperaturen von 0°C bis 150°C, vorzugsweise bei Temperaturen von 0°C bis 80°C.

Zur Durchführung des erfindungsgemäßen Verfahrens IV-c) zur Herstellung der Verbindungen der Formel (I-I)" setzt man pro Mol des Glyoxylsäurederivates der Formel (IV-V) im allgemeinen 1 bis 15 Mol, vorzugsweise 2 bis 8 Mol aktiviertes Säurederivat aus einer der Formeln (IV-VI) bis (IV-XIII) ein.

Die erfindungsgemäßen Verfahren werden im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Die Reaktionsdurchführung, Aufarbeitung und Isolierung der Reaktionsprodukte erfolgt nach bekannten Verfahren (vgl. die Herstellungsbeispiele).

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Altemaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei werden die erfindungsgemäßen Wirkstoffe mit besonders gutem Erfolg zur Bekämpfung von Krankheiten im Wein-, Obst- und Gemüseanbau, wie beispielsweise gegen Plasmopara- und Phytophthora-Arten eingesetzt.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitem oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:

### Fungizide:

Aldimorph, Ampropylfos, Ampropylfos-Kalium, Andoprim, Anilazin, Azaconazol, Azoxystrobin,
Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
Calciumpolysulfid, Capsimycin, Captafol, Captan, Carbendazim, Carboxin, Carvon, Chinomethionat(Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
Ediphenphos, Epoxiconazol, Etaconazol, Ethirimol, Etridiazol,
Famoxadon, Fenapanil, Fenarimol, Fenbuconazol, Fenfuram, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
Guazatin,
Hexachlorobenzol, Hexaconazol, Hymexazol,
Imazalil, Imibenconazol, Iminoctadin, Iminoctadinealbesilate, Iminoctadinetriacetate, Iodocarb, Ipconazol, Iprobenfos(IBP), Iprodione, Irumamycin, Isoprothiolan, Isovaledione,
Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
Nickel-dimethyldithiocarbamat, Nitrothal-isopropyl, Nuarimol,
Ofurace, Oxadixyl, Oxamocarb, Oxolinicacid, Oxycarboxim, Oxyfenthiin,
Paclobutrazol, Pefurazoat, Penconazol, Pencycuron, Phosdiphen, Pimaricin, Piperalin, Polyoxin, Polyoxorim, Probenazol, Prochloraz, Procymidon, Propamocarb, Propanosine-Natrium, Propiconazol, Propineb, Pyrazophos, Pyrifenox, Pyrimethanil, Pyroquilon, Pyroxyfur,
Quinconazol, Quintozen(PCNB),
Schwefel und Schwefel-Zubereitungen,
Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Triflumizol, Triforin, Triticonazol,
Uniconazol,
Validamycin A, Vinclozolin, Viniconazol,
Zarilamid, Zineb, Ziram sowie
Dagger G,
OK-8705,
OK-8801,
α-(1,1-Dimethylethyl)-β-(2-phenoxyethyl)-1H-1,2,4-triazol-1-ethanol,
α-(2,4-Dichlorphenyl)-β-fluor-β-propyl-1H-1,2,4-triazol-1-ethanol,
α-(2,4-Dichlorphenyl)-β-methoxy-α-methyl-1H-1,2,4-triazol-1-ethanol,
α-(5-Methyl-1,3-dioxan-5-yl)-β-[[4-(trifluormethyl)-phenyl]-methylen]-1H-1,2,4-triazol-1-ethanol,
(5RS,6RS)-6-Hydroxy-2,2,7,7-tetramethyl-5-(1H-1,2,4-triazol-1-yl)-3-octanon,
(E)-α-(Methoxyimino)-N-methyl-2-phenoxy-phenylacetamid,
{2-Methyl-1-[[[1-(4-methylphenyl)ethyl]amino]carbonyl]propyl}-carbaminsäure-1-isopropylester
1-(2,4-Dichlorphenyl)-2-(1H-1,2,4-triazol-1-yl)ethanon-O-(phenylmethyl)-oxim,
1-(2-Methyl-1-naphthalenyl)-1H-pyrrol-2,5-dion,
1-(3,5-Dichlorphenyl)-3-(2-propenyl)-2,5-pyrrolidindion,
1-[(Diiodmethyl)-sulfonyl]-4-methyl-benzol,
1-[[2-(2,4-Dichlorphenyl)-1,3-dioxolan-2-yl]-methyl]-1H-imidazol,
1-[[2-(4-Chlorphenyl)-3-phenyloxiranyl]-methyl]-1H-1,2,4-triazol,
1-[1-(2-[(2,4-Dichlorphenyl)methoxy]phenyl]ethenyl]-1H-imidazol,
1-Methyl-5-nonyl-2-(phenylmethyl)-3-pyrrolidinol,
2',6'-Dibrom-2-methyl-4'-trifluormethoxy-4'-trifluor-methyl-1,3-thiazol-5-carboxanilid,
2,2-Dichlor-N-[1-(4-chlorphenyl)ethyl]-1-ethyl-3-methyl-cyclopropancarboxamid,
2,6-Dichlor-5-(methylthio)-4-pyrimidinyl-thiocyanat,
2,6-Dichlor-N-(4-trifluormethylbenzyl)-benzamid,
2,6-Dichlor-N-[[4-(trifluormethyl)phenyl]methyl]-benzamid,
2-(2,3,3-Triiod-2-propenyl)-2H-tetrazol,
2-[(1-Methylethyl)sulfonyl]-5-(trichlormethyl)-1,3,4-thiadiazol,
2-[[6-Deoxy-4-O-(4-O-methyl-β-D-glycopyranosyl)-α-D-glucopyranosyl]-amino]-4-methoxy-1H-pyrrolo[2,3-d]pyrimidin-5-carbonitril,
2-Aminobutan,
2-Brom-2-(brommethyl)-pentandinitril,
2-Chlor-N-(2,3-dihydro-1,1,3-trimethyl-1H-inden-4-yl)-3-pyridincarboxamid,
2-Chlor-N-(2,6-dimethylphenyl)-N-(isothiocyanatomethyl)-acetamid,
2-Phenylphenol(OPP),
3,4-Dichlor-1-[4-(difluormethoxy)-phenyl]-1H-pyrrol-2,5-dion,
3,5-Dichlor-N-[cyan[(1-methyl-2-propynyl)oxy]methyl] -benzamid,
3-(1,1-Dimethylpropyl-1-oxo-1H-inden-2-carbonitril,
3-[2-(4-Chlorphenyl)-5-ethoxy-3-isoxazolidinyl]-pyridin,
4-Chlor-2-cyan-N,N-dimethyl-5-(4-methylphenyl)-1H-imidazol-1-sulfonamid,
4-Methyl-tetrazolo[1,5-a]quinazolin-5(4H)-on,
8-(1,1-Dimethylethyl)-N-ethyl-N-propyl-1,4-dioxaspiro[4.5]decan-2-methanamin,
8-Hydroxychinolinsulfat,
9H-Xanthen-9-carbonsäure-2-[(phenylamino)-carbonyl]-hydrazid,
bis-(1-Methylethyl)-3-methyl-4-[(3-methylbenzoyl)oxy]-2,5-thiophendicarboxylat,
cis-1-(4-Chlorphenyl)-2-(1H-1,2,4-triazol-1-yl)-cycloheptanol,
cis-4-[3-[4-(1,1-Dimethylpropyl)-phenyl-2-methylpropyl]-2,6-dimethyl-morpholinhydrochlorid,
Ethyl-[(4-chlorphenyl)-azo]-cyanoacetat,
Kaliumhydrogencarbonat,
Methantetrathiol, -Natriumsalz,
Methyl-1-(2,3-dihydro-2,2-dimethyl-1H-inden-1-yl)-1H-imidazol-5-carboxylat,
Methyl-N-(2,6-dimethylphenyl)-N-(5-isoxazolylcarbonyl)-DL-alaninat,
Methyl-N-(chloracetyl)-N-(2,6-dimethylphenyl)-DL-alaninat,
N-(2,3-Dichlor-4-hydroxyphenyl)-1-methyl-cyclohexancarboxamid.
N-(2,6-Dimethylphenyl)-2-methoxy-N-(tetrahydro-2-oxo-3-furanyl)-acetamid,
N-(2,6-Dimethylphenyl)-2-methoxy-N-(tetrahydro-2-oxo-3-thienyl)-acetamid,
N-(2-Chlor-4-nitrophenyl)-4-methyl-3-nitro-benzolsulfonamid,
N-(4-Cyclohexylphenyl)-1,4,5,6-tetrahydro-2-pyrimidinamin,
N-(4-Hexylphenyl)-1,4,5,6-tetrahydro-2-pyrimidinamin,
N-(5-Chlor-2-methylphenyl)-2-methoxy-N-(2-oxo-3-oxazolidinyl)-acetamid,
N-(6-Methoxy)-3-pyridinyl)-cyclopropancarboxamid,
N-[2,2,2-Trichlor-1-[(chloracetyl)-amino]-ethyl]-benzamid,
N-[3-Chlor-4,5-bis(2-propinyloxy)-phenyl]-N'-methoxy-methanimidamid,
N-Formyl-N-hydroxy-DL-alanin, -Natriumsalz,
O,O-Diethyl-[2-(dipropylamino)-2-oxoethyl]-ethylphosphoramidothioat,
O-Methyl-S-phenyl-phenylpropylphosphoramidothioat,
S-Methyl-1,2,3-benzothiadiazol-7-carbothioat,
spiro[2H]-1-Benzopyran-2,1'(3'H)-isobenzofuran]-3'-on,

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephat, Acrinathrin, Alanycarb, Aldicarb, Alphamethrin, Amitraz, A-vermectin, AZ 60541, Azadirachtin, Azinphos A, Azinphos M, Azocyclotin,
Bacillus thuringiensis, 4-Bromo-2-(4-chlorphenyl)-1-(ethoxymethyl)-5-(trifluoromethyl)-lH-pyrrole-3-carbonitrile, Bendiocarb, Benfuracarb, Bensultap, Betacyfluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, Chloethocarb, Chlorethoxyfos, Chlorfenvinphos, Chlorfluazuron, Chlormephos, N-[(6-Chloro-3-pyridinyl)-methyl]-N'-cyano-N-methyl-ethanimidamide, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Fluazuron ,Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin,
Lamda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, Nitenpyram
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenophos, Promecarb, Propaphos, Propoxur, Prothiophos, Prothoat, Pymetrozin, Pyrachlophos, Pyridaphenthion, Pyresmethrin, Pyrethrum, Pyridaben, Pyrimidifen, Pyriproxifen,
Quinalphos,
Salithion, Sebufos, Silafluofen, Sulfotep, Sulprofos,
Tebufenozide, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, Zetamethrin.
Auch eine Mischung mit anderen bekannten Wirkstoffen, wie Herbiziden oder mit Düngemitteln und Wachstumsregulatoren ist möglich.

Die erfindungsgemäßen Wirkstoffe können als solche, in Form ihrer handelsüblichen Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verschäumen, Bestreichen usw. Es ist ferner möglich die Wirkstoffe nach dem Ultra-Low-Volume-Verfahren auszubringen oder die Wirkstoffzubereitung oder der Wirkstoff selbst in den Boden zu injizieren. Es kann wird auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

Die Wirkstoffe eignen sich bei guter Pflanzenverträglichkeit und günstiger Warmblütertoxizität auch zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Spodoptera litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp.
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Zu diesen Parasiten gehören:
Aus der Ordnung der Anoplurida z.B. Haematopinus spp., Linognathus spp., Pediculus spp., Phtirus spp., Solenopotes spp..
Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp.
Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp.
Aus der Ordnung der Siphonapterida z.B. Pulex spp., Ctenocephalides spp., Xenopsylla spp., Ceratophyllus spp.
Aus der Ordnung der Heteropterida z.B. Cimex spp., Triatoma spp., Rhodnius spp., Panstrongylus spp.
Aus der Ordnung der Blattarida z.B. Blatta orientalis, Periplaneta americana, Blattela germanica, Supella spp.
Aus der Unterklasse der Acaria (Acarida) und den Ordnungen der Meta- sowie Mesostigmata z.B. Argas spp., Ornithodorus spp., Otobius spp., Ixodes spp., Amblyomma spp., Boophilus spp., Dermacentor spp., Haemophysalis spp., Hyalomma spp., Rhipicephalus spp., Dermanyssus spp., Raillietia spp., Pneumonyssus spp., Stemostoma spp., Varroa spp.
Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändem, Halftern, Markierungsvorrichtungen usw.

Die Herstellung und die Verwendung der erfindungsgemäßen Wirkstoffe gehen aus den nachfolgenden Beispielen hervor.

### Herstellungsbeispiele:

### Beispiel I-1:

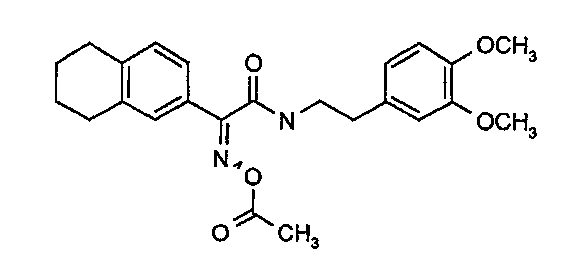

1,91 g (5 mmol) N-[(3,4-Dimethoxyphenyl)-2-ethyl]-2-hydroxyimino-2-(2-tetrahydronaphthyl)-essigsäureamid werden in 20 ml Methylenchlorid gelöst und bei -5°C 0,785 g (10 mmol) Acetylchlorid, gelöst in 5 ml Methylenchlorid, zugetropft. Man läßt 10 Minuten nachrühren, tropft dann eine Lösung von 1,4 ml (10 mmol) Triethylamin in 5 ml Methylenchlorid zu und rührt weitere 2 Stunden bei 0°C nach. Nachdem sich das Gemisch auf Raumtemperatur erwärmt hat, wird mehrmals mit Wasser gewaschen, die organische Phase über Natriumsulfat getrocknet und das Lösungsmittel bei vermindertem Druck abdestilliert.

Man erhält 2,0 g (95 % der Theorie) N-[(3,4-Dimethoxyphenyl)-2-ethyl]-2-acetoxyimino-2-(2-tetrahydronaphthyl)-essigsäureamid als farbloses Öl.

1H-NMR: δ (ppm) = 2,13 (s, 3H).

### Herstellung des Ausgangsstoffes:

### Beispiel I-II-1

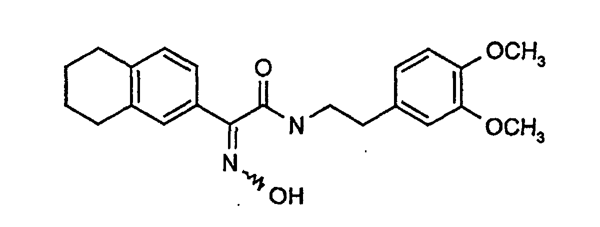

16,8 g (0,0679 mol) 2-Hydroximino-2-(2-tetrahydronaphthyl)-essigsäureethylester, 12,3 g (0,0679 mol) 2-(3,4-Dimethoxyphenyl)-ethylamin und 24,4g (0,136 mol) 30%-ige Natriummethylatlösung werden in 100 ml Methanol 16 Stunden unter Rückfluß zum Sieden erhitzt. Nach Abdestillieren des Lösungsmittels wird der Rückstand in Dichlormethan aufgenommen und mehrmals mit Wasser gewaschen. Die organische Phase wird über Natriumsulfat getrocknet und das Lösungsmittel bei vermindertem Druck abdestilliert.

Man erhält 21,4 g (82,3 % der Theorie) N-[(3,4-Dimethoxyphenyl)-2-ethyl]-2-hydroxyimino-2-(2-tetrahydronaphthyl)-essigsäureamid als Feststoff mit einem Schmelzpunkt von 104°C.

Analog dem Beispielen I-1 sowie entsprechend der allgemeinen Beschreibung des erfindungsgemäßen Herstellungsverfahrens, können beispielsweise auch die in der nachstehenden Tabelle I-8 aufgeführten Verbindungen hergestellt werden:

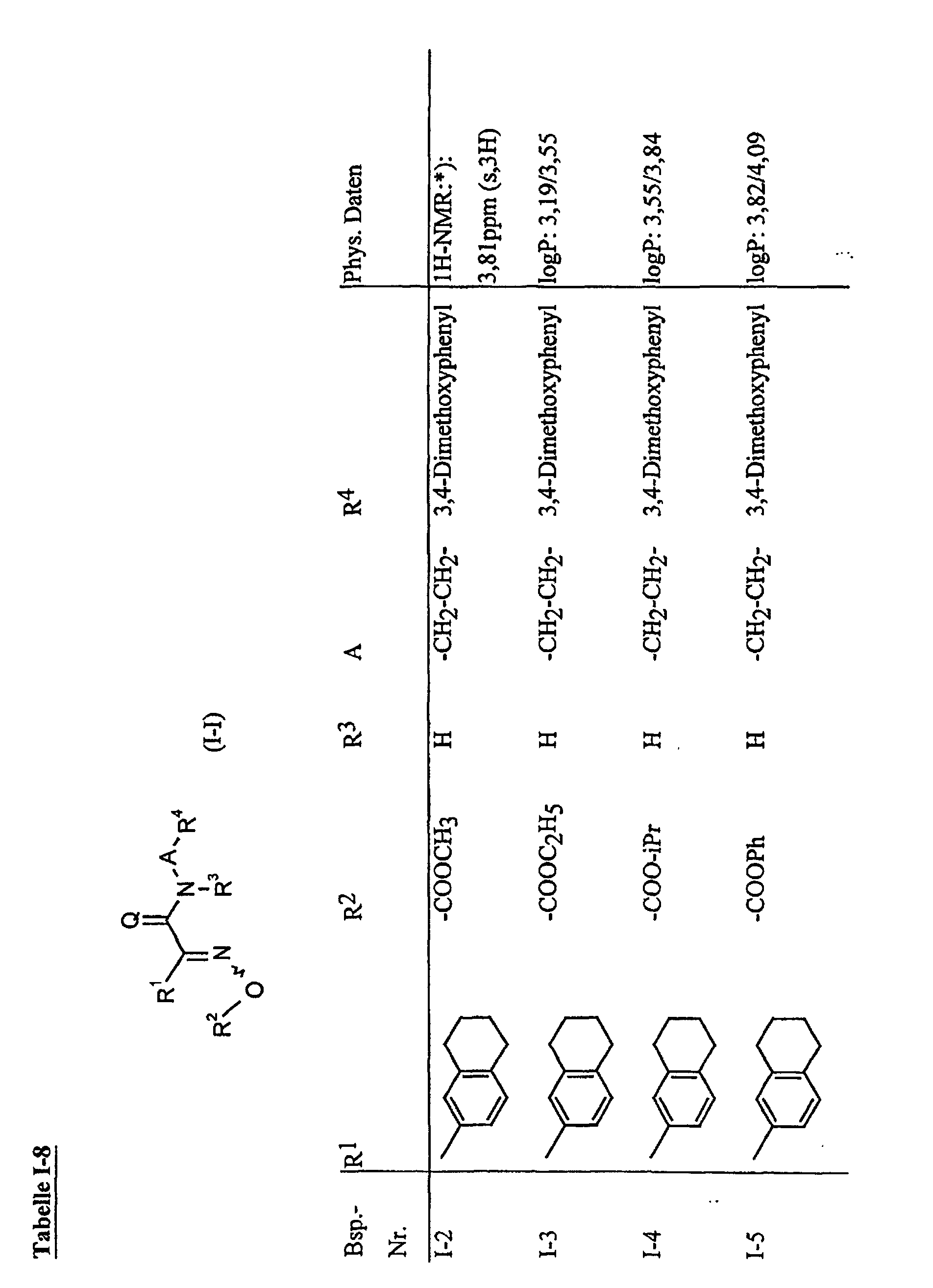

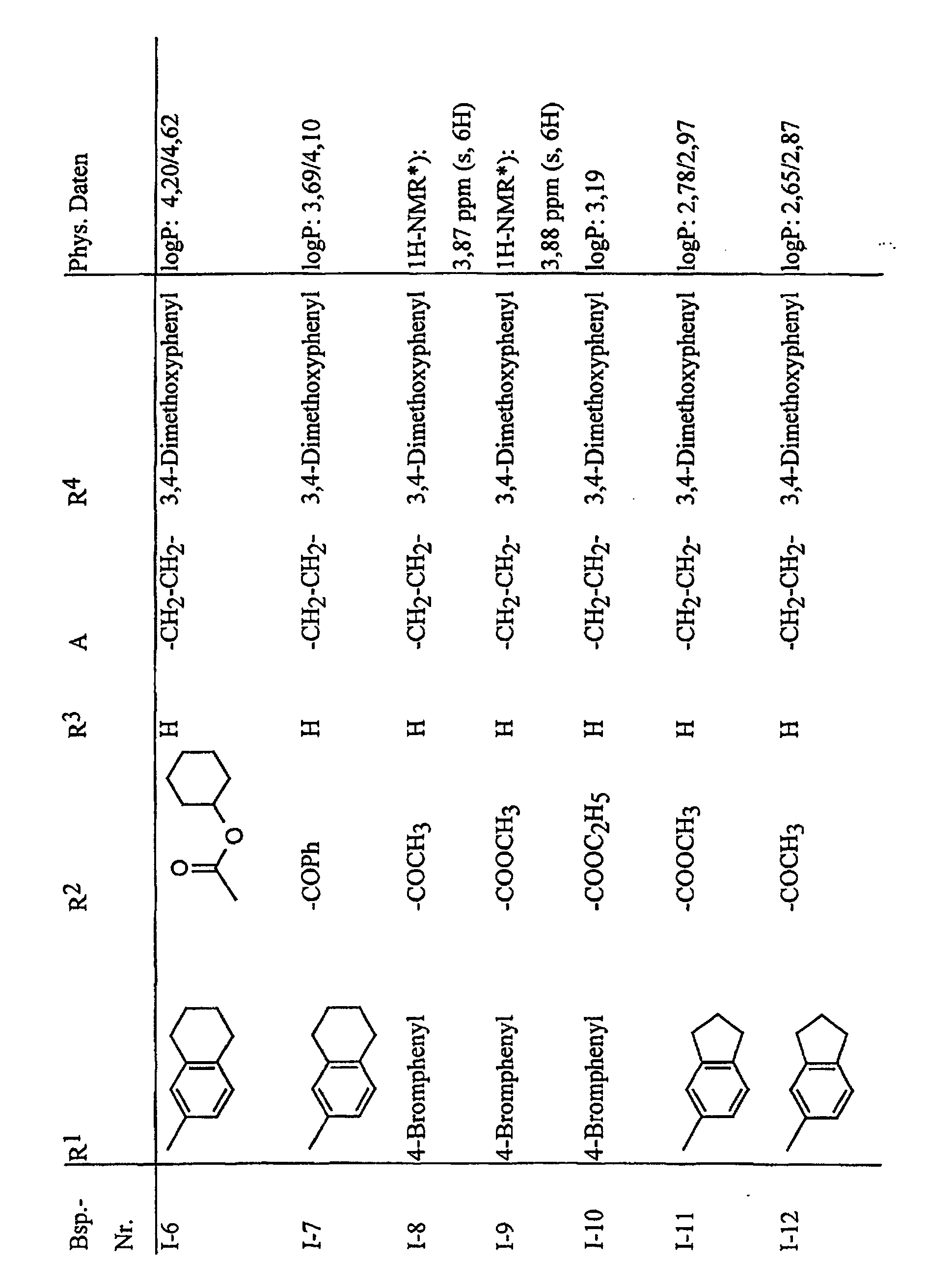

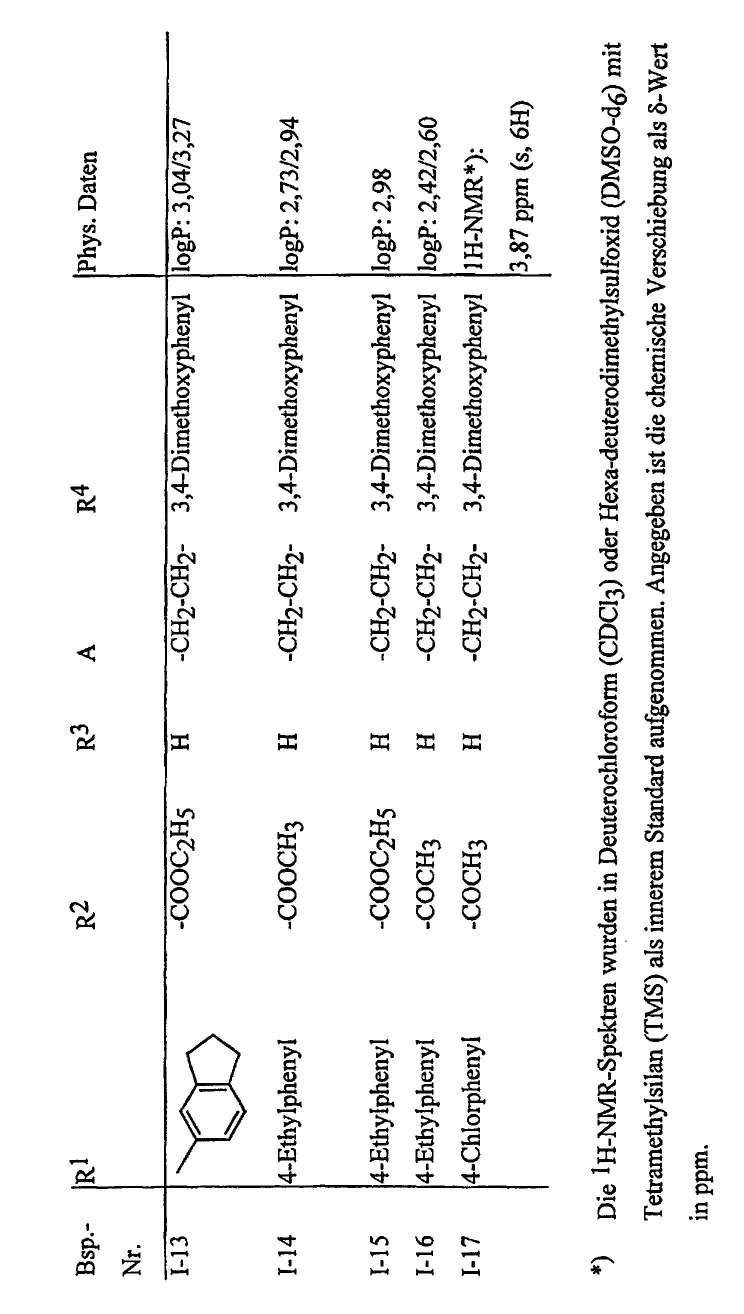

### Anwendungsbeispiele:

### Beispiel I-A

### Phytophthora-Test (Tomate) / protektiv

| | |
|---|---|
| Lösungsmittel | 47 Gewichtsteile Aceton |
| Emulgator | 3 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung taufeucht besprüht. Nach Antrocknen des Spritzbelages werden die Pflanzen mit einer wäßrigen Sporensuspension von Phytophthora infestans inokuliert.

Die Pflanzen werden in einer Inkubationskabine bei ca. 20°C und 100 % relativer Luftfeuchtigkeit aufgestellt.

3 Tage nach der Inokulation erfolgt die Auswertung. Dabei bedeutet ein Wirkungsgrad von 0 %, daß der Befall auf den behandelten Pflanzen demjenigen der unbehandelten Kontrolle entspricht, während ein Wirkungsgrad von 100 % bedeutet, daß auf den behandelten Pflanzen kein Befall beobachtet wird.

Bei diesem Test zeigt z.B. die folgende Verbindung (I-1) der Herstellungsbeispiele bei einer beispielhaften Wirkstoffaufwandmenge von 50 g/ha einen Wirkungsgrad von 95 % gegenüber der unbehandelten Kontrolle.

### Beispiel I-B

### Tetranychus-Test (OP-resistent/Tauchbehandlung)

| | |
|---|---|
| Lösungsmittel | 7,5 Gewichtsteile Dimethylformamid 100 Gewichtsteile Methanol |
| Emulgator | 2,5 Gewichtsteile Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit emulgatorhaltigem Wasser auf die gewünschten Konzentrationen.

Bohnenpflanzen (Phaseolus vulgaris), die stark von allen Stadien der gemeinen Spinnmilbe (Tetranychus urticae) befallen sind, werden in eine Wirkstoffzubereitung der gewünschten Konzentration getaucht.

Nach der gewünschten Zeit wird die Wirkung in % bestimmt. Dabei bedeutet 100 %, daß alle Spinnmilben abgetötet wurden; 0 % bedeutet, daß keine Spinnmilben abgetötet wurden.

In diesem Test bewirkt z.B. die Verbindung (I-14) der Herstellungsbeispiele bei einer beispielhaften Wirkstoffkonzentration von 0,05 % nach 7 Tagen eine Abtötung von 100 %.

## Patentansprüche

1. Verbindungen der allgemeinen Formel (I-I) , in welcher 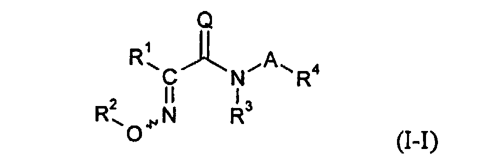
A für eine Einfachbindung oder für gegebenenfalls einfach oder mehrfach, gleich oder verschieden substituiertes Alkylen mit 1 bis 6 Kohlenstoffatomen steht, wobei die möglichen Substituenten aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen, substituiertes Aryl oder Heterocyclyl,
Q für Sauerstoff oder Schwefel steht,
R¹ für jeweils gegebenenfalls einfach oder mehrfach, gleich oder verschieden substituiertes Aryl, Cycloalkyl oder Cycloalkenyl mit 3 bis 7 Kohlenstoffatomen, oder Heterocyclyl mit 3 bis 12 Ringgliedern steht, wobei die möglichen Substituenten aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 8 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 8 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
substituiertes Aryl, Aryloxy, Arylthio, Arylalkyl, Arylalkyloxy, Arylalkylthio, Heterocyclyl, Heterocyclyloxy, Heterocyclylthio, Heterocyclylalkyl, Heterocyclylalkyloxy oder Heterocyclylalkylthio.
R² für eine der folgenden Gruppierungen steht: 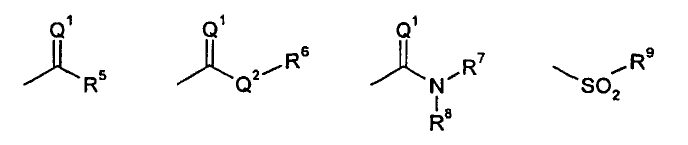 worin
Q¹ und Q² unabhängig voneinander für Sauerstoff oder Schwefel stehen,
R⁵ für Wasserstoff oder Alkyl mit 1 bis 6 Kohlenstoffatomen oder für jeweils gegebenenfalls im Arylteil durch Halogen, Cyano, Nitro, Alkyl oder Alkoxy mit jeweils 1 bis 4 Kohlenstoffatomen substituiertes Aryl oder Arylalkyl mit 1 bis 4 Kohlenstoffatomen im Alkylteil steht,
R⁶ für Alkyl mit 1 bis 6 Kohlenstoffatomen oder für jeweils gegebenenfalls im Arylteil durch Halogen, Cyano, Nitro, Alkyl oder Alkoxy mit jeweils 1 bis 4 Kohlenstoffatomen substituiertes Aryl oder Arylalkyl mit 1 bis 4 Kohlenstoffatomen im Alkylteil steht,
R⁷ für Wasserstoff, Alkyl mit 1 bis 6 Kohlenstoffatomen oder für jeweils gegebenenfalls im Arylteil durch Halogen, Cyano, Nitro, Alkyl oder Alkoxy mit jeweils 1 bis 4 Kohlenstoffatomen substituiertes Aryl oder Arylalkyl mit 1 bis 4 Kohlenstoffatomen im Alkylteil steht,
R⁸ für Alkyl mit 1 bis 6 Kohlenstoffatomen oder für jeweils gegebenenfalls im Arylteil durch Halogen, Cyano, Nitro, Alkyl oder Alkoxy mit jeweils 1 bis 4 Kohlenstoffatomen substituiertes Aryl oder Arylalkyl mit 1 bis 4 Kohlenstoffatomen im Alkylteil steht, oder
R⁷ und R⁸ gemeinsam mit dem Stickstoffatom, an das sie gebunden sind, für einen gegebenenfalls durch Methyl substituierten, heterocyclischen Ring stehen,
R⁹ für Alkyl mit 1 bis 6 Kohlenstoffatomen, Dialkylamino mit jeweils 1 bis 4 Kohlenstoffatomen in den einzelnen Alkylteilen, über Stickstoff gebundenes Heterocyclyl mit 3 bis 7 Ringgliedern oder für jeweils gegebenenfalls im Arylteil durch Halogen, Cyano, Nitro, Alkyl oder Alkoxy mit jeweils 1 bis 4 Kohlenstoffatomen substituiertes Aryl oder Arylalkyl mit 1 bis 4 Kohlenstoffatomen im Alkylteil steht,
R³ für Wasserstoff oder Alkyl mit 1 bis 4 Kohlenstoffatomen steht,
R⁴ für jeweils gegebenenfalls einfach oder mehrfach, gleich oder verschieden substituiertes Aryl, Cycloalkyl oder Cycloalkenyl mit 3 bis 8 Kohlenstoffatomen, oder Heterocyclyl mit 3 bis 12 Ringgliedern steht, wobei die möglichen Substituenten aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
substituiertes Aryl, Aryloxy, Arylthio, Arylalkyl, Arylalkyloxy, Arylalkylthio, Heterocyclyl, Heterocyclyloxy, Heterocyclylthio, Heterocyclylalkyl, Heterocyclylalkyloxy oder Heterocyclylalkylthio.

2. Verbindungen der Formel (I-I) gemäß Anspruch 1, in welcher
A für eine Einfachbindung oder für jeweils gegebenenfalls einfach oder mehrfach, gleich oder verschieden durch Fluor, Chlor, Cyano oder Methoxy substituiertes Methylen, 1,1-Ethylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methyl-propylen) steht,
Q für Sauerstoff oder Schwefel steht,
R¹ für jeweils gegebenenfalls einfach bis dreifach substituiertes Cyclopropyl, Cyclobutyl, Cyclopentyl oder Cyclohexyl, Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, n-Pentyl, n-Hexyl, n-Heptyl, Methoxy, Ethoxy, n-oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinoethyl oder Ethoximinoethyl;
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy;
Cyclopropyl, Cyclobutyl, Cyclopentyl oder Cyclohexyl;
gegebenenfalls durch die obengenannten Substituenten substituiertes Phenyl, Benzyl, Phenoxy, Benzyloxy,
R² für eine der folgenden Gruppierungen steht: 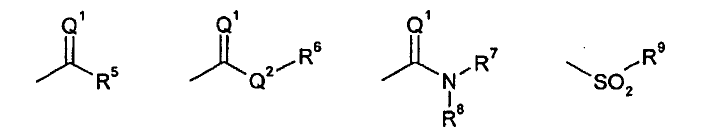 worin
Q¹ und Q² unabhängig voneinander für Sauerstoff oder Schwefel stehen,
R⁵ für Wasserstoff, Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁶ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s-oder t-Butyl, Methoxy oder Ethoxy substituiertes Phenyl, Benzyl, 1-Phenylethyl oder 2-Phenylethyl steht,
R⁷ für Wasserstoff, Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁸ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s-oder t-Butyl, Methoxy oder Ethoxy substituiertes Phenyl, Benzyl, 1-Phenylethyl oder 2-Phenylethyl steht, oder
R⁷ und R⁸ gemeinsam mit dem Stickstoffatom, an das sie gebunden sind, für einen gegebenenfalls durch Methyl substituierten, heterocyclischen Ring stehen,
R⁹ für Methyl, Ethyl, n- oder i-Propyl, n-, i-, s- oder t-Butyl, Dimethylamino, Diethylamino, Pyrrolidin-1-yl, Morpholin-N-yl, Piperidin-1-yl, oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R³ für Wasserstoff oder für Methyl oder Ethyl steht,
R⁴ für jeweils gegebenenfalls einfach bis dreifach substituiertes Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl.

3. Verbindungen der Formel (I-I) gemäß Anspruch 1, in welcher
A für eine Einfachbindung oder für gegebenenfalls einfach oder mehrfach, gleich oder verschieden durch Fluor, Chlor, Cyano, Methoxy substituiertes Methylen, 1,1-Ethylen, 1,2-Ethylen 1,1-, 1,2-, 1,3-oder 2,2-Propylen, 1,1-, 1,2-, 1,3-, 1,4-, 2,2-, 2,3-Butylen oder 1,1-, 1,2- oder 1,3-(2-Methyl-propylen) steht,
Q für Sauerstoff oder Schwefel steht,
R¹ für jeweils gegebenenfalls einfach bis sechsfach substituiertes Cyclobutyl, Cyclopentyl oder Cyclohexyl, wobei als Substituenten die untengenannten bevorzugt sind;
für jeweils gegebenenfalls einfach bis dreifach substituiertes Phenyl, Naphthyl, Furyl, Benzofuranyl, Thienyl, Benzothienyl, Pyridyl, Chinolyl, Tetrahydrofuryl oder Perhydropyranyl steht, wobei die möglichen Substituenten aus der nachstehenden Aufzählung ausgewählt sind:
Fluor, Chlor, Brom, Cyano, Nitro, Methyl, Ethyl, n- oder i-Propyl, n-, i-, s- oder t-Butyl, n-Pentyl, n-Hexyl, n-Heptyl, Methoxy, Ethoxy, n-oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinoethyl oder Ethoximinoethyl;
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy; Cyclopropyl, Cyclopentyl, Cyclohexyl; gegebenenfalls durch die obengenannten Substituenten substituierter Phenyl, Phenoxy, Benzyloxy;
R² für eine der folgenden Gruppierungen steht: 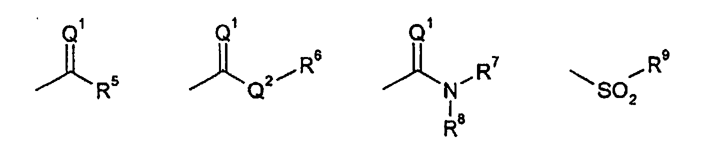 worin
Q¹ und Q² unabhängig voneinander für Sauerstoff oder Schwefel stehen,
R⁵ für Wasserstoff, Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁶ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s-oder t-Butyl, Methoxy oder Ethoxy substituiertes Phenyl, Benzyl, 1-Phenylethyl oder 2-Phenylethyl steht,
R⁷ für Wasserstoff, Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁸ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s-oder t-Butyl, Methoxy oder Ethoxy substituiertes Phenyl, Benzyl, 1-Phenylethyl oder 2-Phenylethyl steht, oder
R⁷ und R⁸ gemeinsam mit dem Stickstoffatom, an das sie gebunden sind, für einen gegebenenfalls durch Methyl substituierten, heterocyclischen Ring stehen,
R⁹ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s-oder t-Butyl, Methoxy oder Ethoxy substituiertes Phenyl, Benzyl, 1-Phenylethyl oder 2-Phenylethyl steht,
R³ für Wasserstoff oder für Methyl steht,
R⁴ für jeweils gegebenenfalls einfach bis dreifach substituiertes Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinoethyl oder Ethoximinoethyl, jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl.

4. Verbindungen der Formel (I-I) gemäß Anspruch 1, in welcher
A für eine Einfachbindung oder für Methylen, 1,1-Ethylen, 1,2-Ethylen, 1,1-Propylen, 1,2-Propylen, 1,3-Propylen oder 2,2-Propylen steht,
Q für Sauerstoff steht,
R¹ für gegebenenfalls durch Brom, Chlor, Fluor, Nitro, Methylsulfonyl, Phenyl, Phenyloxy, Benzyloxy, Cyclopropyl, Cyclohexyl, Methyl, Ethyl, n- oder i-Propyl, n-, i-, s- oder t-Butyl, n-Pentyl, n-Hexyl, n-Heptyl, Methoxy, Ethoxy, Trifluormethyl, Trifluormethoxy und/oder Methylthio einfach oder zweifach substituiertes Phenyl, Thienyl oder Furanyl steht oder für jeweils gegebenenfalls durch Fluor substituiertes 3,4-Methylen- und Ethylendioxo, Propan-1,3-diyl und Butan-1,4-diyl substituiertes Phenyl steht oder für Naphthyl, Benzofuranyl oder Benzothienyl steht,
R² für eine der folgenden Gruppierungen steht: 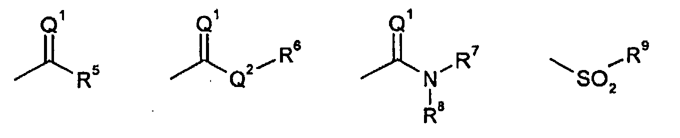 worin
Q¹ und Q² für Sauerstoff stehen,
R⁵ für Wasserstoff, Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁶ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s-oder t-Butyl, Methoxy oder Ethoxy substituiertes Phenyl, Benzyl, 1-Phenylethyl oder 2-Phenylethyl steht,
R⁷ für Wasserstoff, Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁸ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s-oder t-Butyl, Methoxy oder Ethoxy substituiertes Phenyl, Benzyl, 1-Phenylethyl oder 2-Phenylethyl steht, oder
R⁷ und R⁸ gemeinsam mit dem Stickstoffatom, an das sie gebunden sind, für einen gegebenenfalls durch Methyl substituierten, heterocyclischen Ring stehen,
R⁹ für Methyl, Ethyl, n- oder i-Propyl, n-, i-, s- oder t-Butyl, Dimethylamino, Diethylamino, Pyrrolidin-1-yl, Morpholin-N-yl, Piperidin-1-yl, oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R³ für Wasserstoff oder Methyl steht,
R⁴ für Cyclohexyl oder gegebenenfalls einfach bis dreifach substituiertes Phenyl, Thienyl, Furyl, Benzofuryl, Benzothienyl, Pyridyl, Pyrimidinyl, Naphthyl, Chinolyl steht, wobei die möglichen Substituenten aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Triflüormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy steht.

5. Verbindungen der Formel (I-I) , in welcher 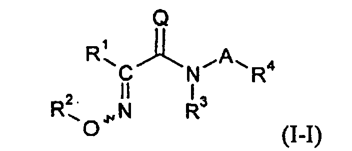
A für eine Einfachbindung oder für gegebenenfalls einfach oder mehrfach, gleich oder verschieden substituiertes Alkylen mit 1 bis 6 Kohlenstoffatomen steht, wobei die möglichen Substituenten aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen, substituiertes Aryl oder Heterocyclyl,
Q für Sauerstoff oder Schwefel steht,
R¹ für gegebenenfalls einfach oder mehrfach, gleich oder verschieden substituiertes, am Benzolring verknüpftes, Benzoheterocyclyl mit 3 bis 12 Ringgliedern im Heterocyclylteil und einem, zwei oder drei Heteroatomen (wobei jedoch höchstens ein Heteroatom für Sauerstoff steht) steht, wobei die möglichen Substituenten aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 8 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 8 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
substituiertes Aryl, Aryloxy, Arylthio, Arylalkyl, Arylalkyloxy, Arylalkylthio, Heterocyclyl, Heterocyclyloxy, Heterocyclylthio, Heterocyclylalkyl, Heterocyclylalkyloxy oder Heterocyclylalkylthio;
R² für eine der folgenden Gruppierungen steht: 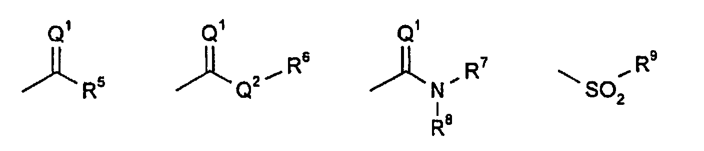 worin
Q¹ und Q² unabhängig voneinander für Sauerstoff oder Schwefel stehen,
R⁵ für Wasserstoff oder Alkyl mit 1 bis 6 Kohlenstoffatomen oder für jeweils gegebenenfalls im Arylteil durch Halogen, Cyano, Nitro, Alkyl oder Alkoxy mit jeweils 1 bis 4 Kohlenstoffatomen substituiertes Aryl oder Arylalkyl mit 1 bis 4 Kohlenstoffatomen im Alkylteil steht,
R⁶ für Alkyl mit 1 bis 6 Kohlenstoffatomen oder für jeweils gegebenenfalls im Arylteil durch Halogen, Cyano, Nitro, Alkyl oder Alkoxy mit jeweils 1 bis 4 Kohlenstoffatomen substituiertes Aryl oder Arylalkyl mit 1 bis 4 Kohlenstoffatomen im Alkylteil steht,
R⁷ für Wasserstoff, Alkyl mit 1 bis 6 Kohlenstoffatomen oder für jeweils gegebenenfalls im Arylteil durch Halogen, Cyano, Nitro, Alkyl oder Alkoxy mit jeweils 1 bis 4 Kohlenstoffatomen substituiertes Aryl oder Arylalkyl mit 1 bis 4 Kohlenstoffatomen im Alkylteil steht,
R⁸ für Alkyl mit 1 bis 6 Kohlenstoffatomen oder für jeweils gegebenenfalls im Arylteil durch Halogen, Cyano, Nitro, Alkyl oder Alkoxy mit jeweils 1 bis 4 Kohlenstoffatomen substituiertes Aryl oder Arylalkyl mit 1 bis 4 Kohlenstoffatomen im Alkylteil steht, oder
R⁷ und R⁸ gemeinsam mit dem Stickstoffatom, an das sie gebunden sind, für einen gegebenenfalls durch Methyl substituierten, heterocyclischen Ring stehen,
R⁹ für Alkyl mit 1 bis 6 Kohlenstoffatomen, Dialkylamino mit jeweils 1 bis 4 Kohlenstoffatomen in den einzelnen Alkylteilen, über Stickstoff gebundenes Heterocyclyl mit 3 bis 7 Ringgliedern oder für jeweils gegebenenfalls im Arylteil durch Halogen, Cyano, Nitro, Alkyl oder Alkoxy mit jeweils 1 bis 4 Kohlenstoffatomen substituiertes Aryl oder Arylalkyl mit 1 bis 4 Kohlenstoffatomen im Alkylteil steht,
R³ für Wasserstoff oder Alkyl mit 1 bis 4 Kohlenstoffatomen steht,
R⁴ für jeweils gegebenenfalls einfach oder mehrfach, gleich oder verschieden substituiertes Aryl, Cycloalkyl oder Cycloalkenyl mit 3 bis 8 Kohlenstoffatomen, oder Heterocyclyl mit 3 bis 12 Ringgliedern steht, wobei die möglichen Substituenten aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
substituiertes Aryl, Aryloxy, Arylthio, Arylalkyl, Arylalkyloxy, Arylalkylthio, Heterocyclyl, Heterocyclyloxy, Heterocyclylthio, Heterocyclylalkyl, Heterocyclylalkyloxy oder Heterocyclylalkylthio.

6. Verbindungen der Formel (I-I) gemäß Anspruch 5, in welcher
A für eine Einfachbindung oder für jeweils gegebenenfalls einfach oder mehrfach, gleich oder verschieden durch Fluor, Chlor, Cyano oder Methoxy substituiertes Methylen, 1,1-Ethylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methyl-propylen)steht,
Q für Sauerstoff oder Schwefel steht,
R¹ für eine der nachstehenden Gruppierungen 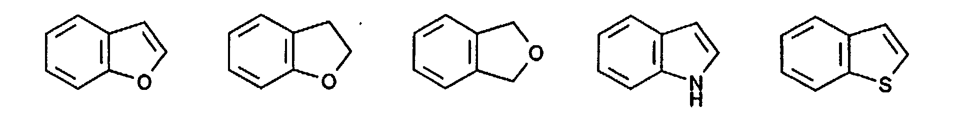 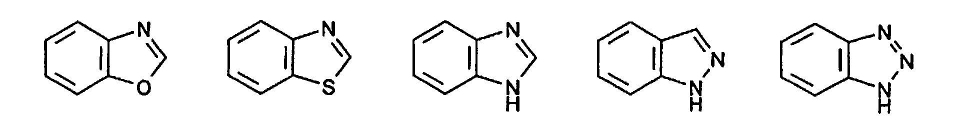 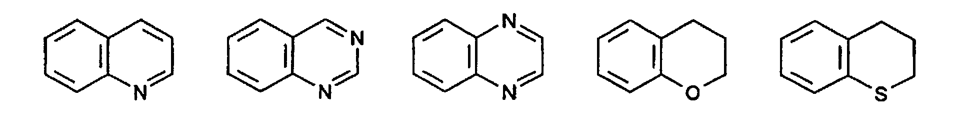 steht, die jeweils über ein Kohlenstoffatom des jeweiligen Benzolringes verbunden sind und die jeweils gegebenenfalls einfach bis dreifach, gleich oder verschieden durch die nachfolgend aufgezählten Substituenten substituiert sein können:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinoethyl oder Ethoximinoethyl;
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy;
Cyclopropyl, Cyclobutyl, Cyclopentyl oder Cyclohexyl;
gegebenenfalls durch die obengenannten Substituenten substituiertes Phenyl, Benzyl, Phenoxy oder Benzyloxy, Cyclopropyl, Cyclobutyl, Cyclopentyl oder Cyclohexyl,
R² für eine der folgenden Gruppierungen steht: 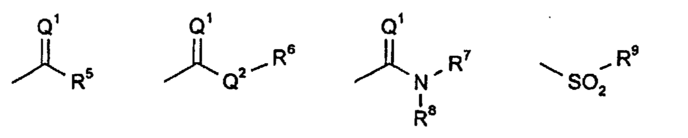 worin
Q¹ und Q² unabhängig voneinander für Sauerstoff oder Schwefel stehen,
R⁵ für Wasserstoff oder Methyl, Ethyl, n- oder i-Propyl, n-, i-, s-oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁶ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s-oder t-Butyl, Methoxy oder Ethoxy substituiertes Phenyl, Benzyl, 1-Phenylethyl oder 2-Phenylethyl steht,
R⁷ für Wasserstoff, Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁸ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s-oder t-Butyl, Methoxy oder Ethoxy substituiertes Phenyl, Benzyl, 1-Phenylethyl oder 2-Phenylethyl steht, oder
R⁷ und R⁸ gemeinsam mit dem Stickstoffatom, an das sie gebunden sind, für einen gegebenenfalls durch Methyl substituierten, heterocyclischen Ring stehen,
R⁹ für Methyl, Ethyl, n- oder i-Propyl, n-, i-, s- oder t-Butyl, Dimethylamino, Diethylamino, Pyrrolidin-1-yl, Morpholin-N-yl, Piperidin-1-yl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R³ für Wasserstoff oder für Methyl oder Ethyl steht,
R⁴ für jeweils gegebenenfalls einfach bis dreifach substituiertes Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl,, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl.

7. Verbindungen der Formel (I-I) gemäß Anspruch 5, in welcher
A für eine Einfachbindung oder für gegebenenfalls einfach oder mehrfach, gleich oder verschieden durch Fluor, Chlor, Cyano, Methoxy substituiertes Methylen, 1,1-Ethylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methyl-propylen) steht,
Q für Sauerstoff oder Schwefel steht,
R¹ für eine der nachstehenden Gruppierungen 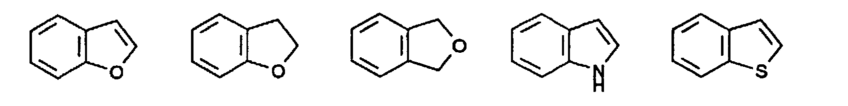 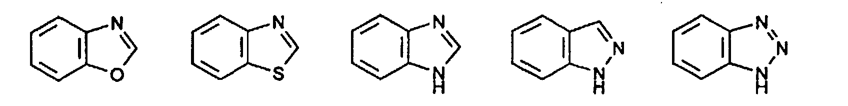 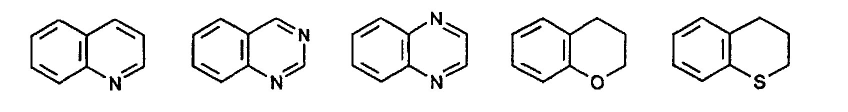 steht, die jeweils über ein Kohlenstoffatom des jeweiligen Benzolringes verbunden sind und die jeweils gegebenenfalls einfach bis dreifach, gleich oder verschieden durch die nachfolgend aufgezählten Substituenten substituiert sein können:
Fluor, Chlor, Brom, Cyano, Nitro, Methyl, Ethyl, n- oder i-Propyl, n-, i-, s- oder t-Butyl, n-Pentyl, n-Hexyl, n-Heptyl, Methoxy, Ethoxy, n-oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, , Methoximinoethyl oder Ethoximinoethyl;
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy; Cyclopropyl, Cyclopentyl, Cyclohexyl; gegebenenfalls durch die obengenannten Substituenten substituiertes Phenyl, Phenoxy, Benzyloxy;
R² für eine der folgenden Gruppierungen steht: 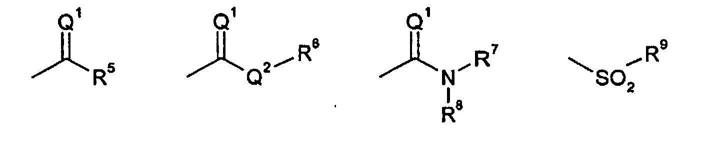 worin
Q¹ und Q² unabhängig voneinander für Sauerstoff oder Schwefel stehen,
R⁵ für Wasserstoff oder Methyl, Ethyl, n- oder i-Propyl, n-, i-, s-oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁶ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s-oder t-Butyl, Methoxy oder Ethoxy substituiertes Phenyl, Benzyl, 1-Phenylethyl oder 2-Phenylethyl steht,
R⁷ für Wasserstoff, Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁸ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s-oder t-Butyl, Methoxy oder Ethoxy substituiertes Phenyl, Benzyl, 1-Phenylethyl oder 2-Phenylethyl steht, oder
R⁷ und R⁸ gemeinsam mit dem Stickstoffatom, an das sie gebunden sind, für einen gegebenenfalls durch Methyl substituierten, heterocyclischen Ring stehen,
R⁹ für Methyl, Ethyl, n- oder i-Propyl, n-, i-, s- oder t-Butyl, Dimethylamino, Diethylamino, Pyrrohdin-1-yl, Morpholin-N-yl, Piperidin-1-yl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R³ für Wasserstoff oder für Methyl steht,
R⁴ für jeweils gegebenenfalls einfach bis dreifach substituiertes Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinoethyl oder Ethoximinoethyl, jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl.

8. Verbindungen der Formel (I-I) gemäß Anspruch 5, in welcher
A für eine Einfachbindung oder für Methylen, 1,1-Ethylen, 1,2-Ethylen, 1,1-Propylen, 1,2-Propylen, 1,3-Propylen oder 2,2-Propylen steht,
Q für Sauerstoff steht,
R¹ für eine der nachstehenden Gruppierungen 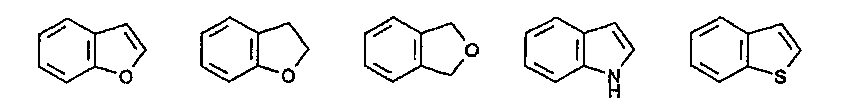 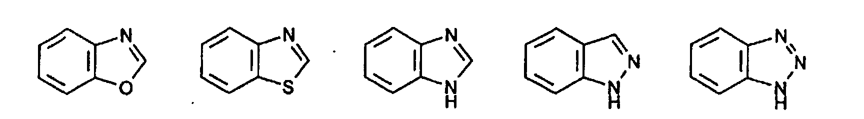 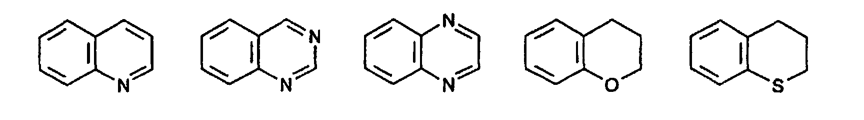 steht, die jeweils über ein Kohlenstoffatom des jeweiligen Benzolringes verbunden sind und die jeweils gegebenenfalls einfach bis dreifach, gleich oder verschieden durch die nachfolgend aufgezählten Substituenten substituiert sein können:
Brom, Chlor, Fluor, Nitro, Methylsulfonyl, Phenyl, Phenyloxy, Benzyloxy, Cyclopropyl, Cyclohexyl, Methyl, Ethyl, n- oder i-Propyl, n-, i-, s- oder t-Butyl, n-Pentyl, n-Hexyl, n-Heptyl, Methoxy, Ethoxy, Trifluormethyl, Trifluormethoxy und/oder Methylthio,
R² für eine der folgenden Gruppierungen steht: 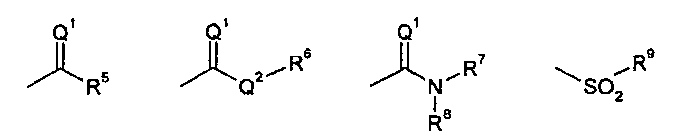 worin
Q¹ und Q² für Sauerstoff stehen,
R⁵ für Wasserstoff oder Methyl, Ethyl, n- oder i-Propyl, n-, i-, s-oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁶ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s-oder t-Butyl, Methoxy oder Ethoxy substituiertes Phenyl, Benzyl, 1-Phenylethyl oder 2-Phenylethyl steht,
R⁷ für Wasserstoff, Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes. Phenyl, Benzyl, 1-Phenylethyl oder 2-Phenylethyl steht,
R⁸ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s-oder t-Butyl, Methoxy oder Ethoxy substituiertes Phenyl, Benzyl, 1-Phenylethyl oder 2-Phenylethyl steht, oder
R⁷ und R⁸ gemeinsam mit dem Stickstoffatom, an das sie gebunden sind, für einen gegebenenfalls durch Methyl substituierten, heterocyclischen Ring stehen,
R⁹ für Methyl, Ethyl, n- oder i-Propyl, n-, i-, s- oder t-Butyl, Dimethylamino, Diethylamino, Pyrrolidin-1-yl, Morpholin-N-yl, Piperidin-1-yl oder für jeweils gegebenenfalls im Phenylteil durch Fludr, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R³ für Wasserstoff oder Methyl steht,
R⁴ für Cyclohexyl oder gegebenenfalls einfach bis dreifach substituertes Phenyl, Thienyl, Furyl, Benzofuryl, Benzothienyl, Pyridyl, Pyrimidinyl, Naphthyl, Chinolyl steht, wobei die möglichen Substituenten aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy steht.

9. Verbindungen der Formel (I-I) , in welcher 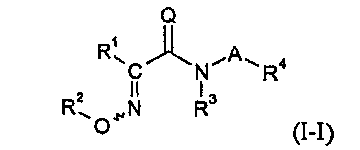
A für eine Einfachbindung oder für gegebenenfalls einfach oder mehrfach, gleich oder verschieden substituiertes Alkylen mit 1 bis 6 Kohlenstoffatomen steht, wobei die möglichen Substituenten aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen, substituiertes Aryl oder Heterocyclyl,
Q für Sauerstoff oder Schwefel steht,
R¹ für einen Tricyclus 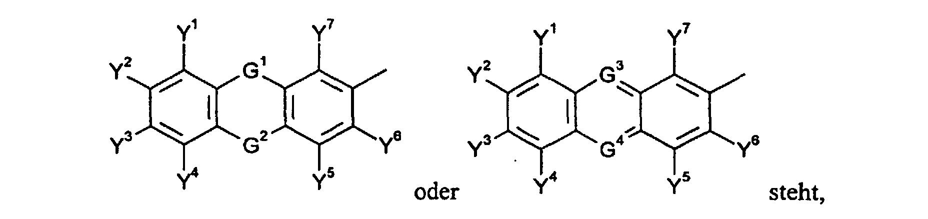 worin
G¹ und G² unabhängig voneinander für eine Einfachbindung, Alkandiyl, Alkendiyl, Sauerstoff, Schwefel, -NH-, -N(Alkyl)- oder Carbonyl stehen,
G³ und G⁴ unabhängig voneinander für Stickstoff oder eine Gruppierung 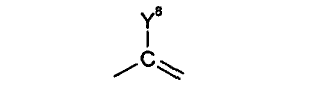 stehen und
Y¹, Y², Y³, Y⁴, Y⁵, Y⁶, Y⁷ und Y⁸ unabhängig voneinander für Wasserstoff, Halogen, Cyano, Nitro; Alkyl, Alkoxy, Alkylamino, Dialkylamino, Alkylthio, Alkylsulfinyl oder Alkylsulfonyl mit jeweils 1 bis 8 Kohlenstoffatomen in den einzelnen Alkylteilen;
Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen oder
Cycloalkyl mit 3 bis 6 Kohlenstoffatomen stehen,
R² für eine der folgenden Gruppierungen steht: 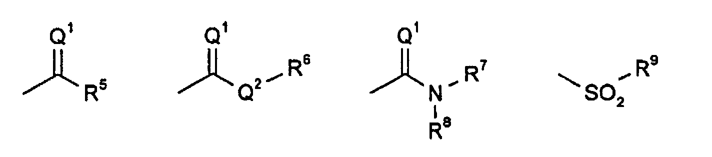 worin
Q¹ und Q² unabhängig voneinander für Sauerstoff oder Schwefel stehen,
R⁵ für Wasserstoff oder Alkyl mit 1 bis 6 Kohlenstoffatomen oder für jeweils gegebenenfalls im Arylteil durch Halogen, Cyano, Nitro, Alkyl oder Alkoxy mit jeweils 1 bis 4 Kohlenstoffatomen substituiertes Aryl oder Arylalkyl mit 1 bis 4 Kohlenstoffatomen im Alkylteil steht,
R⁶ für Alkyl mit 1 bis 6 Kohlenstoffatomen oder für jeweils gegebenenfalls im Arylteil durch Halogen, Cyano, Nitro, Alkyl oder Alkoxy mit jeweils 1 bis 4 Kohlenstoffatomen substituiertes Aryl oder Arylalkyl mit 1 bis 4 Kohlenstoffatomen im Alkylteil steht,
R⁷ für Wasserstoff, Alkyl mit 1 bis 6 Kohlenstoffatomen oder für jeweils gegebenenfalls im Arylteil durch Halogen, Cyano, Nitro, Alkyl oder Alkoxy mit jeweils 1 bis 4 Kohlenstoffatomen substituiertes Aryl oder Arylalkyl mit 1 bis 4 Kohlenstoffatomen im Alkylteil steht,
R⁸ für Alkyl mit 1 bis 6 Kohlenstoffatomen oder für jeweils gegebenenfalls im Arylteil durch Halogen, Cyano, Nitro, Alkyl oder Alkoxy mit jeweils 1 bis 4 Kohlenstoffatomen substituiertes Aryl oder Arylalkyl mit 1 bis 4 Kohlenstoffatomen im Alkylteil steht, oder
R⁷ und R⁸ gemeinsam mit dem Stickstoffatom, an das sie gebunden sind, für einen gegebenenfalls durch Methyl substituierten, heterocyclischen Ring stehen,
R⁹ für Alkyl mit 1 bis 6 Kohlenstoffatomen, Dialkylamino mit jeweils 1 bis 4 Kohlenstoffatomen in den einzelnen Alkylteilen, über Stickstoff gebundenes Heterocyclyl mit 3 bis 7 Ringgliedem oder für jeweils gegebenenfalls im Arylteil durch Halogen, Cyano, Nitro, Alkyl oder Alkoxy mit jeweils 1 bis 4 Kohlenstoffatomen substituiertes Aryl oder Arylalkyl mit 1 bis 4 Kohlenstoffatomen im Alkylteil steht,
R³ für Wasserstoff oder Alkyl mit 1 bis 4 Kohlenstoffatomen steht,
R⁴ für jeweils gegebenenfalls einfach oder mehrfach, gleich oder verschieden substituiertes Aryl, Cycloalkyl oder Cycloalkenyl mit 3 bis 8 Kohlenstoffatomen, oder Heterocyclyl mit 3 bis 12 Ringgliedern steht, wobei die möglichen Substituenten aus der nachstehenden Aufzählung ausgewählt sind:
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen;
Cycloalkyl mit 3 bis 6 Kohlenstoffatomen;
sowie jeweils gegebenenfalls einfach oder mehrfach, gleich oder verschieden durch
Halogen, Cyano und/oder geradkettiges oder verzweigtes Alkyl mit 1 bis 4 Kohlenstoffatomen
und/oder geradkettiges oder verzweigtes Halogenalkyl mit 1 bis 4 Kohlenstoffatomen und 1 bis 9 gleichen oder verschiedenen Halogenatomen
und/oder geradkettiges oder verzweigtes Alkoxy oder Alkylthio mit 1 bis 4 Kohlenstoffatomen
und/oder geradkettiges oder verzweigtes Halogenalkoxy oder Halogenalkylthio mit 1 bis 4 Kohlenstoffatomen und 1 bis 9 gleichen oder verschiedenen Halogenatomen
und/oder gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
substituiertes Aryl, Aryloxy, Arylthio, Arylalkyl, Arylalkyloxy, Arylalkylthio, Heterocyclyl, Heterocyclyloxy, Heterocyclylthio, Heterocyclylalkyl, Heterocyclylalkyloxy oder Heterocyclylalkylthio.

10. Verbindungen der Formel (I-I) gemäß Anspruch 9, in welcher
A für eine Einfachbindung oder für jeweils gegebenenfalls einfach oder mehrfach, gleich oder verschieden durch Fluor, Chlor, Cyano oder Methoxy substituiertes Methylen, 1,1-Ethylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methyl-propylen) steht,
Q für Sauerstoff oder Schwefel steht,
R¹ für einen Tricyclus 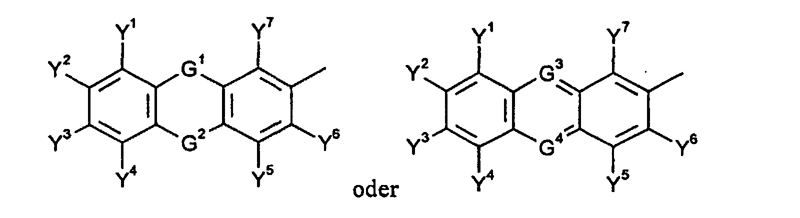 steht,
worin
G¹ und G² unabhängig voneinander für eine Einfachbindung, Alkandiyl, Alkendiyl, Sauerstoff, Schwefel, -NH-, -N(Alkyl)- oder Carbonyl stehen und
G³ und G⁴ unabhängig voneinander für Stickstoff oder eine Gruppierung 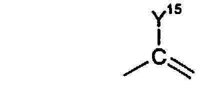 stehen und
Y¹, Y², Y³, Y⁴, Y⁵, Y⁶, Y⁷ und Y⁸ unabhängig voneinander für Wasserstoff, Fluor, Chlor, Brom, Cyano, Nitro, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Cyclopropyl, Cyclobutyl, Cyclopentyl oder Cyclohexyl stehen,
R² für eine der folgenden Gruppierungen steht: 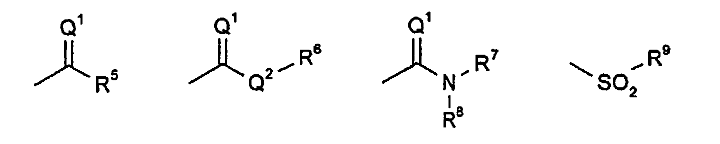 worin
Q¹ und Q² unabhängig voneinander für Sauerstoff oder Schwefel stehen,
R⁵ für Wasserstoff oder Methyl, Ethyl, n- oder i-Propyl, n-, i-, s-oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁶ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s-oder t-Butyl, Methoxy oder Ethoxy substituiertes Phenyl, Benzyl, 1-Phenylethyl oder 2-Phenylethyl steht,
R⁷ für Wasserstoff, Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁸ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s-oder t-Butyl, Methoxy oder Ethoxy substituiertes Phenyl, Benzyl, 1-Phenylethyl oder 2-Phenylethyl steht, oder
R⁷ und R⁸ gemeinsam mit dem Stickstoffatom, an das sie gebunden sind, für einen gegebenenfalls durch Methyl substituierten, heterocyclischen Ring stehen,
R⁹ für Methyl, Ethyl, n- oder i-Propyl, n-, i-, s- oder t-Butyl, Dimethylamino, Diethylamino, Pyrrolidin-1-yl, Morpholin-N-yl, Piperidin-1-yl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R³ für Wasserstoff oder für Methyl oder Ethyl steht,
R⁴ für jeweils gegebenenfalls einfach bis dreifach substituiertes Cyclopropyl, Cyclobutyl, Cyclopentyl oder Cyclohexyl, Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n-oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl.

11. Verbindungen der Formel (I-I) gemäß Anspruch 9, in welcher
A für eine Einfachbindung oder für gegebenenfalls einfach oder mehrfach, gleich oder verschieden durch Fluor, Chlor, Cyano, Methoxy substituiertes Methylen, 1,1-Ethylen, 1,2-Ethylen 1,1-, 1,2-, 1,3- oder 2,2-Propylen, 1,1-, 1,2-, 1,3-, 1,4-, 2,2-, 2,3-Butylen oder 1,1-, 1,2- oder 1,3-(2-Methyl-propylen) steht,
Q für Sauerstoff oder Schwefel steht,
R¹ für einen Tricyclus 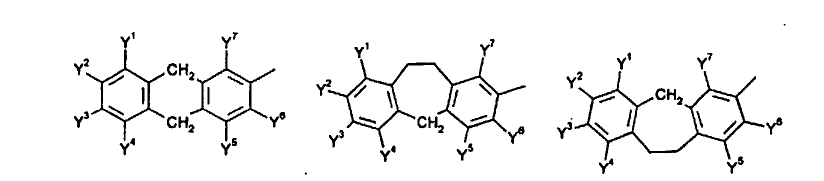 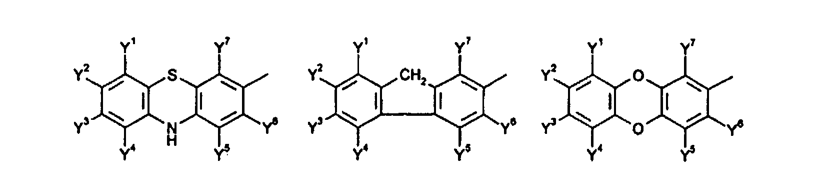 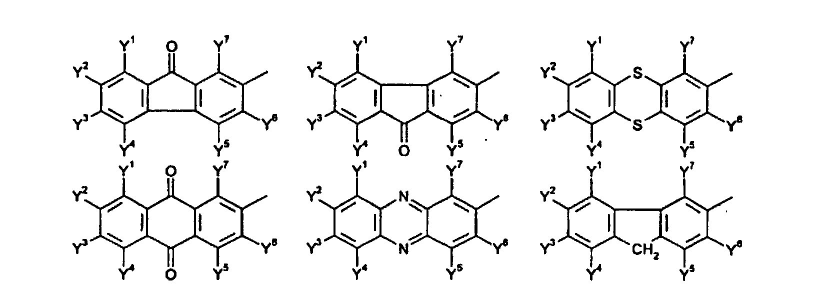 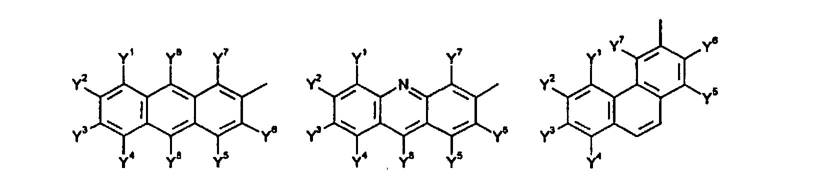 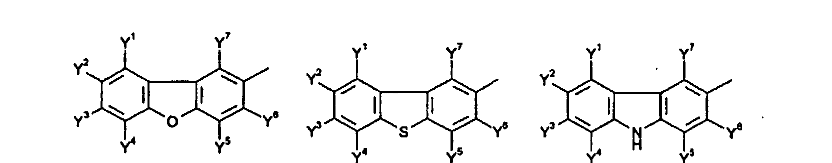 steht, worin
Y¹, Y², Y³, Y⁴, Y⁵, Y⁶, Y⁷ und Y⁸ unabhängig voneinander für Wasserstoff, Fluor, Chlor, Brom, Cyano, Nitro, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Cyclopropyl, Cyclobutyl, Cyclopentyl oder Cyclohexyl stehen,
R² für eine der folgenden Gruppierungen steht: 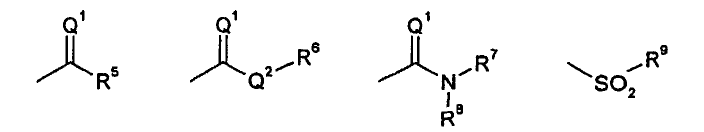 worin
Q¹ und Q² unabhängig voneinander für Sauerstoff oder Schwefel stehen,
R⁵ für Wasserstoff oder Methyl, Ethyl, n- oder i-Propyl, n-, i-, s-oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁶ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s-oder t-Butyl, Methoxy oder Ethoxy substituiertes Phenyl, Benzyl, 1-Phenylethyl oder 2-Phenylethyl steht,
R⁷ für Wasserstoff, Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁸ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s-oder t-Butyl, Methoxy oder Ethoxy substituiertes Phenyl, Benzyl, 1-Phenylethyl oder 2-Phenylethyl steht, oder
R⁷ und R⁸ gemeinsam mit dem Stickstoffatom, an das sie gebunden sind, für einen gegebenenfalls durch Methyl substituierten, heterocyclischen Ring stehen,
R⁹ für Methyl, Ethyl, n- oder i-Propyl, n-, i-, s- oder t-Butyl, Dimethylamino, Diethylamino, Pyrrolidin-1-yl, Morpholin-N-yl, Piperidin-1-yl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R³ für Wasserstoff oder für Methyl steht,
R⁴ für jeweils gegebenenfalls einfach bis dreifach substituiertes Phenyl, Naphthyl, Furyl, Benzofuranyl, Pyrrolyl, Indolyl, Thienyl, Benzothienyl, Oxazolyl, Isoxazolyl, Thiazolyl, Isothiazolyl, Oxadiazolyl, Thiadiazolyl, Pyridyl, Chinolyl, Pyrimidyl, Pyridazinyl, Pyrazinyl, Oxiranyl, Oxetanyl, Tetrahydrofuryl, Perhydropyranyl, Pyrrolidinyl, Piperidinyl oder Morpholinyl steht, wobei die möglichen Substituenten aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinoethyl oder Ethoximinoethyl, jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy,
Cyclopropyl, Cyclobutyl, Cyclopentyl oder Cyclohexyl.

12. Verbindungen der Formel (I-I) gemäß Anspruch 9, in welcher
A für eine Einfachbindung oder für Methylen, 1,1-Ethylen, 1,2-Ethylen, 1,1-Propylen, 1,2-Propylen, 1,3-Propylen oder 2,2-Propylen steht,
Q für Sauerstoff steht,
R¹ für einen Tricyclus 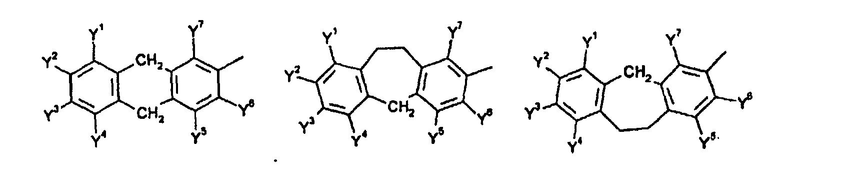 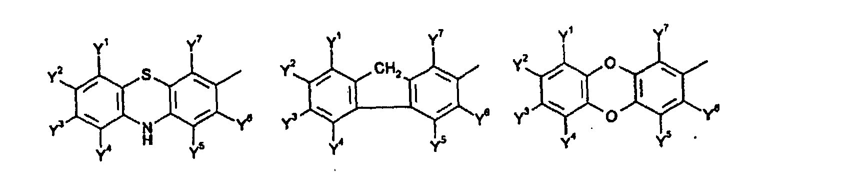 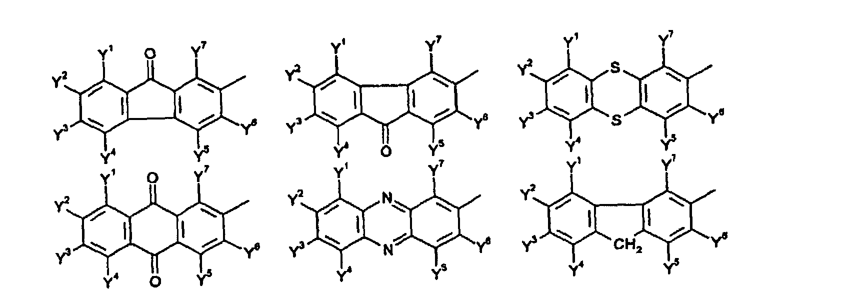 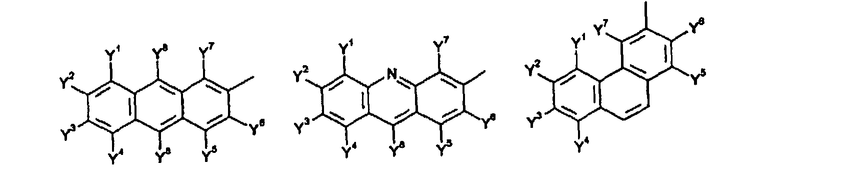 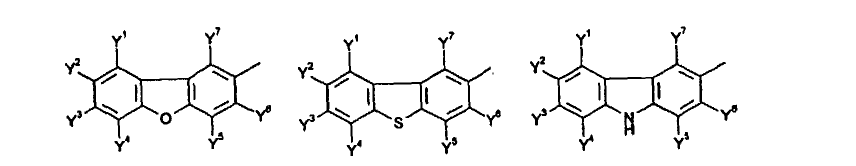 steht, worin
Y¹, Y², Y³, Y⁴, Y⁵, Y⁶, Y⁷ und Y⁸ unabhängig voneinander für Wasserstoff, Fluor, Chlor, Brom, Cyano, Nitro, Methyl, Ethyl, n- oder i-Propyl, n-, i-, s- oder t-Butyl, n-Pentyl, n-Hexyl, n-Heptyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Cyclopropyl, Cyclobutyl, Cyclopentyl oder Cyclohexyl stehen,
R² für eine der folgenden Gruppierungen steht: 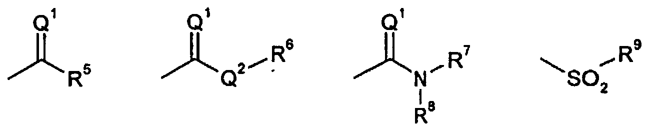 worin
Q¹ und Q² für Sauerstoff stehen,
R⁵ für Wasserstoff oder Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁶ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R⁷ für Wasserstoff, Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i- Propyl, n-, i-, s- oder t-Butyl, Methoxy oder Ethoxy substituiertes Phenyl, Benzyl, 1-Phenylethyl oder 2-Phenylethyl steht,
R⁸ für Methyl, Ethyl, n- oder i-Propyl, n-, i-, s- oder t-Butyl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht, oder
R⁷ und R⁸ gemeinsam mit dem Stickstoffatom, an das sie gebunden sind, für einen gegebenenfalls durch Methyl substituierten, heterocyclischen Ring stehen,
R⁹ für Methyl, Ethyl, n- oder i-Propyl, n-, i-, s- oder t-Butyl, Dimethylamino, Diethylamino, Pyrrolidin-1-yl, Morpholin-N-yl, Piperidin-1-yl oder für jeweils gegebenenfalls im Phenylteil durch Fluor, Chlor, Brom, Cyano, Nitro, Methyl, Ethyl, n- oder i-Propyl, n-, i-, s- oder t-Butyl, Methoxy oder Ethoxy substituiertes Phenyl, Benzyl, 1-Phenylethyl oder 2-Phenylethyl steht,
R³ für Wasserstoff oder Methyl steht,
R⁴ für Cyclohexyl oder gegebenenfalls einfach bis dreifach substituiertes Phenyl, Thienyl, Furyl, Benzofuryl, Benzothienyl, Pyridyl, Pyrimidinyl, Naphthyl, Chinolyl steht, wobei die möglichen Substituenten aus der nachstehenden Aufzählung ausgewählt sind:
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinoethyl oder Ethoximinoethyl,
jeweils gegebenenfalls einfach oder mehrfach, gleich oder verschieden durch Fluor, Chlor, Methyl, Trifluormethyl, Ethyl, n- oder i-Propyl substituiertes, jeweils zweifach verknüpftes Trimethylen (Propan-1,3-diyl), Tetramethylen (Butan-1,4-diyl), Methylendioxy oder Ethylendioxy steht.

13. Schädlingsbekämpfungsmittel, **gekennzeichnet durch** einen Gehalt an mindestens einer Verbindung der Formel (I) gemäß Anspruch 1.

14. Verfahren zur Bekämpfung von Schädlingen, **dadurch gekennzeichnet, daß** man Verbindungen der Formel (I) nach Anspruch 1 auf Schädlinge und/oder ihren Lebensraum einwirken läßt.

15. Verwendungen von Verbindungen nach den Ansprüchen 1 bis 21 zur Bekämpfung von Schädlingen.

16. Verfahren zur Herstellung von Schädlingsbekämpfungsmitteln, **dadurch gekennzeichnet, daß** man Verbindungen nach den Ansprüchen 1 bis 21 mit Streckmitteln und/oder oberflächenaktiven Mitteln vermischt.

## Claims

1. Compounds of the general formula (I-I) 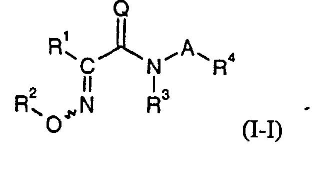 in which
A represents a single bond or represents alkylene having 1 to 6 carbon atoms which is optionally mono- or polysubstituted by identical or different substituents, the possible substituents being selected from the list below:
halogen, cyano, nitro, amino, hydroxyl, formyl, carboxyl, carbamoyl, thiocarbamoyl;
alkoxy, alkylthio, alkylsulphinyl or alkylsulphonyl having in each case 1 to 6 carbon atoms and being in each case straight-chain or branched;
alkenyl or alkenyloxy having in each case 2 to 6 carbon atoms and being in each case straight-chain or branched;
halogenoalkoxy, halogenoalkylthio, halogenoalkylsulphinyl or halogenoalkylsulphonyl having in each case 1 to 6 carbon atoms and 1 to 13 identical or different halogen atoms and being in each case straight-chain or branched;
halogenoalkenyl or halogenoalkenyloxy having in each case 2 to 6 carbon atoms and 1 to 13 identical or different halogen atoms and being in each case straight-chain or branched;
alkylamino, dialkylamino, alkylcarbonyl, alkylcarbonyloxy, alkoxycarbonyl, alkylsulphonyloxy, hydroximinoalkyl or alkoximinoalkyl having in each case 1 to 6 carbon atoms in the individual alkyl moieties and being in each case straight-chain or branched;
cycloalkyl having 3 to 6 carbon atoms;
and also aryl or heterocyclyl, each of which is optionally mono- or polysubstituted by identical or different substituents selected from the group consisting of
halogen, cyano and straight-chain or branched alkyl having 1 to 4 carbon atoms
and straight-chain or branched halogenoalkyl having 1 to 4 carbon atoms and 1 to 9 identical or different halogen atoms
and straight-chain or branched alkoxy or alkylthio having 1 to 4 carbon atoms
and straight-chain or branched halogenoalkoxy or halogenoalkylthio having 1 to 4 carbon atoms and 1 to 9 identical or different halogen atoms
and alkylene or dioxyalkylene having in each case 1 to 6 carbon atoms and being in each case doubly attached and optionally mono- or polysubstituted by identical or different substituents selected from the group consisting of halogen and straight-chain or branched alkyl having 1 to 4 carbon atoms
and straight-chain or branched halogenoalkyl having 1 to 4 carbon atoms and 1 to 9 identical or different halogen atoms,
Q represents oxygen or sulphur,
R¹ represents aryl, cycloalkyl or cycloalkenyl having 3 to 7 carbon atoms, or heterocyclyl having 3 to 12 ring members, each of which is optionally mono- or polysubstituted by identical or different substituents, the possible substituents being selected from the list below:
halogen, cyano, nitro, amino, hydroxyl, formyl, carboxyl, carbamoyl, thiocarbamoyl;
alkyl, alkoxy, alkylthio, alkylsulphinyl or alkylsulphonyl having in each case 1 to 8 carbon atoms and being in each case straight-chain or branched;
alkenyl or alkenyloxy having in each case 2 to 6 carbon atoms and being in each case straight-chain or branched;
halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms and being in each case straight-chain or branched;
halogenoalkenyl or halogenoalkenyloxy having in each case 2 to 6 carbon atoms and 1 to 13 identical or different halogen atoms and being in each case straight-chain or branched;
alkylamino, dialkylamino, alkylcarbonyl, alkylcarbonyloxy, alkoxycarbonyl, alkylsulphonyloxy, hydroximinoalkyl or alkoximinoalkyl having in each case 1 to 8 carbon atoms in the individual alkyl moieties and being in each case straight-chain or branched;
alkylene or dioxyalkylene having in each case 1 to 6 carbon atoms and being in each case doubly attached and in each case optionally mono- or polysubstituted by identical or different substituents selected from the group consisting of halogen and straight-chain or branched alkyl having 1 to 4 carbon atoms and straight-chain or branched halogenoalkyl having 1 to 4 carbon atoms and 1 to 9 identical or different halogen atoms;
cycloalkyl having 3 to 6 carbon atoms;
and also aryl, aryloxy, arylthio, arylalkyl, arylalkyloxy, arylalkylthio, heterocyclyl, heterocyclyloxy, heterocyclylthio, heterocyclylalkyl, heterocyclylalkyloxy or heterocyclylalkylthio, each of which is optionally mono- or polysubstituted by identical or different substituents selected from the group consisting of
halogen, cyano and straight-chain or branched alkyl having 1 to 4 carbon atoms
and straight-chain or branched halogenoalkyl having 1 to 4 carbon atoms and 1 to 9 identical or different halogen atoms
and straight-chain or branched alkoxy or alkylthio having 1 to 4 carbon atoms
and straight-chain or branched halogenoalkoxy or halogenoalkylthio having 1 to 4 carbon atoms and 1 to 9 identical or different halogen atoms
and alkylene or dioxyalkylene having in each case 1 to 6 carbon atoms and being in each case doubly attached and optionally mono- or polysubstituted by identical or different substituents selected from the group consisting of halogen and straight-chain or branched alkyl having 1 to 4 carbon atoms and straight-chain or branched halogenoalkyl having 1 to 4 carbon atoms and 1 to 9 identical or different halogen atoms.
R² represents one of the groupings below: 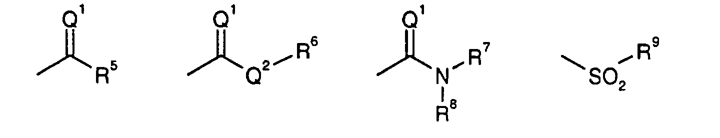 in which
Q¹ and Q² independently of one another each represent oxygen or sulphur,
R⁵ represents hydrogen or alkyl having 1 to 6 carbon atoms or represents aryl or arylalkyl having 1 to 4 carbon atoms in the alkyl moiety, each of which is optionally substituted in the aryl moiety by halogen, cyano, nitro, alkyl or alkoxy having in each case 1 to 4 carbon atoms,
R⁶ represents alkyl having 1 to 6 carbon atoms or represents aryl or arylalkyl having 1 to 4 carbon atoms in the alkyl moiety, each of which is optionally substituted in the aryl moiety by halogen, cyano, nitro, alkyl or alkoxy having in each case 1 to 4 carbon atoms,
R⁷ represents hydrogen, alkyl having 1 to 6 carbon atoms or represents aryl or arylalkyl having 1 to 4 carbon atoms in the alkyl moiety, each of which is optionally substituted in the aryl moiety by halogen, cyano, nitro, alkyl or alkoxy having in each case 1 to 4 carbon atoms,
R⁸ represents alkyl having 1 to 6 carbon atoms or represents aryl or arylalkyl having 1 to 4 carbon atoms in the alkyl moiety, each of which is optionally substituted in the aryl moiety by halogen, cyano, nitro, alkyl or alkoxy having in each case 1 to 4 carbon atoms, or
R⁷ and R⁸ together with the linking nitrogen atom represent an optionally methyl-substituted heterocyclic ring,
R⁹ represents alkyl having 1 to 6 carbon atoms, dialkylamino having in each case 1 to 4 carbon atoms in the individual alkyl moieties, heterocyclyl having 3 to 7 ring members which is attached via nitrogen or represents aryl or arylalkyl having 1 to 4 carbon atoms in the alkyl moiety, each of which is optionally substituted in the aryl moiety by halogen, cyano, nitro, alkyl or alkoxy having in each case 1 to 4 carbon atoms,
R³ represents hydrogen or alkyl having 1 to 4 carbon atoms,
R⁴ represents aryl, cycloalkyl or cycloalkenyl having 3 to 8 carbon atoms, or heterocyclyl having 3 to 12 ring members, each of which is optionally mono- or polysubstituted by identical or different substituents, the possible substituents being selected from the list below:
halogen, cyano, nitro, amino, hydroxyl, formyl, carboxyl, carbamoyl, thiocarbamoyl;
alkyl, alkoxy, alkylthio, alkylsulphinyl or alkylsulphonyl having in each case 1 to 6 carbon atoms and being in each case straight-chain or branched;
alkenyl or alkenyloxy having in each case 2 to 6 carbon atoms and being in each case straight-chain or branched;
halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms and being in each case straight-chain or branched;
halogenoalkenyl or halogenoalkenyloxy having in each case 2 to 6 carbon atoms and 1 to 13 identical or different halogen atoms and being in each case straight-chain or branched;
alkylamino, dialkylamino, alkylcarbonyl, alkylcarbonyloxy, alkoxycarbonyl, alkylsulphonyloxy, hydroximinoalkyl or alkoximinoalkyl having in each case 1 to 6 carbon atoms in the individual alkyl moieties and being in each case straight-chain or branched;
alkylene or dioxyalkylene having in each case 1 to 6 carbon atoms and being in each case doubly attached and being in each case optionally mono- or polysubstituted by identical or different substituents selected from the group consisting of halogen and straight-chain or branched alkyl having 1 to 4 carbon atoms and straight-chain or branched halogenoalkyl having 1 to 4 carbon atoms and 1 to 9 identical or different halogen atoms; cycloalkyl having 3 to 6 carbon atoms;
and also aryl, aryloxy, arylthio, arylalkyl, arylalkyloxy, arylalkylthio, heterocyclyl, heterocyclyloxy, heterocyclylthio, heterocyclylalkyl, heterocyclylalkyloxy or heterocyclylalkylthio, each of which is optionally mono- or polysubstituted by identical or different substituents selected from the group consisting of
halogen, cyano and straight-chain or branched alkyl having 1 to 4 carbon atoms
and straight-chain or branched halogenoalkyl having 1 to 4 carbon atoms and 1 to 9 identical or different halogen atoms
and straight-chain or branched alkoxy or alkylthio having 1 to 4 carbon atoms
and straight-chain or branched halogenoalkoxy or halogenoalkylthio having 1 to 4 carbon atoms and 1 to 9 identical or different halogen atoms
and alkylene or dioxyalkylene having in each case 1 to 6 carbon atoms and being in each case doubly attached and optionally mono- or polysubstituted by identical or different substituents selected from the group consisting of halogen and straight-chain or branched alkyl having 1 to 4 carbon atoms and straight-chain or branched halogenoalkyl having 1 to 4 carbon atoms and 1 to 9 identical or different halogen atoms.

2. Compounds of the formula (I-I) according to Claim 1, in which
A represents a single bond or represents methylene, 1,1-ethylene, 1,2-ethylene 1,1-, 1,2-, 1,3- or 2,2-propylene, 1,1-, 1,2-, 1,3-, 1,4-, 2,2-, 2,3-butylene or 1,1-, 1,2- or 1,3-(2-methyl-propylene), each of which is optionally mono- or polysubstituted by identical or different substituents selected from the group consisting of fluorine, chlorine, cyano and methoxy,
Q represents oxygen or sulphur,
R¹ represents cyclopropyl, cyclobutyl, cyclopentyl or cyclohexyl, phenyl, naphthyl, furyl, benzofuranyl, pyrrolyl, indolyl, thienyl, benzothienyl, oxazolyl, isoxazolyl, thiazolyl, isothiazolyl, oxadiazolyl, thiadiazolyl, pyridyl, quinolyl, pyrimidyl, pyridazinyl, pyrazinyl, oxiranyl, oxetanyl, tetrahydrofuryl, perhydropyranyl, pyrrolidinyl, piperidinyl or morpholinyl, each of which is optionally mono- to trisubstituted, the possible substituents being selected from the list below:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, n-pentyl, n-hexyl, n-heptyl, methoxy, ethoxy, n- or i-propoxy, methylthio, ethylthio; n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinoethyl or ethoximinoethyl;
trimethylene (propane-1,3-diyl), tetramethylene (butane-1,4-diyl), methylenedioxy or ethylenedioxy, each of which is doubly attached and each of which is optionally mono- or polysubstituted by identical or different substituents selected from the group consisting of fluorine, chlorine, methyl, trifluoromethyl, ethyl, n- and i-propyl;
cyclopropyl, cyclobutyl, cyclopentyl or cyclohexyl;
benzyl, phenoxy, benzyloxy or phenyl, which is optionally substituted by the abovementioned substituents,
R² represents one of the groupings below: 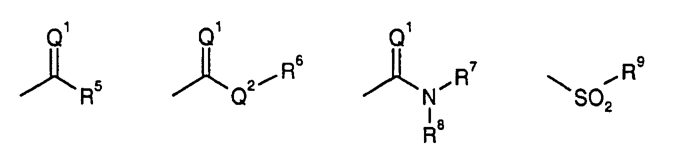 in which
Q¹ and Q² independently of one another each represent oxygen or sulphur,
R⁵ represents hydrogen, methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R⁶ represents methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R⁷ represents hydrogen, methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R⁸ represents methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy, or
R⁷ and R⁸ together with the linking nitrogen atom represent an optionally methyl-substituted heterocyclic ring,
R⁹ represents methyl, ethyl, n- or i-propyl, n-, i-, s- or t-butyl, dimethylamino, diethylamino, pyrrolidin-1-yl, morpholin-N-yl, piperidin-1-yl, or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R³ represents hydrogen or represents methyl or ethyl,
R⁴ represents phenyl, naphthyl, furyl, benzofuranyl, pyrrolyl, indolyl, thienyl, benzothienyl, oxazolyl, isoxazolyl, thiazolyl, isothiazolyl, oxadiazolyl, thiadiazolyl, pyridyl, quinolyl, pyrimidyl, pyridazinyl, pyrazinyl, oxiranyl, oxetanyl, tetrahydrofuryl, perhydropyranyl, pyrrolidinyl, piperidinyl or morpholinyl, each of which is optionally mono- to trisubstituted, the possible substituents being selected from the list below:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinoethyl or ethoximinoethyl,
trimethylene (propane-1,3-diyl), tetramethylene (butane-1,4-diyl), methylenedioxy or ethylenedioxy, each of which is doubly attached and each of which is optionally mono- or polysubstituted by identical or different substituents selected from the group consisting of fluorine, chlorine, methyl, trifluoromethyl, ethyl, n- and i-propyl, cyclopropyl, cyclobutyl, cyclopentyl or cyclohexyl.

3. Compounds of the formula (I-I) according to Claim 1, in which
A represents a single bond or represents methylene, 1,1-ethylene, 1,2-ethylene 1,1-, 1,2-, 1,3- or 2,2-propylene, 1,1-, 1,2-, 1,3-, 1,4-, 2,2-, 2,3-butylene or 1,1-, 1,2- or 1,3-(2-methyl-propylene), each of which is optionally mono- or polysubstituted by identical or different substituents selected from the group consisting of fluorine, chlorine, cyano, methoxy,
Q represents oxygen or sulphur,
R¹ represents cyclobutyl, cyclopentyl or cyclohexyl, each of which is optionally mono- to hexasubstituted, preterred substituents being those mentioned below;
represents phenyl, naphthyl, furyl, benzofuranyl, thienyl, benzothienyl, pyridyl, quinolyl, tetrahydrofuryl or perhydropyranyl, each of which is optionally mono- to trisubstituted, the possible substituents being selected from the list below:
fluorine, chlorine, bromine, cyano, nitro, methyl, ethyl, n- or i-propyl, n-, i-, s- or t-butyl, n-pentyl, n-hexyl, n-heptyl, methoxy, ethoxy, n- or i-propoxy, methylthio, ethylthio, n- or i-propylthio, methylsulphinyl, ethylsulphinyl, methyl sulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl, trifluoromethylsulphonyl, acetyl, propionyl, acetyloxy, methoxycarbonyl, ethoxycarbonyl, methylsulphonyloxy, ethylsulphonyloxy, hydroximinomethyl, hydroximinoethyl, methoximinoethyl or ethoximinoethyl;
trimethylene (propane-1,3-diyl), tetramethylene (butane-1,4-diyl), methylenedioxy or ethylenedioxy, each of which is doubly attached and each of which is optionally mono- or polysubstituted by identical or different substituents selected from the group consisting of fluorine, chlorine, methyl, trifluoromethyl, ethyl, n- and i-propyl; cyclopropyl, cyclopentyl, cyclohexyl; phenyl, phenoxy, benzyloxy, optionally substituted by the abovementioned substituents;
R² represents one of the groupings below: 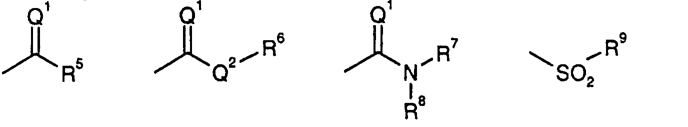 in which
Q¹ and Q² independently of one another each represent oxygen or sulphur,
R⁵ represents hydrogen, methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R⁶ represents methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R⁷ represents hydrogen, methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R⁸ represents methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy, or
R⁷ and R⁸ together with the linking nitrogen atom represent an optionally methyl-substituted heterocyclic ring,
R⁹ represents methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R³ represents hydrogen or represents methyl,
R⁴ represents phenyl, naphthyl, furyl, benzofuranyl, pyrrolyl, indolyl, thienyl, benzothienyl, oxazolyl, isoxazolyl, thiazolyl, isothiazolyl, oxadiazolyl, thiadiazolyl, pyridyl, quinolyl, pyrimidyl, pyridazinyl, pyrazinyl, oxiranyl, oxetanyl, tetrahydrofuryl, perhydropyranyl, pyrrolidinyl, piperidinyl or morpholinyl, each of which is optionally mono- to trisubstituted, the possible substituents being selected from the list below:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinoethyl or ethoximinoethyl, represents trimethylene (propane-1,3-diyl), tetramethylene (butane-1,4-diyl), methylenedioxy or ethylenedioxy, each of which is doubly attached and each of which is optionally mono- or polysubstituted by identical or different substituents selected from the group consisting of fluorine, chlorine, methyl, trifluoromethyl, ethyl, n- and i-propyl,
cyclopropyl, cyclobutyl, cyclopentyl or cyclohexyl.

4. Compounds of the formula (I-I) according to Claim 1, in which
A represents a single bond or represents methylene, 1,1-ethylene, 1,2-ethylene, 1,1-propylene, 1,2-propylene, 1,3-propylene or 2,2-propylene,
Q represents oxygen,
R¹ represents thienyl, furanyl or phenyl which is optionally mono- or disubstituted by bromine, chlorine, fluorine, nitro, methylsulphonyl, phenyl, phenyloxy, benzyloxy, cyclopropyl, cyclohexyl, methyl, ethyl, n- or i-propyl, n-, i-, s- or t-butyl, n-pentyl, n-hexyl, n-heptyl, methoxy, ethoxy, trifluoromethyl, trifluoromethoxy and/or methylthio or represents phenyl which is substituted by 3,4-methylene- and ethylenedioxo, propane-1,3-diyl and butane-1,4-diyl, each of which is optionally substituted by fluorine, or represents naphthyl, benzofuranyl or benzothienyl,
R² represents one of the groupings below: 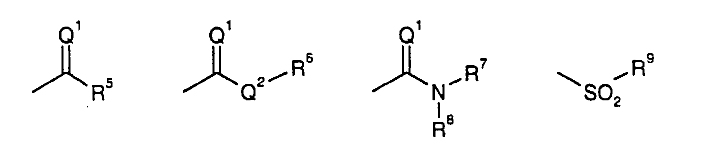 in which
Q¹ and Q² each represent oxygen,
R⁵ represents hydrogen, methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R⁶ represents methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R⁷ represents hydrogen, methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R⁸ represents methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy, or
R⁷ and R⁸ together with the linking nitrogen atom represent an optionally methyl-substituted heterocyclic ring,
R⁹ represents methyl, ethyl, n- or i-propyl, n-, i-, s- or t-butyl, dimethylamino, diethylamino, pyrrolidin-1-yl, morpholin-N-yl, piperidin-1-yl, or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R³ represents hydrogen or methyl,
R⁴ represents cyclohexyl or optionally mono- to trisubstituted phenyl, thienyl, furyl, benzofuryl, benzothienyl, pyridyl, pyrimidinyl, naphthyl, quinolyl, the possible substituents being selected from the list below:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamino, ethylamino, n- or i-propylamino, dimethylamino, diethylamino, acetyl, propionyl. acetyloxy, methoxycarbonyl, ethoxycarbonyl, methylsulphonyloxy, ethylsulphonyloxy, hydroximinomethyl, hydroximinoethyl, methoximinoethyl or ethoximinoethyl,
trimethylene (propane-1,3-diyl), tetramethylene (butane-1,4-diyl), methylenedioxy or ethylenedioxy, each of which is doubly attached and each of which is optionally mono- or polysubstituted by identical or different substituents selected from the group consisting of fluorine, chlorine, methyl, trifluoromethyl, ethyl, n- and i-propyl.

5. Compounds of the formula (I-I), 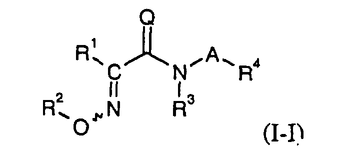 in which
A represents a single bond or represents alkylene having 1 to 6 carbon atoms which is optionally mono- or polysubstituted by identical or different substituents, the possible substituents being selected from the list below:
halogen, cyano, nitro, amino, hydroxyl, formyl, carboxyl, carbamoyl, thiocarbamoyl;
alkoxy, alkylthio, alkylsulphinyl or alkylsulphonyl having in each case 1 to 6 carbon atoms and being in each case straight-chain or branched;
alkenyl or alkenyloxy having in each case 2 to 6 carbon atoms and being in each case straight-chain or branched;
halogenoalkoxy, halogenoalkylthio, halogenoalkylsulphinyl or halogenoalkylsulphonyl having in each case 1 to 6 carbon atoms and 1 to 13 identical or different halogen atoms and being in each case straight-chain or branched;
halogenoalkenyl or halogenoalkenyloxy having in each case 2 to 6 carbon atoms and 1 to 13 identical or different halogen atoms and being in each case straight-chain or branched;
alkylamino, dialkylamino, alkylcarbonyl, alkylcarbonyloxy, alkoxycarbonyl, alkylsulphonyloxy, hydroximinoalkyl or alkoximinoalkyl having in each case 1 to 6 carbon atoms in the individual alkyl moieties and being in each case straight-chain or branched;
cycloalkyl having 3 to 6 carbon atoms;
and also aryl or heterocyclyl, each of which is optionally mono- or polysubstituted by identical or different substituents selected from the group consisting of
halogen, cyano and straight-chain or branched alkyl having 1 to 4 carbon atoms
and straight-chain or branched halogenoalkyl having 1 to 4 carbon atoms and 1 to 9 identical or different halogen atoms
and straight-chain or branched alkoxy or alkylthio having 1 to 4 carbon atoms
and straight-chain or branched halogenoalkoxy or halogenoalkylthio having 1 to 4 carbon atoms and 1 to 9 identical or different halogen atoms
and alkylene or dioxyalkylene having in each case 1 to 6 carbon atoms and being in each case doubly attached and optionally mono- or polysubstituted by identical or different substituents selected from the group consisting of halogen and straight-chain or branched alkyl having 1 to 4 carbon atoms
and straight-chain or branched halogenoalkyl having 1 to 4 carbon atoms and 1 to 9 identical or different halogen atoms,
Q represents oxygen or sulphur,
R¹ represents benzoheterocyclyl having 3 to 12 ring members in the heterocyclyl moiety and one, two or three heteroatoms (but at most one heteroatom representing oxygen) which is attached on the benzene ring and is optionally mono- or polysubstituted by identical or different substituents, the possible substituents being selected from the list below:
halogen, cyano, nitro, amino, hydroxyl, formyl, carboxyl, carbamoyl, thiocarbamoyl;
alkyl, alkoxy, alkylthio, alkylsulphinyl or alkylsulphonyl having in each case 1 to 8 carbon atoms and being in each case straight-chain or branched;
alkenyl or alkenyloxy having in each case 2 to 6 carbon atoms and being in each case straight-chain or branched;
halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms and being in each case straight-chain or branched;
halogenoalkenyl or halogenoalkenyloxy having in each case 2 to 6 carbon atoms and 1 to 13 identical or different halogen atoms and being in each case straight-chain or branched;
alkylamino, dialkylamino, alkylcarbonyl, alkylcarbonyloxy, alkoxycarbonyl, alkylsulphonyloxy, hydroximinoalkyl or alkoximinoalkyl having in each case 1 to 8 carbon atoms in the individual alkyl moieties and being in each case straight-chain or branched;
alkylene or dioxyalkylene having in each case 1 to 6 carbon atoms and being in each case doubly attached and in each case optionally mono- or polysubstituted by identical or different substituents selected from the group consisting of halogen and straight-chain or branched alkyl having 1 to 4 carbon atoms and straight-chain or branched halogenoalkyl having 1 to 4 carbon atoms and 1 to 9 identical or different halogen atoms;
cycloalkyl having 3 to 6 carbon atoms;
and also aryl, aryloxy, arylthio, arylalkyl, arylalkyloxy, arylalkylthio, heterocyclyl, heterocyclyloxy, heterocyclylthio, heterocyclylalkyl, heterocyclylalkyloxy or heterocyclylalkylthio, each of which is optionally mono- or polysubstituted by identical or different substituents selected from the group consisting of
halogen, cyano and straight-chain or branched alkyl having 1 to 4 carbon atoms
and straight-chain or branched halogenoalkyl having 1 to 4 carbon atoms and 1 to 9 identical or different halogen atoms
and straight-chain or branched alkoxy or alkylthio having 1 to 4 carbon atoms
and straight-chain or branched halogenoalkoxy or halogenoalkylthio having 1 to 4 carbon atoms and 1 to 9 identical or different halogen atoms
and alkylene or dioxyalkylene having in each case 1 to 6 carbon atoms and being in each case doubly attached and optionally mono- or polysubstituted by identical or different substituents selected from the group consisting of halogen and straight-chain or branched alkyl having 1 to 4 carbon atoms and straight-chain or branched halogenoalkyl having 1 to 4 carbon atoms and 1 to 9 identical or different halogen atoms.
R² represents one of the groupings below: 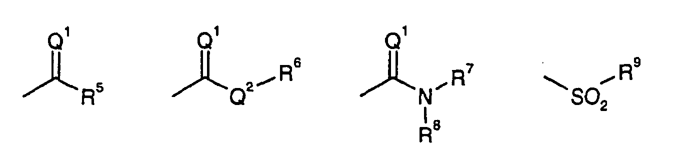 in which
Q¹ and Q² independently of one another each represent oxygen or sulphur,
R⁵ represents hydrogen or alkyl having 1 to 6 carbon atoms or represents aryl or arylalkyl having 1 to 4 carbon atoms in the alkyl moiety, each of which is optionally substituted in the aryl moiety by halogen, cyano, nitro, alkyl or alkoxy having in each case 1 to 4 carbon atoms,
R⁶ represents alkyl having 1 to 6 carbon atoms or represents aryl or arylalkyl having 1 to 4 carbon atoms in the alkyl moiety, each of which is optionally substituted in the aryl moiety by halogen, cyano, nitro, alkyl or alkoxy having in each case 1 to 4 carbon atoms,
R⁷ represents hydrogen, alkyl having 1 to 6 carbon atoms or represents aryl or arylalkyl having 1 to 4 carbon atoms in the alkyl moiety, each of which is optionally substituted in the aryl moiety by halogen, cyano, nitro, alkyl or alkoxy having in each case 1 to 4 carbon atoms,
R⁸ represents alkyl having 1 to 6 carbon atoms or represents aryl or arylalkyl having 1 to 4 carbon atoms in the alkyl moiety, each of which is optionally substituted in the aryl moiety by halogen, cyano, nitro, alkyl or alkoxy having in each case 1 to 4 carbon atoms, or
R⁷ and R⁸ together with the linking nitrogen atom represent an optionally methyl-substituted heterocyclic ring,
R⁹ represents alkyl having 1 to 6 carbon atoms, dialkylamino having in each case 1 to 4 carbon atoms in the individual alkyl moieties, heterocyclyl having 3 to 7 ring members which is attached via nitrogen or represents aryl or arylalkyl having 1 to 4 carbon atoms in the alkyl moiety, each of which is optionally substituted in the aryl moiety by halogen, cyano, nitro, alkyl or alkoxy having in each case 1 to 4 carbon atoms,
R³ represents hydrogen or alkyl having 1 to 4 carbon atoms,
R⁴ represents aryl, cycloalkyl or cycloalkenyl having 3 to 8 carbon atoms, or heterocyclyl having 3 to 12 ring members, each of which is optionally mono- or polysubstituted by identical or different substituents, the possible substituents being selected from the list below:
halogen, cyano, nitro, amino, hydroxyl, formyl, carboxyl, carbamoyl, thiocarbamoyl;
alkyl, alkoxy, alkylthio, alkylsulphinyl or alkylsulphonyl having in each case 1 to 6 carbon atoms and being in each case straight-chain or branched;
alkenyl or alkenyloxy having in each case 2 to 6 carbon atoms and being in each case straight-chain or branched;
halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms and being in each case straight-chain or branched;
halogenoalkenyl or halogenoalkenyloxy having in each case 2 to 6 carbon atoms and 1 to 13 identical or different halogen atoms and being in each case straight-chain or branched;
alkylamino, dialkylamino, alkylcarbonyl, alkylcarbonyloxy, alkoxycarbonyl, alkylsulphonyloxy, hydroximinoalkyl or alkoximinoalkyl having in each case 1 to 6 carbon atoms in the individual alkyl moieties and being in each case straight-chain or branched;
alkylene or dioxyalkylene having in each case 1 to 6 carbon atoms and being in each case doubly attached and being in each case optionally mono- or polysubstituted by identical or different substituents selected from the group consisting of halogen and straight-chain or branched alkyl having 1 to 4 carbon atoms and straight-chain or branched halogenoalkyl having 1 to 4 carbon atoms and 1 to 9 identical or different halogen atoms;
cycloalkyl having 3 to 6 carbon atoms;
and also aryl, aryloxy, arylthio, arylalkyl, arylalkyloxy, arylalkylthio, heterocyclyl, heterocyclyloxy, heterocyclylthio, heterocyclylalkyl, heterocyclylalkyloxy or heterocyclylalkylthio, each of which is optionally mono- or polysubstituted by identical or different substituents selected from the group consisting of
halogen, cyano and straight-chain or branched alkyl having 1 to 4 carbon atoms
and straight-chain or branched halogenoalkyl having 1 to 4 carbon atoms and 1 to 9 identical or different halogen atoms
and straight-chain or branched alkoxy or alkylthio having 1 to 4 carbon atoms
and straight-chain or branched halogenoalkoxy or halogenoalkylthio having 1 to 4 carbon atoms and 1 to 9 identical or different halogen atoms
and alkylene or dioxyalkylene having in each case 1 to 6 carbon atoms and being in each case doubly attached and optionally mono- or polysubstituted by identical or different substituents selected from the group consisting of halogen and straight-chain or branched alkyl having 1 to 4 carbon atoms and straight-chain or branched halogenoalkyl having 1 to 4 carbon atoms and 1 to 9 identical or different halogen atoms.

6. Compounds of the formula (I-I) according to Claim 5, in which
A represents a single bond or represents methylene, 1,1-ethylene, 1,2-ethylene 1,1-, 1,2-, 1,3- or 2,2-propylene, 1,1-, 1,2-, 1,3-, 1,4-, 2,2-, 2,3-butylene or 1,1-, 1,2- or 1,3-(2-methyl-propylene), each of which is optionally mono- or polysubstituted by identical or different substituents selected from the group consisting of fluorine, chlorine, cyano and methoxy,
Q represents oxygen or sulphur,
R¹ represents one of the groupings below 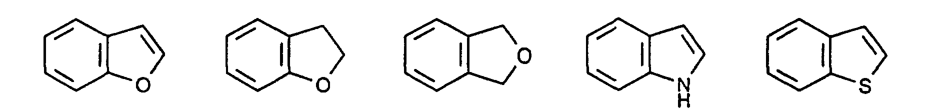 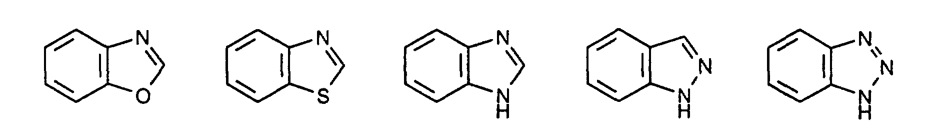 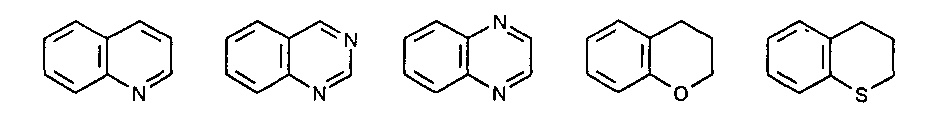 each of which is attached via a carbon atom of the respective benzene ring and each of which may be optionally mono- to trisubstituted by identical or different substituents from the substituents listed below:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, n-pentyl, n-hexyl, n-heptyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinoethyl or ethoximinoethyl;
trimethylene (propane-1,3-diyl), tetramethylene (butane-1,4-diyl), methylenedioxy or ethylenedioxy, each of which is doubly attached and each of which is optionally mono- or polysubstituted by identical or different substituents selected from the group consisting of fluorine, chlorine, methyl, trifluoromethyl, ethyl, n- and i-propyl;
cyclopropyl, cyclobutyl, cyclopentyl or cyclohexyl;
cyclopropyl, cyclobutyl, cyclopentyl or cyclohexyl, benzyl, phenoxy, benzyloxy or phenyl which is optionally substituted by the abovementioned substituents,
R² represents one of the groupings below: 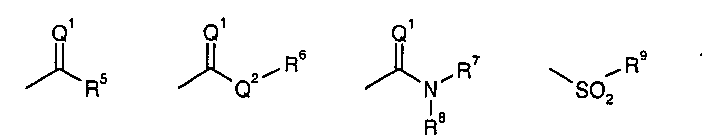 in which
Q¹ and Q² independently of one another each represent oxygen or sulphur,
R⁵ represents hydrogen or methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R⁶ represents methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R⁷ represents hydrogen, methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R⁸ represents methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy, or
R⁷ and R⁸ together with the linking nitrogen atom represent an optionally methyl-substituted heterocyclic ring,
R⁹ represents methyl, ethyl, n- or i-propyl, n-, i-, s- or t-butyl, dimethylamino, diethylamino, pyrrolidin-1-yl, morpholin-N-yl, piperidin-1-yl, or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R³ represents hydrogen or represents methyl or ethyl,
R⁴ represents phenyl, naphthyl, furyl, benzofuranyl, pyrrolyl, indolyl, thienyl, benzothienyl, oxazolyl, isoxazolyl, thiazolyl, isothiazolyl, oxadiazolyl, thiadiazolyl, pyridyl, quinolyl, pyrimidyl, pyridazinyl, pyrazinyl, oxiranyl, oxetanyl, tetrahydrofuryl, perhydropyranyl, pyrrolidinyl, piperidinyl or morpholinyl, each of which is optionally mono- to trisubstituted, the possible substituents being selected from the list below:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinoethyl or ethoximinoethyl,
trimethylene (propane-1,3-diyl), tetramethylene (butane-1,4-diyl), methylenedioxy or ethylenedioxy, each of which is doubly attached and each of which is optionally mono- or polysubstituted by identical or different substituents selected from the group consisting of fluorine, chlorine, methyl, trifluoromethyl, ethyl, n- and i-propyl,
cyclopropyl, cyclobutyl, cyclopentyl or cyclohexyl.

7. Compounds of the formula (I-I) according to Claim 5, in which
A represents a single bond or represents methylene, 1,1-ethylene, 1,2-ethylene 1,1-, 1,2-, 1,3- or 2,2-propylene, 1,1-, 1,2-, 1,3-, 1,4-, 2,2-, 2,3-butylene or 1,1-, 1,2- or 1,3- (2-methyl-propylene), optionally mono- or polysubstituted by identical or different substituents selected from the group consisting of fluorine, chlorine, cyano, methoxy,
Q represents oxygen or sulphur,
R¹ represents one of the groupings below 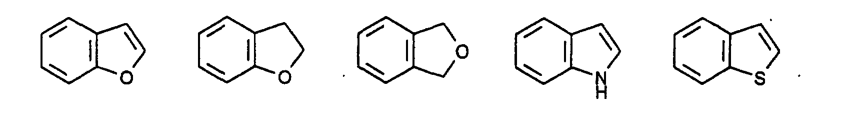 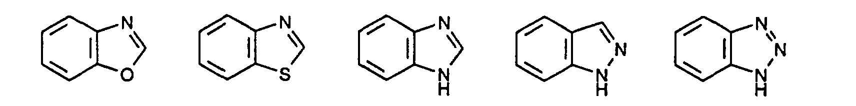 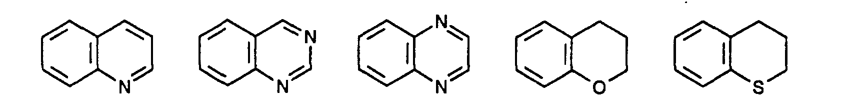 each of which is attached via a carbon atom of the respective benzene ring and each of which may be optionally mono- to trisubstituted by identical or different substituents from the substituents listed below:
fluorine, chlorine, bromine, cyano, nitro, methyl, ethyl, n- or i-propyl, n-, i-, s- or t-butyl, n-pentyl, n-hexyl, n-heptyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl, trifluoromethylsulphonyl, acetyl, propionyl, acetyloxy, methoxycarbonyl, ethoxycarbonyl, methylsulphonyloxy, ethylsulphonyloxy, hydroximinomethyl, hydroximinoethyl, methoximinoethyl or ethoximinoethyl;
trimethylene (propane-1,3-diyl), tetramethylene (butane-1,4-diyl), methylenedioxy or ethylenedioxy, each of which is doubly attached and each of which is optionally mono- or polysubstituted by identical or different substituents selected from the group consisting of fluorine, chlorine, methyl, trifluoromethyl, ethyl, n- and i-propyl; cyclopropyl, cyclopentyl, cyclohexyl; phenoxy, benzyloxy, phenyl, which is optionally substituted by the abovementioned substituents;
R² represents one of the groupings below: 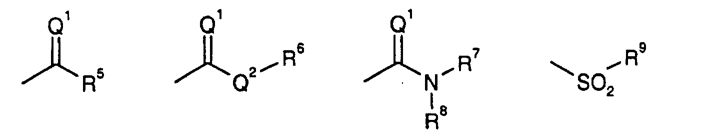 in which
Q¹ and Q² independently of one another each represent oxygen or sulphur,
R⁵ represents hydrogen or methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R⁶ represents methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R⁷ represents hydrogen, methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R⁸ represents methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy, or
R⁷ and R⁸ together with the linking nitrogen atom represent an optionally methyl-substituted heterocyclic ring,
R⁹ represents methyl, ethyl, n- or i-propyi, n-, i-, s- or t-butyl, dimethylamino, diethylamino, pyrrolidin-1-yl, morpholin-N-yl, piperidin-1-yl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R³ represents hydrogen or represents methyl,
R⁴ represents phenyl, naphthyl, furyl, benzofuranyl, pyrrolyl, indolyl, thienyl, benzothienyl, oxazolyl, isoxazolyl, thiazolyl, isothiazolyl, oxadiazolyl, thiadiazolyl, pyridyl, quinolyl, pyrimidyl, pyridazinyl, pyrazinyl, oxiranyl, oxetanyl, tetrahydrofuryl, perhydropyranyl, pyrrolidinyl, piperidinyl or morpholinyl, each of which is optionally mono- to trisubstituted, the possible substituents being selected from the list below:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinoethyl or ethoximinoethyl, represents trimethylene (propane-1,3-diyl), tetramethylene (butane-1,4-diyl), methylenedioxy or ethylenedioxy, each of which is doubly attached and each of which is optionally mono- or polysubstituted by identical or different substituents selected from the group consisting of fluorine, chlorine, methyl, trifluoromethyl, ethyl, n- and i-propyl, cyclopropyl, cyclobutyl, cyclopentyl or cyclohexyl.

8. Compounds of the formula (I-I) according to Claim 5, in which
A represents a single bond or represents methylene, 1,1-ethylene, 1,2-ethylene, 1,1-propylene, 1,2-propylene, 1,3-propylene or 2,2-propylene,
Q represents oxygen,
R¹ represents one of the groupings below 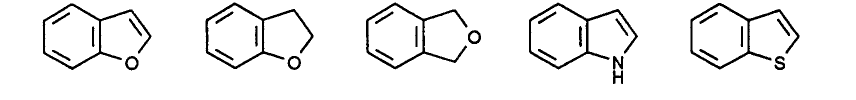 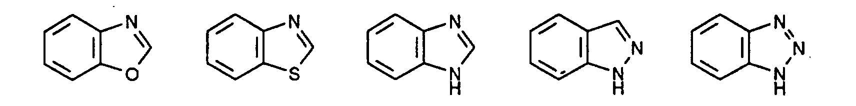 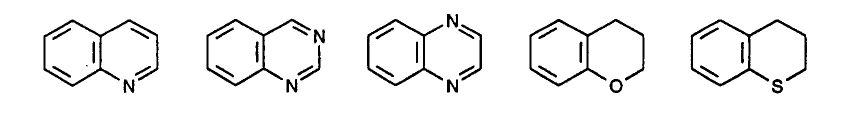 each of which is attached via a carbon atom of the respective benzene ring and each of which may be optionally mono- to trisubstituted by identical or different substituents from the substituents listed below:
bromine, chlorine, fluorine, nitro, methylsulphonyl, phenyl, phenyloxy, benzyloxy, cyclopropyl, cyclohexyl, methyl, ethyl, n- or i-propyl, n-, i-, s- or t-butyl, n-pentyl, n-hexyl, n-heptyl, methoxy, ethoxy, trifluoromethyl, trifluoromethoxy and/or methylthio,
R² represents one of the groupings below: 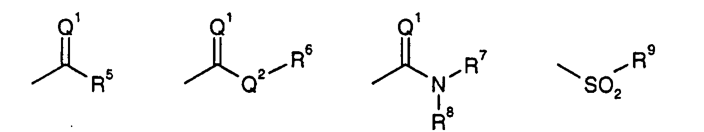 in which
Q¹ and Q² each represent oxygen,
R⁵ represents hydrogen or methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R⁶ represents methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R⁷ represents hydrogen, methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R⁸ represents methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy, or
R⁷ and R⁸ together with the linking nitrogen atom represent an optionally methyl-substituted heterocyclic ring,
R⁹ represents methyl, ethyl, n- or i-propyl, n-, i-, s- or t-butyl, dimethylamino, diethylamino, pyrrolidin-1-yl, morpholin-N-yl, piperidin-1-yl, or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R³ represents hydrogen or methyl,
R⁴ represents cyclohexyl or optionally mono- to trisubstituted phenyl, thienyl, furyl, benzofuryl, benzothienyl, pyridyl, pyrimidinyl, naphthyl, quinolyl, the possible substituents being selected from the list below:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinoethyl or ethoximinoethyl,
trimethylene (propane-1,3-diyl), tetramethylene (butane-1,4-diyl), methylenedioxy or ethylenedioxy, each of which is doubly attached and each of which is optionally mono- or polysubstituted by identical or different substituents selected from the group consisting of fluorine, chlorine, methyl, trifluoromethyl, ethyl, n- and i-propyl.

9. Compounds of the formula (I-I), 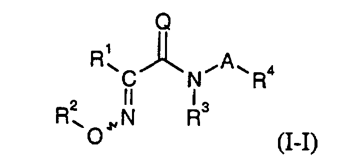 in which
A represents a single bond or represents alkylene having 1 to 6 carbon atoms which is optionally mono- or polysubstituted by identical or different substituents, the possible substituents being selected from the list below:
halogen, cyano, nitro, amino, hydroxyl, formyl, carboxyl, carbamoyl, thiocarbamoyl;
alkoxy, alkylthio, alkylsulphinyl or alkylsulphonyl having in each case 1 to 6 carbon atoms and being in each case straight-chain or branched;
alkenyl or alkenyloxy having in each case 2 to 6 carbon atoms and being in each case straight-chain or branched;
halogenoalkoxy, halogenoalkylthio, halogenoalkylsulphinyl or halogenoalkylsulphonyl having in each case 1 to 6 carbon atoms and 1 to 13 identical or different halogen atoms and being in each case straight-chain or branched;
halogenoalkenyl or halogenoalkenyloxy having in each case 2 to 6 carbon atoms and 1 to 13 identical or different halogen atoms and being in each case straight-chain or branched;
alkylamino, dialkylamino, alkylcarbonyl, alkylcarbonyloxy, alkoxycarbonyl, alkylsulphonyloxy, hydroximinoalkyl or alkoximinoalkyl having in each case 1 to 6 carbon atoms in the individual alkyl moieties and being in each case straight-chain or branched;
cycloalkyl having 3 to 6 carbon atoms;
and also aryl or heterocyclyl, each of which is optionally mono- or polysubstituted by identical or different substituents selected from the group consisting of
halogen, cyano and straight-chain or branched alkyl having 1 to 4 carbon atoms
and straight-chain or branched halogenoalkyl having 1 to 4 carbon atoms and 1 to 9 identical or different halogen atoms
and straight-chain or branched alkoxy or alkylthio having 1 to 4 carbon atoms
and straight-chain or branched halogenoalkoxy or halogenoalkylthio having 1 to 4 carbon atoms and 1 to 9 identical or different halogen atoms
and alkylene or dioxyalkylene having in each case 1 to 6 carbon atoms and being in each case doubly attached and optionally mono- or polysubstituted by identical or different substituents selected from the group consisting of halogen and straight-chain or branched alkyl having 1 to 4 carbon atoms
and straight-chain or branched halogenoalkyl having 1 to 4 carbon atoms and 1 to 9 identical or different halogen atoms,
Q represents oxygen or sulphur,
R¹ represents a tricycle 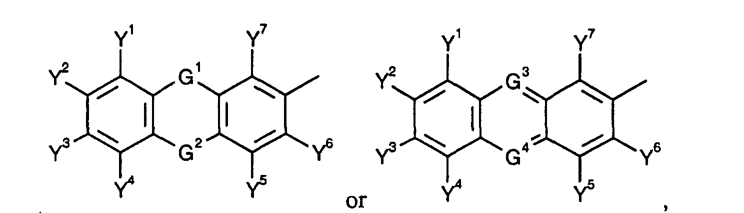 in which
G¹ and G² independently of one another each represent a single bond, alkanediyl, alkenediyl, oxygen, sulphur, -NH-, -N(alkyl)- or carbonyl,
G³ and G⁴ independently of one another each represent nitrogen or a grouping 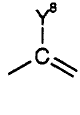 and
Y¹, Y², Y³, Y⁴, Y⁵, Y⁶, Y⁷ and Y⁸ independently of one another each represent hydrogen, halogen, cyano, nitro; alkyl, alkoxy, alkylamino, dialkylamino, alkylthio, alkylsulphinyl or alkylsulphonyl having in each case 1 to 8 carbon atoms in the individual alkyl moieties;
halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms or
cycloalkyl having 3 to 6 carbon atoms,
R² represents one of the groupings below: 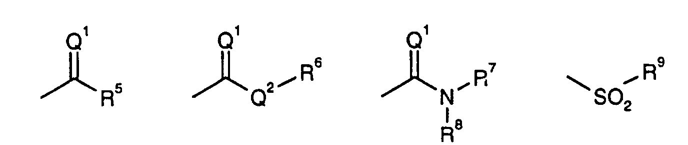 in which
Q¹ and Q² independently of one another each represent oxygen or sulphur,
R⁵ represents hydrogen or alkyl having 1 to 6 carbon atoms or represents aryl or arylalkyl having 1 to 4 carbon atoms in the alkyl moiety, each of which is optionally substituted in the aryl moiety by halogen, cyano, nitro, alkyl or alkoxy having in each case 1 to 4 carbon atoms,
R⁶ represents alkyl having 1 to 6 carbon atoms or represents aryl or arylalkyl having 1 to 4 carbon atoms in the alkyl moiety, each of which is optionally substituted in the aryl moiety by halogen, cyano, nitro, alkyl or alkoxy having in each case 1 to 4 carbon atoms,
R⁷ represents hydrogen, alkyl having 1 to 6 carbon atoms or represents aryl or arylalkyl having 1 to 4 carbon atoms in the alkyl moiety, each of which is optionally substituted in the aryl moiety by halogen, cyano, nitro, alkyl or alkoxy having in each case 1 to 4 carbon atoms,
R⁸ represents alkyl having 1 to 6 carbon atoms or represents aryl or arylalkyl having 1 to 4 carbon atoms in the alkyl moiety, each of which is optionally substituted in the aryl moiety by halogen, cyano, nitro, alkyl or alkoxy having in each case 1 to 4 carbon atoms, or
R⁷ and R⁸ together with the linking nitrogen atom represent an optionally methyl-substituted heterocyclic ring,
R⁹ represents alkyl having 1 to 6 carbon atoms, dialkylamino having in each case 1 to 4 carbon atoms in the individual alkyl moieties, heterocyclyl having 3 to 7 ring members which is attached via nitrogen or represents aryl or arylalkyl having 1 to 4 carbon atoms in the alkyl moiety, each of which is optionally substituted in the aryl moiety by halogen, cyano, nitro, alkyl or alkoxy having in each case 1 to 4 carbon atoms,
R³ represents hydrogen or alkyl having 1 to 4 carbon atoms,
R⁴ represents aryl, cycloalkyl or cycloalkenyl having 3 to 8 carbon atoms, or heterocyclyl having 3 to 12 ring members, each of which is optionally mono- or polysubstituted by identical or different substituents, the possible substituents being selected from the list below:
halogen, cyano, nitro, amino, hydroxyl, formyl, carboxyl, carbamoyl, thiocarbamoyl;
alkyl, alkoxy, alkylthio, alkylsulphinyl or alkylsulphonyl having in each case 1 to 6 carbon atoms and being in each case straight-chain or branched;
alkenyl or alkenyloxy having in each case 2 to 6 carbon atoms and being in each case straight-chain or branched;
halogenoalkyl, halogenoalkoxy, halogenoalkylthio, halogenoalkylsulphinyl or halogenoalkylsulphonyl having in each case 1 to 6 carbon atoms and 1 to 13 identical or different halogen atoms and being in each case straight-chain or branched;
halogenoalkenyl or halogenoalkenyloxy having in each case 2 to 6 carbon atoms and 1 to 13 identical or different halogen atoms and being in each case straight-chain or branched;
alkylamino, dialkylamino, alkylcarbonyl, alkylcarbonyloxy, alkoxycarbonyl, alkylsulphonyloxy, hydroximinoalkyl or alkoximinoalkyl having in each case 1 to 6 carbon atoms in the individual alkyl moieties and being in each case straight-chain or branched;
alkylene or dioxyalkylene having in each case 1 to 6 carbon atoms and being in each case doubly attached and being in each case optionally mono- or polysubstituted by identical or different substituents selected from the group consisting of halogen and straight-chain or branched alkyl having 1 to 4 carbon atoms and straight-chain or branched halogenoalkyl having 1 to 4 carbon atoms and 1 to 9 identical or different halogen atoms;
Cycloalkyl having 3 to 6 carbon atoms;
and also aryl, aryloxy, arylthio, arylalkyl, arylalkyloxy, arylalkylthio, heterocyclyl, heterocyclyloxy, heterocyclylthio, heterocyclylalkyl, heterocyclylalkyloxy or heterocyclylalkylthio, each of which is optionally mono- or polysubstituted by identical or different substituents selected from the group consisting of
halogen, cyano and straight-chain or branched alkyl having 1 to 4 carbon atoms
and straight-chain or branched halogenoalkyl having 1 to 4 carbon atoms and 1 to 9 identical or different halogen atoms
and straight-chain or branched alkoxy or alkylthio having 1 to 4 carbon atoms
and straight-chain or branched halogenoalkoxy or halogenoalkylthio having 1 to 4 carbon atoms and 1 to 9 identical or different halogen atoms
and alkylene or dioxyalkylene having in each case 1 to 6 carbon atoms and being in each case doubly attached and optionally mono- or polysubstituted by identical or different substituents selected from the group consisting of halogen and straight-chain or branched alkyl having 1 to 4 carbon atoms and straight-chain or branched halogenoalkyl having 1 to 4 carbon atoms and 1 to 9 identical or different halogen atoms.

10. Compounds of the formula (I-I) according to Claim 9, in which
A represents a single bond or represents methylene, 1,1-ethylene, 1,2-ethylene 1,1-, 1,2-, 1,3- or 2,2-propylene, 1,1-, 1,2-, 1,3-, 1,4-, 2,2-, 2,3-butylene or 1,1-, 1,2- or 1,3-(2-methyl-propylene), each of which is optionally mono- or polysubstituted by identical or different substituents selected from the group consisting of fluorine, chlorine, cyano and methoxy,
Q represents oxygen or sulphur,
R¹ represents a tricycle 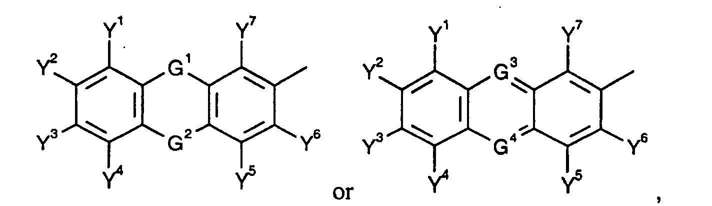 in which
G¹ and G² independently of one another each represent a single bond, alkanediyl, alkenediyl, oxygen, sulphur, -NH-, -N(alkyl)- or carbonyl and
G³ and G⁴ independently of one another each represent nitrogen or a grouping 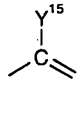 and
Y¹, Y², Y³, Y⁴, Y⁵, Y⁶, Y⁷ and Y⁸ independently of one another each represent hydrogen, fluorine, chlorine, bromine, cyano, nitro, methyl, ethyl, n- or i-propyl, n-, i-, s- or t-butyl, n-pentyl, n-hexyl, n-heptyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamino, ethylamino, n- or i-propylamino, dimethylamino, diethylamino, cyclopropyl, cyclobutyl, cyclopentyl or cyclohexyl,
R² represents one of the groupings below: 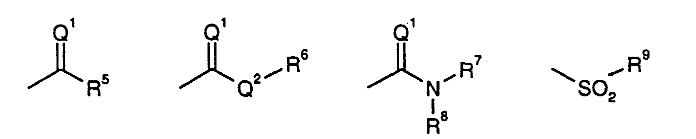 in which
Q¹ and Q² independently of one another each represent oxygen or sulphur,
R⁵ represents hydrogen or methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R⁶ represents methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R⁷ represents hydrogen, methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R⁸ represents methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy, or
R⁷ and R⁸ together with the linking nitrogen atom represent an optionally methyl-substituted heterocyclic ring,
R⁹ represents methyl, ethyl, n- or i-propyl, n-, i-, s- or t-butyl, dimethylamino, diethylamino, pyrrolidin-1-yl, morpholin-N-yl, piperidin-1-yl, or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R³ represents hydrogen or represents methyl or ethyl,
R⁴ represents cyclopropyl, cyclobutyl, cyciopentyl or cyclohexyl, phenyl, naphthyl, furyl, benzofuranyl, pyrrolyl, indolyl, thienyl, benzothienyl, oxazolyl, isoxazolyl, thiazolyl, isothiazolyl, oxadiazolyl, thiadiazolyl, pyridyl, quinolyl, pyrimidyl, pyridazinyl, pyrazinyl, oxiranyl, oxetanyl, tetrahydrofuryl, perhydropyranyl, pyrrolidinyl, piperidinyl or morpholinyl, each of which is optionally mono- to trisubstituted, the possible substituents being selected from the list below:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinoethyl or ethoximinoethyl,
trimethylene (propane-1,3-diyl), tetramethylene (butane-1,4-diyl), methylenedioxy or ethylenedioxy, each of which is doubly attached and each of which is optionally mono- or polysubstituted by identical or different substituents selected from the group consisting of fluorine, chlorine, methyl, trifluoromethyl, ethyl, n- and i-propyl,
cyclopropyl, cyclobutyl, cyclopentyl or cyclohexyl.

11. Compounds of the formula (I-I) according to Claim 9, in which
A represents a single bond or represents methylene, 1,1-ethylene, 1,2-ethylene 1,1-, 1,2-, 1,3- or 2,2-propylene, 1,1-, 1,2-, 1,3-, 1,4-, 2,2-, 2,3-butylene or 1,1-, 1,2- or 1,3-(2-methyl-propylene), optionally mono- or polysubstituted by identical or different substituents selected from the group consisting of fluorine, chlorine, cyano, methoxy,
Q represents oxygen or sulphur,
R¹ represents a tricycle 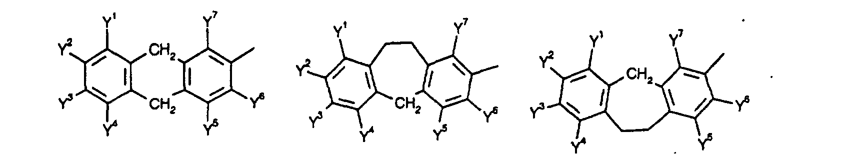 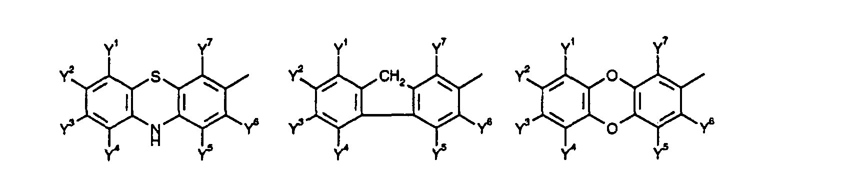 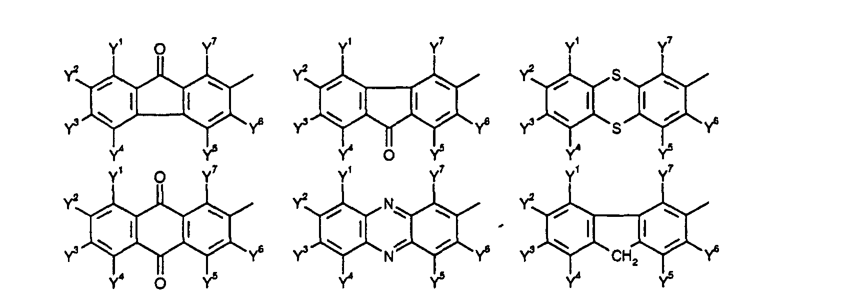 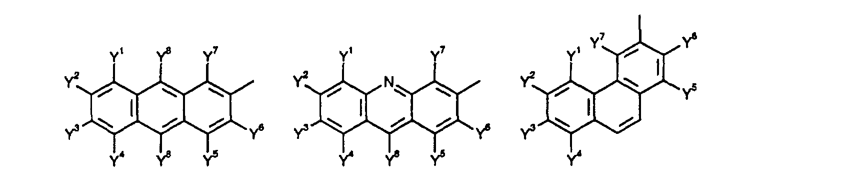 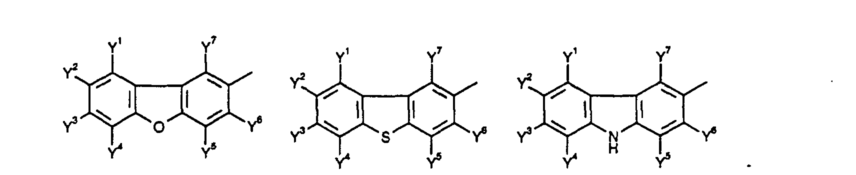 in which
Y¹, Y², Y³, Y⁴, Y⁵, Y⁶, Y⁷ and Y⁸ independently of one another each represent hydrogen, fluorine, chlorine, bromine, cyano, nitro, methyl, ethyl, n- or i-propyl, n-, i-, s- or t-butyl, n-pentyl, n-hexyl, n-heptyl, methoxy, ethoxy, n- or i-propoxy, methylthio, ethylthio, nor i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamino, ethylamino, n- or i-propylamino, dimethylamino, diethylamino, cyclopropyl, cyclobutyl, cyclopentyl or cyclohexyl,
R² represents one of the groupings below: 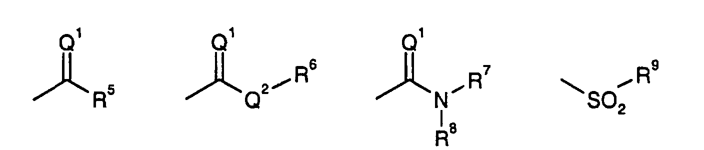 in which
Q¹ and Q² independently of one another each represent oxygen or sulphur,
R⁵ represents hydrogen or methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R⁶ represents methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R⁷ represents hydrogen, methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R⁸ represents methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy, or
R⁷ and R⁸ together with the linking nitrogen atom represent an optionally methyl-substituted heterocyclic ring,
R⁹ represents methyl, ethyl, n- or i-propyl, n-, i-, s- or t-butyl, dimethylamino, diethylamino, pyrrolidin-1-yl, morpholin-N-yl, piperidin-1-yl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R³ represents hydrogen or represents methyl,
R⁴ represents phenyl, naphthyl, furyl, benzofuranyl, pyrrolyl, indolyl, thienyl, benzothienyl, oxazolyl, isoxazolyl, thiazolyl, isothiazolyl, oxadiazolyl, thiadiazolyl, pyridyl, quinolyl, pyrimidyl, pyridazinyl, pyrazinyl, oxiranyl, oxetanyl, tetrahydrofuryl, perhydropyranyl, pyrrolidinyl, piperidinyl or morpholinyl, each of which is optionally mono- to trisubstituted, the possible substituents being selected from the list below:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinoethyl or ethoximinoethyl, represents trimethylene (propane-1,3-diyl), tetramethylene (butane-1,4-diyl), methylenedioxy or ethylenedioxy, each of which is doubly attached and each of which is optionally mono- or polysubstituted by identical or different substituents selected from the group consisting of fluorine, chlorine, methyl, trifluoromethyl, ethyl, n- and i-propyl,
cyclopropyl, cyclobutyl, cyclopentyl or cyclohexyl.

12. Compounds of the formula (I-I) according to Claim 9, in which
A represents a single bond or represents methylene, 1,1-ethylene, 1,2-ethylene, 1,1-propylene, 1,2-propylene, 1,3-propylene or 2,2-propylene,
Q represents oxygen,
R¹ represents a tricycle 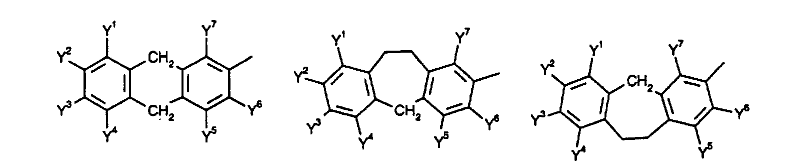 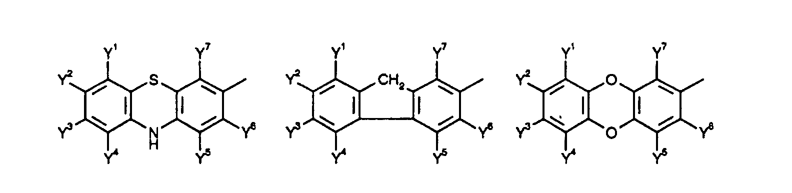 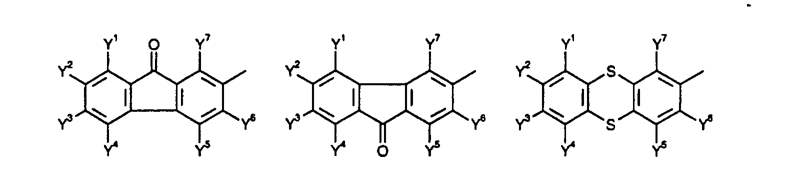 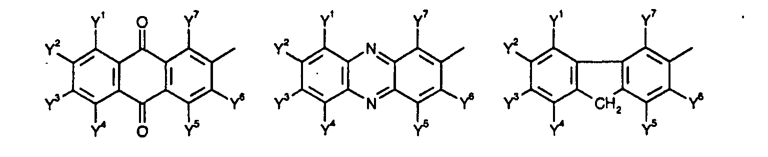 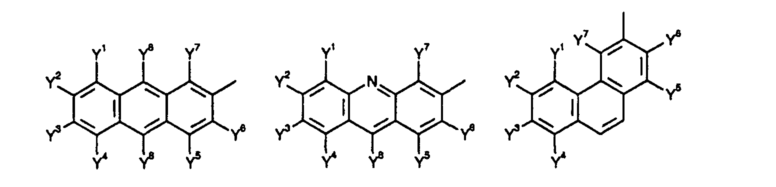 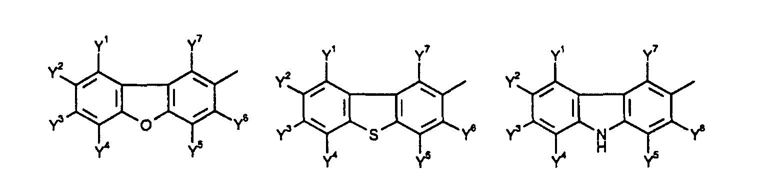 in which
Y¹, Y², Y³, Y⁴, Y⁵, Y⁶, Y⁷ and Y⁸ independently of one another each represent hydrogen, fluorine, chlorine, bromine, cyano, nitro, methyl, ethyl, n- or i-propyl, n-, i-, s- or t-butyl, n-pentyl, n-hexyl, n-heptyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl or trifluoromethylsulphonyl, methylamino, ethylamino, n- or i-propylamino, dimethylamino, diethylamino, cyclopropyl, cyclobutyl, cyclopentyl or cyclohexyl,
R² represents one of the groupings below: 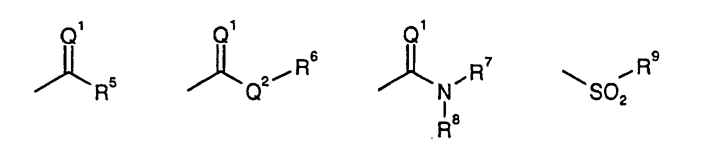 in which
Q¹ and Q² each represent oxygen,
R⁵ represents hydrogen or methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R⁶ represents methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R⁷ represents hydrogen, methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R⁸ represents methyl, ethyl, n- or i-propyl, n-, i-, s- or t-butyl or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy, or
R⁷ and R⁸ together with the linking nitrogen atom represent an optionally methyl-substituted heterocyclic ring,
R⁹ represents methyl, ethyl, n- or i-propyl, n-, i-, s- or t-butyl, dimethylamino, diethylamino, pyrrolidin-1-yl, morpholin-N-yl, piperidin-1-yl, or represents phenyl, benzyl, 1-phenylethyl or 2-phenylethyl, each of which is optionally substituted in the phenyl moiety by fluorine, chlorine, bromine, cyano, nitro, methyl, ethyl, n- or i-propyl, n-, i-, s- or t-butyl, methoxy or ethoxy,
R³ represents hydrogen or methyl,
R⁴ represents cyclohexyl or optionally mono- to trisubstituted phenyl, thienyl, furyl, benzofuryl, benzothienyl, pyridyl, pyrimidinyl, naphthyl, quinolyl, the possible substituents being selected from the list below:
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, tri-fluoroethoxy, difluoromethylthio, difluorochloromethylthio, trifluoromethylthio, trifluoromethylsulphinyl or trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinoethyl or ethoximinoethyl,
trimethylene (propane-1,3-diyl), tetramethylene (butane-1,4-diyl), methylenedioxy or ethylenedioxy, each of which is doubly attached and each of which is optionally mono- or polysubstituted by identical or different substituents selected from the group consisting of fluorine, chlorine, methyl, trifluoromethyl, ethyl, n- and i-propyl.

13. Pesticides, **characterized in that** they contain at least one compound of the formula (I) according to Claim 1.

14. Method for controlling pests, **characterized in that** compounds of the formula (I) according to Claim 1 are allowed to act on pests and/or their habitat.

15. Uses of compounds according to Claims 1 to 12 for controlling pests.

16. Process for preparing pesticides, **characterized in that** compounds according to Claims 1 to 12 are mixed with extenders and/or surfactants.

## Revendications

1. Composés de formule générale (I-I) 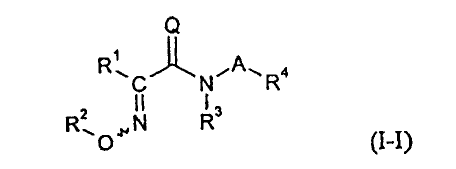 dans laquelle
A représente une liaison simple ou un groupe alkylène ayant 1 à 6 atomes de carbone, portant le cas échéant un ou plusieurs substituants identiques ou différents, les substituants possibles étant choisis parmi ceux qui sont énumérés ci-après :
halogène, cyano, nitro, amino, hydroxy, formyle, carboxy, carbamoyle, thiocarbamoyle ;
alkoxy, alkylthio, alkylsulfinyle ou alkylsulfonyle, chacun linéaire ou ramifié et ayant chacun 1 à 6 atomes de carbone ;
alcényle ou alcényloxy chacun linéaire ou ramifié ayant chacun 2 à 6 atomes de carbone ;
halogénalkoxy, halogénalkylthio, halogénalkylsulfinyle ou halogénalkylsulfonyle chacun linéaire ou ramifié ayant chacun 1 à 6 atomes de carbone et 1 à 13 atomes d'halogènes identiques ou différents ;
halogénalcényle ou halogénalcényloxy chacun linéaire ou ramifié ayant chacun 2 à 6 atomes de carbone et 1 à 13 atomes d'halogènes identiques ou différents ;
alkylamino, dialkylamino, alkylcarbonyle, alkylcarbonyloxy, alkoxycarbonyle, alkylsulfonyloxy, hydroximino-alkyle ou alkoximino-alkyle chacun linéaire ou ramifié, ayant chacun 1 à 6 atomes de carbone dans les parties alkyle individuelles ;
cycloalkyle ayant 3 à 6 atomes de carbone ;
ainsi qu'aryle ou hétérocyclyle portant chacun le cas échéant un ou plusieurs substituants, identiques ou différents
halogène, cyano et/ou alkyle linéaire ou ramifié ayant 1 à 4 atomes de carbone
et/ou halogénalkyle linéaire ou ramifié ayant 1 à 4 atomes de carbone et 1 à 9 atomes d'halogènes identiques ou différents
et/ou alkoxy ou alkylthio linéaire ou ramifié ayant 1 à 4 atomes de carbone
et/ou halogénalkoxy ou halogénalkylthio linéaire ou ramifié ayant 1 à 4 atomes de carbone et 1 à 9 atomes d'halogènes identiques ou différents et/ou alkylène ou dioxyalkylène ayant chacun deux liaisons et chacun 1 à 6 atomes de carbone, chacun étant éventuellement substitué une ou plusieurs fois identiques ou différentes par un halogène et/ou un radical alkyle linéaire ou ramifié ayant 1 à 4 atomes de carbone
et/ou un radical halogénalkyle linéaire ou ramifié ayant 1 à 4 atomes de carbone et 1 à 9 atomes d'halogènes identiques ou différents,
Q représente l'oxygène ou le soufre,
R¹ est un groupe aryle, cycloalkyle ou cycloalcényle ayant 3 à 7 atomes de carbone ou un groupe hétérocyclyle à noyau de 3 à 12 atomes, chacun portant éventuellement un ou plusieurs substituants, identiques ou différents, les substituants possibles étant choisis parmi ceux qui sont énumérés ci-après :
halogène, cyano, nitro, amino, hydroxy, formyle, carboxy, carbamoyle, thiocarbamoyle ;
alkyle, alkoxy, alkylthio, alkylsulfinyle ou alkylsulfonyle chacun linéaire ou ramifié ayant chacun 1 à 8 atomes de carbone ;
alcényle ou alcényloxy chacun linéaire ou ramifié ayant chacun 2 à 6 atomes de carbone ;
halogénalkyle, halogénalkoxy, halogénalkylthio, halogénalkylsulfinyle ou halogénalkylsulfonyle chacun linéaire ou ramifié ayant chacun 1 à 6 atomes de carbone et 1 à 13 atomes d'halogènes identiques ou différents ;
halogénalcényle ou halogénalcényloxy chacun linéaire ou ramifié ayant chacun 2 à 6 atomes de carbone et 1 à 13 atomes d'halogènes identiques ou différents ;
alkylamino, dialkylamino, alkylcarbonyle, alkylcarbonyloxy, alkoxycarbonyle, alkylsulfonyloxy, hydroximino-alkyle ou alkoximino-alkyle chacun linéaire ou ramifié, ayant chacun 1. à 8 atomes de carbone dans les parties alkyle individuelles ;
alkylène ou dioxyalkylène ayant chacun deux liaisons et chacun 1 à 6 atomes de carbone, chacun étant éventuellement substitué une ou plusieurs fois identiques ou différentes par un halogène et/ou un radical alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents ;
cycloalkyle ayant 3 à 6 atomes de carbone ;
ainsi qu'aryle, aryloxy, arylthio, arylalkyle, arylalkyloxy, arylalkylthio, hétérocyclyle, hétérocyclyloxy, hétérocyclylthio, hétérocyclylalkyle, hétérocyclylalkyloxy ou hétérocyclylalkylthio portant chacun le cas échéant un ou plusieurs substituants, identiques ou différents
halogène, cyano et/ou alkyle linéaire ou ramifié ayant 1 à 4 atomes de carbone
et/ou halogénalkyle linaire ou ramifié ayant 1 à 4 atomes de carbone et 1 à 9 atomes d'halogènes identiques ou différents
et/ou alkoxy ou alkylthio linéaire ou ramifié ayant 1 à 4 atomes de carbone
et/ou halogénalkoxy ou halogénalkylthio linéaire ou ramifié ayant 1 à 4 atomes de carbone et 1 à 9 atomes d'halogènes identiques ou différents
alkylène ou dioxyalkylène ayant chacun deux liaisons et chacun 1 à 6 atomes de carbone,
chacun étant éventuellement substitué une ou plusieurs fois identiques ou différentes par un halogène et/ou un radical alkyle linéaire ou ramifié ayant 1 à 4 atomes de carbone et/ou un radical halogénalkyle linéaire ou ramifié ayant 1 à 4 atomes de carbone et 1 à 9 atomes d'halogènes identiques ou différents,
R² représente l'un des groupements suivants : 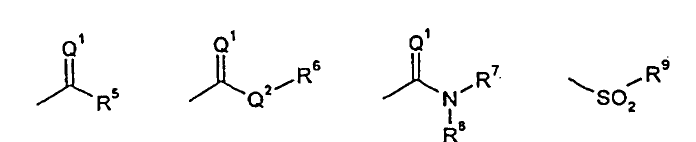 dans lesquels
Q¹ et Q² représentent indépendamment l'un de l'autre l'oxygène ou le soufre,
R⁵ représente l'hydrogène ou un groupe alkyle ayant 1 à 6 atomes de carbone, ou bien un groupe aryle ou un groupe arylalkyle ayant 1 à 4 atomes de carbone dans la partie alkyle, chacun étant éventuellement substitué dans la partie aryle par un radical halogéno, cyano, nitro, alkyle ou alkoxy ayant chacun 1 à 4 atomes de carbone,
R⁶ est un groupe alkyle ayant 1 à 6 atomes de carbone ou bien un groupe aryle ou un groupe arylalkyle ayant 1 à 4 atomes de carbone dans la partie alkyle, chacun étant éventuellement substitué dans la partie aryle par un radical halogéno, cyano, nitro, alkyle ou alkoxy ayant chacun 1 à 4 atomes de carbone,
R⁷ représente l'hydrogène, un groupe alkyle ayant 1 à 6 atomes de carbone ou bien un groupe aryle ou un groupe arylalkyle ayant 1 à 4 atomes de carbone dans la partie alkyle, chacun étant éventuellement substitué dans la partie aryle par un radical halogéno, cyano, nitro, alkyle ou alkoxy ayant chacun 1 à 4 atomes de carbone,
R⁸ est un groupe alkyle ayant 1 à 6 atomes de carbone ou bien un groupe aryle ou un groupe arylalkyle ayant 1 à 4 atomes de carbone dans la partie alkyle, chacun étant substitué le cas échéant dans la partie aryle par un radical halogéno, cyano, nitro, alkyle, ou alkoxy ayant chacun 1 à 4 atomes de carbone, ou bien
R⁷ et R⁸ forment, conjointement avec l'atome d'azote auquel ils sont liés, un noyau hétérocyclique éventuellement substitué par un radical méthyle,
R⁹ est un groupé alkyle ayant 1 à 6 atomes de carbone, dialkylamino ayant 1 à 4 atomes de carbone dans chacune des parties alkyle individuelles, un groupe hétérocyclyle à noyau de 3 à 7 atomes lié par de l'azote ou un groupe aryle, ou arylalkyle ayant 1 à 4 atomes de carbone dans la partie alkyle, chacun étant éventuellement substitué dans la partie aryle par un radical halogéno, cyano, nitro, alkyle ou alkoxy ayant chacun 1 à 4 atomes de carbone,
R³ représente l'hydrogène ou un groupe alkyle ayant 1 à 4 atomes de carbone,
R⁴ représente un groupe aryle, cycloalkyle ou cycloalcényle ayant 3 à 8 atomes de carbone ou un groupe hétérocyclyle à noyau de 3 à 12 atomes, chacun étant éventuellement substitué une ou plusieurs fois identiques ou différentes, les substituants possibles étant choisis parmi ceux qui sont énumérés ci-après :
halogène, cyano, nitro, amino, hydroxy, formyle, carboxy, carbamoyle, thiocarbamoyle ;
alkyle, alkoxy, alkylthio, alkylsulfinyle ou alkylsulfonyle chacun linéaire ou ramifié ayant chacun 1 à 6 atomes de carbone ;
alcényle ou alcényloxy chacun linéaire ou ramifié ayant chacun 2 à 6 atomes de carbone ;
halogénalkyle, halogénalkoxy, halogénalkylthio, halogénalkylsulfinyle ou halogénalkylsulfonyle chacun linéaire ou ramifié ayant chacun 1 à 6 atomes de carbone et 1 à 13 atomes d'halogènes identiques ou différents ;
halogénalcényle ou halogénalcényloxy chacun linéaire ou ramifié ayant chacun 2 à 6 atomes de carbone et 1 à 13 atomes d'halogènes identiques ou différents ;
alkylamino, dialkylamino, alkylcarbonyle, alkylcarbonyloxy, alkoxycarbonyle, alkylsulfonyloxy, hydroximino-alkyle ou alkoximino-alkyle chacun linéaire ou ramifié, ayant chacun 1 à 6 atomes de carbone dans les parties alkyle individuelles ;
alkylène ou dioxyalkylène ayant chacun deux liaisons et chacun 1 à 6 atomes de carbone, chacun portant le cas échéant un ou plusieurs substituants, identiques ou différents, halogéno et/ou alkyle linéaire ou ramifié ayant 1 à 4 atomes de carbone et halogénalkyle linéaire ou ramifié ayant 1 à 4 atomes de carbone et 1 à 9 atomes d'halogènes identiques ou différents ;
cycloalkyle ayant 3 à 6 atomes de carbone ;
ainsi qu'aryle, aryloxy, arylthio, arylalkyle, arylalkyloxy, arylalkylthio, hétérocyclyle, hétérocyclyloxy, hétérocyclylthio, hétérocyclylalkyle, hétérocyclylalkyloxy ou hétérocyclylalkylthio portant chacun le cas échéant un ou plusieurs substituants, identiques ou différents, halogéno, cyano et/ou alkyle linéaire ou ramifié ayant 1 à 4 atomes de carbone,
et/ou halogénalkyle linéaire ou ramifié ayant 1 à 4 atomes de carbone et 1 à 9 atomes d'halogènes identiques ou différents
et/ou alkoxy ou alkylthio linéaire ou ramifié ayant 1 à 4 atomes de carbone
et/ou halogénalkoxy ou halogénalkylthio linéaire ou ramifié ayant 1 à 4 atomes de carbone et 1 à 9 atomes d'halogènes identiques ou différents
et/ou alkylène ou dioxyalkylène ayant chacun deux liaisons et chacun 1 à 6 atomes de carbone, chacun étant éventuellement substitué une ou plusieurs fois identiques ou différentes par un halogène et/ou un radical alkyle linéaire ou ramifié ayant 1 à 4 atomes de carbone et/ou halogénalkyle linéaire ou ramifié ayant 1 à 4 atomes de carbone et 1 à 9 atomes d'halogène identiques ou différents.

2. Composés suivant la revendication 1, de formule (I-I) dans laquelle
A est une liaison simple ou un groupe méthylène, 1,1-éthylène, 1,2-éthylène, 1,1-, 1,2-, 1,3- ou 2,2-propylène, 1,1-, 1,2-, 1,3-, 1,4-, 2,2-, 2,3-butylène ou 1,1-, 1,2- ou 1,3-(2-méthylpropylène), portant chacun le cas échéant un ou plusieurs substituants fluoro, chloro, cyano ou méthoxy identiques ou différents,
Q représente l'oxygène ou le soufre,
R¹ est un groupe cyclopropyle, cyclobutyle, cyclopentyle, cyclohexyle, phényle, naphtyle, furyle, benzofurannyle, pyrrolyle, indolyle, thiényle, benzothiényle, oxazolyle, isoxazolyle, thiazolyle, isothiazolyle, oxadiazolyle, thiadiazolyle, pyridyle, quinolyle, pyrimidyle, pyridazinyle, pyrazinyle, oxirannyle, oxétanyle, tétrahydrofuryle, perhydropyrannyle, pyrrolidinyle, pipéridinyle ou morpholinyle portant chacun le cas échéant 1 à 3 substituants, les substituants possibles étant choisis parmi ceux qui sont énumérés ci-après :
fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertiobutyle, n-pentyle, n-hexyle, n-heptyle, méthoxy, éthoxy, n-propoxy, isopropoxy, méthylthio, éthylthio, n-propylthio, isopropylthio, méthylsufinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydoximinoéthyle, méthoximino-éthyle ou éthoximino-éthyle ; triméthylène- (propane-1,3-diyle), tétraméthylène- (butane-1,4-diyle), méthylène-dioxy ou éthylènedioxy ayant chacun deux liaisons et portant chacun le cas échéant un ou plusieurs substituants fluoro, chloro, méthyle, tri-fluorométhyle, éthyle, n-propyle ou isopropyle identiques ou différents ;
cyclopropyle, cyclobutyle, cyclopentyle ou cyclohexyle ;
phényle, benzyle, phénoxy, benzyloxy portant chacun les substituants indiqués ci-dessus,
R² représente l'un des groupements suivants : 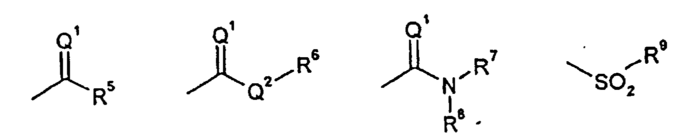 dans lesquels
Q¹ et Q² représentent indépendamment l'un de l'autre l'oxygène ou le soufre,
R⁵ représente l'hydrogène, un groupe méthyle, éthyle, n-propyle, isopropyle, n-butyle, isobutyle, sec.-butyle, tertiobutyle ou un groupe phényle, benzyle, 1-phényléthyle ou 2-phényléthyle dont chacun est éventuellement substitué dans la partie phényle par du fluor, du chlore, du brome, un radical cyano, nitro, méthyle, éthyle, n-propyle, isopropyle, n-butyle, isobutyle, sec.-butyle, tertiobutyle, méthoxy ou éthoxy,
R⁶ est un groupe méthyle, éthyle, n-propyle, isopropyle, n-butyle, isobutyle, sec.-butyle ou tertiobutyle ou un groupe phényle, benzyle, 1-phényléthyle ou 2-phényléthyle, chacun étant éventuellement substitué dans la partie phényle par du fluor, du chlore, du brome, un radical cyano, nitro, méthyle, éthyle, n-propyle, isopropyle, n-butyle, isobutyle, sec.-butyle, tertiobutyle, méthoxy ou éthoxy,
R⁷ représente l'hydrogène, un groupe méthyle, éthyle, n-propyle, isopropyle, n-butyle, isobutyle, sec.-butyle, tertiobutyle ou un groupe phényle, benzyle, 1-phényléthyle ou 2-phényléthyle dont chacun est substitué le cas échéant dans la partie phényle par du fluor, du chlore, du brome, un radical cyano, nitro, méthyle, éthyle, n-propyle, isopropyle, n-butyle, isobutyle, sec.-butyle, tertiobutyle, méthoxy ou éthoxy,
R⁸ est un groupe méthyle, éthyle, n-propyle, isopropyle, n-butyle, isobutyle, sec.-butyle, tertiobutyle ou un groupe phényle, benzyle, 1-phényléthyle ou 2-phényléthyle dont chacun est éventuellement substitué dans la partie phényle par du fluor, du chlore, du brome, un radical cyano, nitro, méthyle, éthyle, n-propyle, isopropyle, n-butyle, isobutyle, sec.-butyle, tertiobutyle, méthoxy ou éthoxy,
R⁷ et R⁸ forment conjointement avec l'atome d'azote auquel ils sont liés un noyau hétérocyclique éventuellement substitué par un radical méthyle,
R⁹ est un groupe méthyle, éthyle, n-propyle, isopropyle, n-butyle, isobutyle, sec.-butyle, tertiobutyle, diméthylamino, diéthylamino, pyrrolidine-1-yle, morpholine-N-yle, pipéridine-1-yle ou un groupe phényle, benzyle, 1-phényléthyle ou 2-phényléthyle dont chacun est éventuellement substitué dans la partie phényle par du fluor, du chlore, du brome, un radical cyano, nitro, méthyle, éthyle, n-propyle, isopropyle, n-butyle, isobutyle, sec.-butyle, tertiobutyle, méthoxy ou éthoxy,
R³ représente l'hydrogène ou un groupe méthyle ou éthyle,
R⁴ est un groupe phényle, naphtyle, furyle, benzofurannyle, pyrrolyle, indolyle, thiényle, benzothiényle, oxazolyle, isoxazolyle, thiazolyle, isothiazolyle, oxadiazolyle, thiadiazolyle, pyridyle, quinolyle, pyrimidyle, pyridazinyle, pyrazinyle, oxirannyle, oxéthanyle, tétra-hydrofuryle, perhydropyrannyle, pyrrolidinyle, pipéridinyle ou morpholinyle portant chacun le cas échéant 1 à 3 substituants, les substituants possibles étant choisis parmi ceux qui sont énumérés ci-après :
fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertiobutyle, méthoxy, éthoxy, n-propoxy, isopropoxy, méthylthio, éthylthio, n-propylthio, isopropylthio, méthylsufinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, iso-propylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximino-éthyle, méthoximino-éthyle ou éthoximino-éthyle ;
triméthylène-(propane-1,3-diyle), tétraméthylène-(butane-1,4-diyle), méthylène-dioxy ou éthylène-dioxy ayant chacun deux liaisons et chacun étant éventuellement substitué une ou plusieurs fois identiques ou différentes par du fluor, du chlore, un radical méthyle, trifluorométhyle, éthyle, n-propyle ou isopropyle,
cyclopropyle, cyclobutyle, cyclopentyle ou cyclohexyle.

3. Composés suivant la revendication 1, de formule (I-I) dans laquelle
A est une liaison simple ou un groupe méthylène, 1,1-éthylène, 1,2-éthylène, 1,1-, 1,2-, 1,3- ou 2,2-propylène, 1,1-, 1,2-, 1,3-, 1,4-, 2,2-, 2,3-butylène ou 1,1-, 1,2- ou 1,3-(2-méthylpropylène), portant chacun le cas échéant un ou plusieurs substituants fluoro, chloro, cyano ou méthoxy identiques ou différents,
Q représente l'oxygène ou le soufre,
R¹ est un groupe cyclobutyle, cyclopentyle, ou cyclohexyle portant chacun le cas échéant 1 à 6 substituants, les substituants étant de préférence ceux qui sont mentionnés ci-dessous ; un groupe phényle, naphtyle, furyle, benzofurannyle, thiényle, benzothiényle, pyridyle, quinolyle, tétrahydrofuryle ou perhydropyrannyle portant chacun le cas échéant 1 à 3 substituants, les substituants possibles étant choisis parmi ceux qui sont énumérés ci-après :
fluor, chlore, brome, cyano, nitro, méthyle, éthyle, n-propyle, isopropyle, n-butyle, isobutyle, sec.-butyle, tertiobutyle, n-pentyle, n-hexyle, n-heptyle, méthoxy, éthoxy, n-propoxy, isopropoxy, méthylthio, éthylthio, n-propylthio, isopropylthio, méthylsufinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, acétyle, propionyle, acétyloxy, méthoxycarbonyle, éthoxycarbonyle, méthylsulfonyloxy, éthylsulfonyloxy, hydroximinométhyle, hydroximinoéthyle, méthoximino-éthyle ou éthoximino-éthyle ;
triméthylène-(propane-1,3-diyle), tétraméthylène-(butane-1,4-diyle), méthylène-dioxy ou éthylènedioxy ayant chacun deux liaisons et portant chacun le cas échéant un ou plusieurs substituants fluoro, chloro, méthyle, trifluorométhyle, éthyle, n-propyle ou isopropyle identiques ou différents ; cyclopropyle, cyclopentyle, cyclohexyle ; phényle, phénoxy, benzyloxy portant éventuellement les substituants mentionnés ci-dessus,
R² représente l'un des groupements suivants : 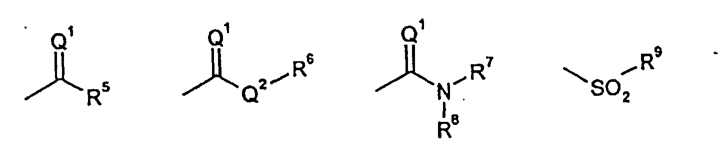 dans lesquels
Q¹ et Q² représentent indépendamment l'un de l'autre l'oxygène ou le soufre,
R⁵ est l'hydrogène, un groupe méthyle, éthyle, n-propyle, isopropyle, n-butyle, isobutyle, sec.-butyle ou tertiobutyle ou un groupe phényle, benzyle, 1-phényléthyle ou 2-phényléthyle dont chacun est éventuellement substitué dans la partie phényle par du fluor, du chlore, du brome, un radical cyano, nitro, méthyle, éthyle, n-propyle, isopropyle, n-butyle, isobutyle, sec.-butyle, tertiobutyle, méthoxy ou éthoxy,
R⁶ représente un groupe méthyle, éthyle, n-propyle, isopropyle, n-butyle, isobutyle, sec.-butyle ou tertiobutyle ou un groupe phényle, benzyle, 1-phényléthyle ou 2-phényléthyle dont chacun est éventuellement substitué dans la partie phényle par du fluor, du chlore, du brome, un radical cyano, nitro, méthyle, éthyle, n-propyle, isopropyle, n-butyle, isobutyle, sec.-butyle, tertiobutyle, méthoxy ou éthoxy,
R⁷ représente l'hydrogène, un groupe méthyle, éthyle, n-propyle, isopropyle, n-butyle, isobutyle, sec.-butyle ou tertiobutyle ou un groupe phényle, benzyle, 1-phényléthyle ou 2-phényléthyle dont chacun est éventuellement substitué dans la partie phényle par du fluor, du chlore, du brome, un radical cyano, nitro, méthyle, éthyle, n-propyle, isopropyle, n-butyle, isobutyle, sec.-butyle, tertiobutyle, méthoxy ou éthoxy,
R⁸ est un groupe méthyle, éthyle, n-propyle, isopropyle, n-butyle, isobutyle, sec.-butyle ou tertiobutyle ou un groupe phényle, benzyle, 1-phényléthyle ou 2-phényléthyle dont chacun est éventuellement substitué dans la partie phényle par du fluor, du chlore, du brome, un radical cyano, nitro, méthyle, éthyle, n-propyle, isopropyle, n-butyle, isobutyle, sec.-butyle, tertiobutyle, méthoxy ou éthoxy, ou bien
R⁷ et R⁸ forment conjointement avec l'atome d'azote auquel ils sont liés un noyau hétérocyclique éventuellement substitué par un radical méthyle,
R⁹ est un groupe méthyle, éthyle, n-propyle, isopropyle, n-butyle, isobutyle, sec.-butyle ou tertiobutyle ou un groupe phényle, benzyle, 1-phényléthyle ou 2-phényléthyle dont chacun est éventuellement substitué dans la partie phényle par du fluor, du chlore, du brome, un radical cyano, nitro, méthyle, éthyle, n-propyle, isopropyle, n-butyle, isobutyle, sec.-butyle, tertiobutyle, méthoxy ou éthoxy,
R³ représente l'hydrogène ou un groupe méthyle,
R⁴ est un groupe phényle, naphtyle, furyle, benzofurannyle, pyrrolyle, indolyle, thiényle, benzothiényle, oxazolyle, isoxazolyle, thiazolyle, isothiazolyle, oxadiazolyle, thiadiazolyle, pyridyle, quinolyle, pyrimidyle, pyridazinyle, pyrazinyle, oxirannyle, oxétannyle, tétrahydrofuryle, perhydropyrannyle, pyrrolidinyle, pipéridinyle ou morpholinyle portant chacun le cas échéant 1 à 3 substituants, les substituants possibles étant choisis parmi ceux qui sont énumérés ci-après :
fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertiobutyle, méthoxy, éthoxy, n-propoxy, isopropoxy, méthylthio, éthylthio, n-propylthio, isopropylthio, méthylsufinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximino-éthyle, méthoximino-éthyle ou éthoximino-éthyle ;
triméthylène-(propane-1,3-diyle), tétraméthylène-(butane-1,4-diyle), méthylène-dioxy ou éthylène-dioxy ayant chacun deux liaisons et portant chacun le cas échéant un ou plusieurs substituants fluoro, chloro, méthyle, trifluorométhyle, éthyle, n-propyle ou isopropyle identiques ou différents ;
cyclopropyle, cyclobutyle, cyclopentyle ou cyclohexyle.

4. Composés suivant la revendication 1, de formule (I-I) dans laquelle
A représente une liaison simple ou un groupe méthylène, 1,1-éthylène, 1,2-éthylène, 1,1-propylène, 1,2-propylène, 1,3-propylène ou 2,2-propylène,
Q est l'oxygène,
R¹ est un groupe phényle, thiényle ou furannyle portant éventuellement 1 ou 2 substituants, identiques ou différents, bromo, chloro, fluoro, nitro, méthylsulfonyle, phényle, phényloxy, benzyloxy, cyclopropyle, cyclohexyle, méthyle, éthyle, n-propyle, isopropyle, n-butyle, isobutyle, sec.-butyle, tertiobutyle, n-pentyle, n-hexyle, n-heptyle, méthoxy, éthoxy, trifluorométhyle, trifluorométhoxy et/ou méthylthio ou un groupe substitué par un radical 3,4-méthylène- et éthylène-dioxo, propane-1,3-dyle et butane-1,4-diyle dont chacun est éventuellement substitué par du fluor, ou un groupe naphtyle, benzofurannyle ou benzothiényle,
R² représente l'un des groupements suivants : 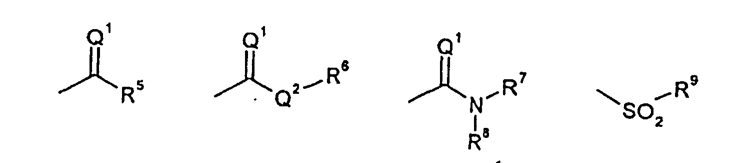 dans lesquels
Q¹ et Q² représentent l'oxygène,
R⁵ représente l'hydrogène, un groupe méthyle, éthyle, n-propyle, isopropyle, n-butyle, isobutyle, sec.-butyle ou tertiobutyle ou un groupe phényle, benzyle, 1-phényléthyle ou 2-phényléthyle dont chacun est éventuellement substitué dans la partie phényle par du fluor, du chlore, du brome, un radical cyano, nitro, méthyle, éthyle, n-propyle, isopropyle, n-butyle, iso-butyle, sec.-butyle, tertiobutyle, méthoxy ou éthoxy,
R⁶ est un groupe méthyle, éthyle, n-propyle, isopropyle, n-butyle, isobutyle, sec.-butyle ou tertiobutyle ou un groupe phényle, benzyle, 1-phényléthyle ou 2-phényléthyle, dont chacun est éventuellement substitué dans la partie phényle par du fluor, du chlore, du brome, un radical cyano, nitro, méthyle, éthyle, n-propyle, isopropyle, n-butyle, isobutyle, sec.-butyle, tertiobutyle, méthoxy ou éthoxy,
R⁷ représente l'hydrogène, un groupe méthyle, éthyle, n-propyle, isopropyle, n-butyle, isobutyle, sec.-butyle, tertiobutyle ou un groupe phényle, benzyle, 1-phényléthyle ou 2-phényléthyle dont chacun est éventuellement substitué dans la partie phényle par du fluor, du chlore, du brome, un radical cyano, nitro, méthyle, éthyle, n-propyle, isopropyle, n-butyle, isobutyle, sec.-butyle, tertiobutyle, méthoxy ou éthoxy,
R⁸ est un groupe méthyle, éthyle, n-propyle, isopropyle, n-butyle, isobutyle, sec.-butyle, tertiobutyle ou un groupe phényle, benzyle, 1-phényléthyle ou 2-phényléthyle dont chacun est éventuellement substitué dans la partie phényle par du fluor, du chlore, du brome, un radical cyano, nitro, méthyle, éthyle, n-propyle, isopropyle, n-butyle, isobutyle, sec.-butyle, tertiobutyle, méthoxy ou éthoxy, ou bien
R⁷ et R⁸ forment conjointement avec l'atome d'azote auquel ils sont liés un noyau hétérocyclique éventuellement substitué par un radical méthyle,
R⁹ est un groupe méthyle, éthyle, n-propyle, isopropyle, n-butyle, isobutyle, sec.-butyle, tertiobutyle, diméthylamino, diéthylamino, pyrrolidine-1-yle, morpholine-N-yle, pipéridine-1-yle ou un groupe phényle, benzyle, 1-phényléthyle ou 2-phényléthyle dont chacun est éventuellement substitué dans la partie phényle par du fluor, du chlore, du brome, un radical cyano, nitro, méthyle, éthyle, n-propyle, isopropyle, n-butyle, isobutyle, sec.-butyle, tertiobutyle, méthoxy ou éthoxy,
R³ représente l'hydrogène ou un groupe méthyle,
R⁴ est un groupe cyclohexyle ou un groupe phényle, thiényle, furyle, benzofuryle, benzothiényle, pyridyle, pyrimidyle, naphtyle, quinolyle dont chacun porte le cas échéant 1 à 3 substituants, les substituants possibles étant choisis parmi ceux qui sont énumérés ci-après :
fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertiobutyle, méthoxy, éthoxy, n-propoxy, isopropoxy, méthylthio, éthylthio, n-propylthio, isopropylthio, méthylsufinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximino-éthyle, méthoximino-éthyle ou éthoximino-éthyle ;
triméthylène-(propane-1,3-diyle), tétraméthylène-(butane-1,4-diyle), méthylènedioxy ou éthylène-dioxy ayant chacun deux liaisons et portant chacun le cas échéant un ou plusieurs substituants fluoro, chloro, méthyle, trifluorométhyle, éthyle, n-propyle ou isopropyle identiques ou différents.

5. Composés de formule (I-I) 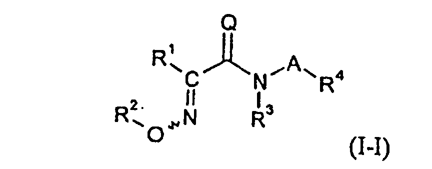 dans laquelle
A représente une liaison simple ou un groupe alkylène ayant 1 à 6 atomes de carbone portant éventuellement un ou plusieurs substituants identiques ou différentes, les substituants possibles étant choisis parmi ceux qui sont énumérés ci-après :
halogène, cyano, nitro, amino, hydroxy, formyle, carboxy, carbamoyle, thiocarbamoyle ;
alkoxy, alkylthio, alkylsulfinyle ou alkylsulfonyle chacun linéaire ou ramifié et ayant chacun 1 à 6 atomes de carbone ;
alcényle ou alcényloxy chacun linéaire ou ramifié ayant chacun 2 à 6 atomes de carbone ;
halogénalkoxy, halogénalkylthio, halogénalkylsulfinyle ou halogénalkylsulfonyle chacun linéaire ou ramifié ayant chacun 1 à 6 atomes de carbone et 1 à 13 atomes d'halogènes identiques ou différents ;
halogénalcényle ou halogénalcényloxy chacun linéaire ou ramifié ayant chacun 2 à 6 atomes de carbone et 1 à 13 atomes d'halogènes identiques ou différents ;
alkylamino, dialkylamino, alkylcarbonyle, alkylcarbonyloxy, alkoxycarbonyle, alkylsulfonyloxy, hydroximino-alkyle ou alkoximino-alkyle chacun linéaire ou ramifié ayant chacun 1 à 6 atomes de carbone dans les parties alkyle individuelles ;
cycloalkyle ayant 3 à 6 atomes de carbone ;
ainsi qu'aryle ou hétérocyclyle portant chacun le cas échéant un ou plusieurs substituants, identiques ou différents
halogéno, cyano et/ou alkyle linéaire ou ramifié ayant 1 à 4 atomes de carbone
et/ou halogénalkyle linéaire ou ramifié ayant 1 à 4 atomes de carbone et 1 à 9 atomes d'halogènes identiques ou différents
et/ou alkoxy ou alkylthio linéaire ou ramifié ayant 1 à 4 atomes de carbone
et/ou halogénalkoxy ou halogénalkylthio linéaire ou ramifié ayant 1 à 4 atomes de carbone et 1 à 9 atomes d'halogènes identiques ou différents et/ou alkylène ou dioxyalkylène ayant chacun deux liaisons et chacun 1 à 6 atomes de carbone, portant chacun un ou plusieurs substituants, identiques ou différents, halogéno et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents,
Q est l'oxygène ou le soufre,
R¹ est un groupe benzo-hétérocyclyle ayant 3 à 12 chaînons dans la partie hétérocyclyle et 1, 2 ou 3 hétéro-atomes avec toutefois au maximum un atome d'oxygène comme hétéro-atome), lié par le noyau benzénique et substitué le cas échéant une ou plusieurs fois identiques ou différentes, les substituants possibles étant choisis parmi ceux qui sont énumérés ci-après :
halogène, cyano, nitro, amino, hydroxy, formyle, carboxy, carbamoyle, thiocarbamoyle ;
alkyle, alkoxy, alkylthio, alkylsulfinyle ou alkylsulfonyle chacun linéaire ou ramifié ayant chacun 1 à 8 atomes de carbone ;
alcényle ou alcényloxy chacun linéaire ou ramifié ayant chacun 2 à 6 atomes de carbone ;
halogénalkyle, halogénalkoxy, halogénalkylthio, halogénalkylsulfinyle ou halogénalkylsulfonyle chacun linéaire ou ramifié ayant chacun 1 à 6 atomes de carbone et 1 à 13 atomes d'halogènes identiques ou différents ;
halogénalcényle ou halogénalcényloxy chacun linéaire ou ramifié ayant chacun 2 à 6 atomes de carbone et 1 à 13 atomes d'halogènes identiques ou différents ;
alkylamino, dialkylamino, alkylcarbonyle, alkylcarbonyloxy, alkoxycarbonyle, alkylsulfonyloxy, hydroximino-alkyle ou alkoximino-alkyle chacun linéaire ou ramifié, ayant chacun 1 à 8 atomes de carbone dans les parties alkyle individuelles ;
alkylène ou dioxyalkylène ayant chacun deux liaisons et chacun 1 à 6 atomes de carbone, portant chacun le cas échéant un ou plusieurs substituants, identiques ou différents, halogéno et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents ;
cycloalkyle ayant 3 à 6 atomes de carbone ;
ainsi qu'aryle, aryloxy, arylthio, arylalkyle, arylalkyloxy, arylalkylthio, hétérocyclyle, hétérocyclyloxy, hétérocyclylthio, hétérocyclylalkyle, hétérocyclylalkyloxy ou hétérocyclylalkylthio portant chacun le cas échéant un ou plusieurs substituants, identiques ou différents, halogéno, cyano et/ou alkyle linéaire ou ramifié ayant 1 à 4 atomes de carbone
et/ou halogénalkyle linaire ou ramifié ayant 1 à 4 atomes de carbone et 1 à 9 atomes d'halogènes identiques ou différents
et/ou alkoxy ou alkylthio linéaire ou ramifié ayant 1 à 4 atomes de carbone
et/ou halogénalkoxy ou halogénalkylthio linéaire ou ramifié ayant 1 à 4 atomes de carbone et 1 à 9 atomes d'halogènes identiques ou différents
et/ou alkylène ou dioxyalkylène ayant chacun deux liaisons et chacun 1 à 6 atomes de carbone, portant chacun le cas échéant un ou plusieurs substituants, identiques ou différents, halogéno, et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents,
R² représente l'un des groupements suivants : 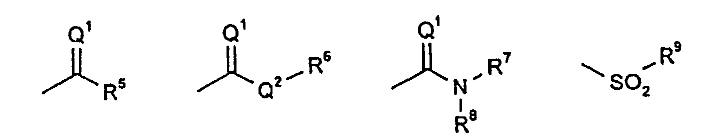 dans lesquels
Q¹ et Q² représentent indépendamment l'un de l'autre l'oxygène ou le soufre,
R⁵ représente l'hydrogène ou un groupe alkyle ayant 1 à 6 atomes de carbone, ou bien un groupe aryle, ou un groupe arylalkyle ayant 1 à 4 atomes de carbone dans la partie alkyle, dont chacun est éventuellement substitué dans la partie aryle par un halogène, un radical cyano, nitro, alkyle ou alkoxy ayant chacun 1 à 4 atomes de carbone,
R⁶ est un groupe alkyle ayant 1 à 6 atomes de carbone ou bien un groupe aryle ou un groupe arylalkyle ayant 1 à 4 atomes de carbone dans la partie alkyle, portant chacun le cas échéant dans la partie aryle un substituant halogéno, cyano, nitro, alkyle ou alkoxy ayant chacun 1 à 4 atomes de carbone,
R⁷ représente l'hydrogène, un groupe alkyle ayant 1 à 6 atomes de carbone ou bien un groupe aryle ou un groupe arylalkyle ayant 1 à 4 atomes de carbone dans la partie alkyle, portant chacun le cas échéant dans la partie aryle un substituant halogéno, cyano, nitro, alkyle ou alkoxy ayant chacun 1 à 4 atomes de carbone,
R⁸ est un groupe alkyle ayant 1 à 6 atomes de carbone ou bien un groupe aryle ou arylalkyle ayant 1 à 4 atomes de carbone dans la partie alkyle, portant chacun le cas échéant dans la partie aryle un substituant halogéno, cyano, nitro, alkyle, ou alkoxy ayant chacun 1 à 4 atomes de carbone, ou bien
R⁷ et R⁸ forment conjointement avec l'atome d'azote auquel ils sont liés, un noyau hétérocyclique éventuellement substitué par un radical méthyle,
R⁹ est un groupe alkyle ayant 1 à 6 atomes de carbone, dialkylamino ayant 1 à 4 atomes de carbone dans chacune des parties alkyle individuelles, un groupe hétérocyclyle à cycle de 3 à 7 atomes lié par de l'azote ou un groupe aryle, ou arylalkyle ayant 1 à 4 atomes de carbone dans la partie alkyle, chacun étant éventuellement substitué dans la partie aryle par un radical halogéno, cyano, nitro, alkyle ou alkoxy ayant chacun 1 à 4 atomes de carbone,
R³ est l'hydrogène ou un groupe alkyle ayant 1 à 4 atomes de carbone,
R⁴ est un groupe aryle, cycloalkyle ou cycloalcényle de 3 à 8 atomes de carbone ou un groupe hétérocyclyle à noyau de 3 à 12 atomes portant chacun le cas échéant un ou plusieurs substituants identiques ou différents, les substituants possibles étant choisis parmi ceux qui sont énumérés ci-après :
halogène, cyano, nitro, amino, hydroxy, formyle, carboxy, carbamoyle, thiocarbamoyle ;
alkyle, alkoxy, alkylthio, alkylsulfinyle ou alkylsulfonyle chacun linéaire ou ramifié ayant chacun 1 à 6 atomes de carbone ;
alcényle ou alcényloxy chacun linéaire ou ramifié ayant chacun 2 à 6 atomes de carbone ;
halogénalkyle, halogénalkoxy, halogénalkylthio, halogénalkylsulfinyle ou halogénalkylsulfonyle chacun linéaire ou ramifié ayant chacun 1 à 6 atomes de carbone et 1 à 13 atomes d'halogènes identiques ou différents ;
halogénalcényle ou halogénalcényloxy chacun linéaire ou ramifié ayant chacun 2 à 6 atomes de carbone et 1 à 13 atomes d'halogènes identiques ou différents ;
alkylamino, dialkylamino, alkylcarbonyle, alkylcarbonyloxy, alkoxycarbonyle, alkylsulfonyloxy, hydroximino-alkyle ou alkoximino-alkyle chacun linéaire ou ramifié, ayant chacun 1 à 6 atomes de carbone dans les parties alkyle individuelles ;
alkylène ou dioxyalkylène ayant chacun deux liaisons et chacun 1 à 6 atomes de carbone, chacun portant le cas échéant un ou plusieurs substituants, identiques ou différents, halogéno et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents ;
cycloalkyle ayant 3 à 6 atomes de carbone ;
ainsi qu'aryle, aryloxy, arylthio, arylalkyle, arylalkyloxy, arylalkylthio, hétérocyclyle, hétérocyclyloxy, hétérocyclylthio, hétérocyclylalkyle, hétérocyclylalkyloxy ou hétérocyclylalkylthio portant chacun le cas échéant un ou plusieurs substituants, identiques ou différents, halogéno, cyano et/ou alkyle linéaire ou ramifié ayant 1 à 4 atomes de carbone,
et/ou halogénalkyle linéaire ou ramifié ayant 1 à 4 atomes de carbone et 1 à 9 atomes d'halogènes identiques ou différents
et/ou alkoxy ou alkylthio linéaire ou ramifié ayant 1 à 4 atomes de carbone
et/ou halogénalkoxy ou halogénalkylthio linéaire ou ramifié ayant 1 à 4 atomes de carbone et 1 à 9 atomes d'halogènes identiques ou différents
et/ou alkylène ou dioxyalkylène ayant chacun deux liaisons et chacun 1 à 6 atomes de carbone, chacun portant le cas échéant un ou plusieurs substituants, identiques ou différents, halogéno et/ou alkyle linéaire ou ramifié ayant 1 à 4 atomes de carbone et/ou halogénalkyle linéaire ou ramifié ayant 1 à 4 atomes de carbone et 1 à 9 atomes d'halogène identiques ou différents.

6. Composés suivant la revendication 5, de formule (I-I) dans laquelle
A esc une liaison simple ou un groupe méthylène, 1,1-éthylène, 1,2-éthylène, 1,1-, 1,2-, 1,3- ou 2,2-propylène, 1,1-, 1,2-, 1,3-, 1,4-, 2,2-, 2,3-butylène ou 1,1-, 1,2- ou 1,3-(2-méthylpropylène), portant chacun le cas échéant un ou plusieurs substituants fluoro, chloro, cyano ou méthoxy identiques ou différents,
Q est l'oxygène ou le soufre,
R¹ représente l'un des groupements suivants 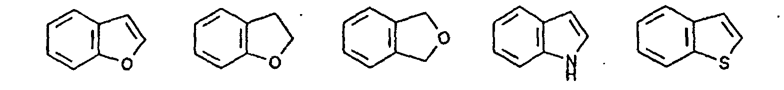 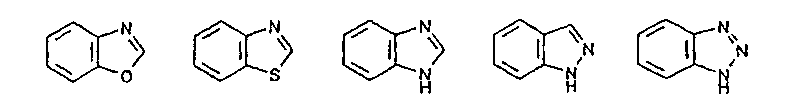 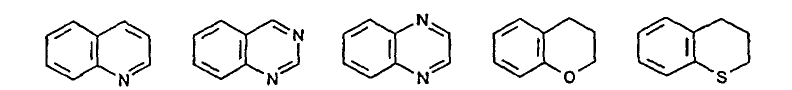 dont chacun est lié par un atome de carbone du noyau benzénique correspondant et qui peuvent être substitués le cas échéant une à trois fois identiques ou différentes par les substituants énumérés ci-après :
fluor, chlore, brome, cyano, nitro, amino, hydroxy, formyle, carboxy, carbamoyle, thiocarbamoyle, thiocarbamoyle, méthyle, éthyle, n-propyle, isopropyle, n-butyle, isobutyle, sec.-butyle, tertiobutyle, n-pentyle, n-hexyle, n-heptyle, méthoxy, éthoxy, n-propoxy, isopropoxy, méthylthio, éthylthio, n-propylthio, isopropylthio, méthylsufinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximino-éthyle, méthoximino-éthyle ou éthoximino-éthyle ;
triméthylène-(propane-1,3-diyle), tétraméthylène-(butane-1,4-diyle), méthylène-dioxy ou éthylène-dioxy ayant chacun deux liaisons et portant chacun le cas échéant un ou plusieurs substituants fluoro, chloro, méthyle, trifluorométhyle, éthyle, n-propyle ou isopropyle identiques ou différents ;
cyclopropyle, cyclobutyle, cyclopentyle ou cyclohexyle ;
phényle, benzyle, phénoxy, benzyloxy, cyclopropyle, cyclobutyle, cyclopentyle ou cyclohexyle portant éventuellement les substituants mentionnés ci-dessus,
R² représente l'un des groupements suivants : 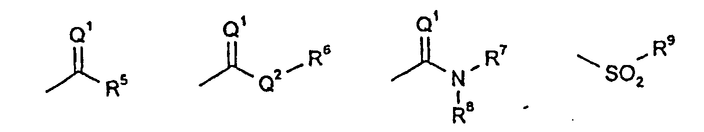 dans lesquels
Q¹ et Q² représentent indépendamment l'un de l'autre l'oxygène ou le soufre,
R⁵ est l'hydrogène ou un groupe méthyle, éthyle, n-propyle, isopropyle, n-butyle, isobutyle, sec.-butyle, tertiobutyle ou un groupe phényle, benzyle, 1-phényléthyle ou 2-phényléthyle dont chacun est éventuellement substitué dans la partie phényle par du fluor, du chlore, du brome, un radical cyano, nitro, méthyle, éthyle, n-propyle, isopropyle, n-butyle, isobutyle, sec.-butyle, tertiobutyle, méthoxy ou éthoxy,
R⁶ est un groupe méthyle, éthyle, n-propyle, isopropyle, n-butyle, isobutyle, sec.-butyle, tertiobutyle ou un groupe phényle, benzyle, 1-phényléthyle ou 2-phényléthyle éventuellement substitué dans la partie phényle par du fluor, du chlore, du brome, un radical cyano, nitro, méthyle, éthyle, n-propyle, isopropyle, n-butyle, isobutyle, sec.-butyle, tertiobutyle, méthoxy ou éthoxy,
R⁷ représente l'hydrogène, un groupe méthyle, éthyle, n-propyle, isopropyle, n-butyle, isobutyle, sec.-butyle, tertiobutyle ou un groupe phényle, benzyle, 1-phényléthyle ou 2-phényléthyle portant chacun le cas échéant dans la partie phényle un substituant fluoro, chloro, bromo, cyano, nitro, méthyle, éthyle, n-propyle, isopropyle, n-butyle, isobutyle, sec.-butyle, tertiobutyle, méthoxy ou éthoxy,
R⁸ est un groupe méthyle, éthyle, n-propyle, isopropyle, n-butyle, isobutyle, sec.-butyle, tertiobutyle ou un groupe phényle, benzyle, 1-phényléthyle ou 2-phényléthyle dont chacun est éventuellement substitué dans la partie phényle par du fluor, du chlore, du brome, un radical cyano, nitro, méthyle, éthyle, n-propyle, isopropyle, n-butyle, isobutyle, sec.-butyle, tertiobutyle, méthoxy ou éthoxy, ou bien
R⁷ et R⁸ forment conjointement avec l'atome d'azote auquel ils sont liés un noyau hétérocyclique éventuellement substitué par un radical méthyle,
R⁹ est un groupe méthyle, éthyle, n-propyle, isopropyle, n-butyle, isobutyle, sec.-butyle, tertiobutyle, diméthylamino, diéthylamino, pyrrolidine-1-yle, morpholine-N-yle, pipéridine-1-yle ou un groupe phényle, benzyle, 1-phényléthyle ou 2-phényléthyle dont chacun est éventuellement substitué dans la partie phényle par du fluor, du chlore, du brome, un radical cyano, nitro, méthyle, éthyle, n-propyle, isopropyle, n-butyle, isobutyle, sec.-butyle, tertiobutyle, méthoxy ou éthoxy,
R³ représente l'hydrogène ou un groupe méthyle ou éthyle,
R⁴ est un groupe phényle, naphtyle, furyle, benzofurannyle, pyrrolyle, indolyle, thiényle, benzothiényle, oxazolyle, isoxazolyle, thiazolyle, isothiazolyle, oxadiazolyle, thiadiazolyle, pyridyle, quinolyle, pyrimidyle, pyridazinyle, pyrazinyle, oxirannyle, oxétannyle, tétrahydrofuryle, perhydropyrannyle, pyrrolidinyle, pipéridinyle ou morpholinyle dont chacun porte éventuellement 1 à 3 substituants, les substituants possibles étant choisis parmi ceux qui sont énumérés ci-après :
fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertiobutyle, méthoxy, éthoxy, n-propoxy, isopropoxy, méthylthio, éthylthio, n-propylthio, iso-propylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximino-éthyle, méthoximinoéthyle ou éthoximino-éthyle ;
triméthylène-(propane-1,3-diyle), tétraméthylène-(butane-1,4-diyle), méthylène-dioxy ou éthylène-dioxy ayant chacun deux liaisons et portant chacun le cas échéant un ou plusieurs substituants fluoro, chloro, méthyle, trifluorométhyle, éthyle, n-propyle ou isopropyle identiques ou différents ;
cyclopropyle, cyclobutyle, cyclopentyle ou cyclohexyle.

7. Composés suivant la revendication 5, de formule (I-I) dans laquelle
A est une liaison simple ou un groupe méthylène, 1,1-éthylène, 1,2-éthylène, 1,1-, 1,2-, 1,3- ou 2,2-propylène, 1,1-, 1,2-, 1,3-, 1,4-, 2,2- ou 2,3-butylène ou 1,1-, 1,2- ou 1,3-(2-méthylpropylène) portant chacun le cas échéant un ou plusieurs substituants fluoro, chloro, cyano ou méthoxy identiques ou différents,
Q représente l'oxygène ou le soufre,
R¹ représente l'un des groupements suivants 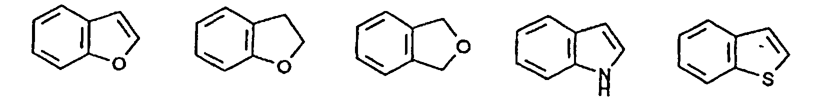 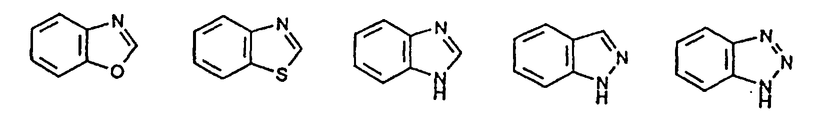 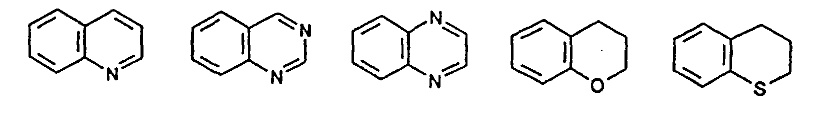 dont chacun est lié par un atome de carbone du noyau benzénique correspondant et qui peuvent être substitués chacun le cas échéant une à trois fois identiques ou différentes par les substituants énumérés ci-après :
fluor, chlore, brome, cyano, nitro, méthyle, éthyle, n-propyle, isopropyle, n-butyle, isobutyle, sec.-butyle, tertiobutyle, n-pentyle, n-hexyle, n-heptyle, méthoxy, éthoxy, n-propoxy, isopropoxy, méthylthio, éthylthio, n-propylthio, isopropylthio, méthylsufinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, acétyle, propionyle, acétyloxy, méthoxycarbonyle, éthoxycarbonyle, méthylsulfonyloxy, éthylsulfonyloxy, hydroximinométhyle, hydroximino-éthyle, méthoximino-éthyle ou éthoximino-éthyle ;
triméthylène-(propane-1,3-diyle), tétraméthylène-(butane-1,4-diyle), méthylène-dioxy ou éthylène-dioxy ayant chacun deux liaisons et portant chacun le cas échéant un ou plusieurs substituants fluoro, chloro, méthyle, trifluorométhyle, éthyle, n-propyle ou isopropyle identiques ou différents ;
cyclopropyle, cyclopentyle, cyclohexyle ;
phényle, phénoxy, benzyloxy, portant éventuellement les substituants indiqués ci-dessus,
R² représente l'un des groupements suivants : 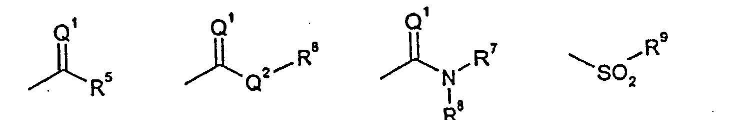 dans lesquels
Q¹ et Q² représentent indépendamment l'un de l'autre l'oxygène ou le soufre,
R⁵ est l'hydrogène ou un groupe méthyle, éthyle, n-propyle, isopropyle, n-butyle, isobutyle, sec.-butyle, tertiobutyle ou bien un groupe phényle, benzyle, 1-phényléthyle ou 2-phényléthyle portant chacun le cas échéant dans la partie phényle un substituant fluoro, chloro, bromo, cyano nitro, méthyle, éthyle, n-propyle, isopropyle, n-butyle, isobutyle, sec.-butyle, tertiobutyle, méthoxy ou éthoxy,
R⁶ est un groupe méthyle, éthyle, n-propyle, isopropyle, n-butyle, isobutyle, sec.-butyle, tertiobutyle ou un groupe phényle, benzyle, 1-phényléthyle 'ou 2-phényléthyle portant chacun le cas échéant dans la partie phényle un substituant fluoro, chloro, bromo, cyano, nitro, méthyle, éthyle, n-propyle, isopropyle, n-butyle, isobutyle, sec.-butyle, tertiobutyle, méthoxy ou éthoxy,
R⁷ représente l'hydrogène, un groupe méthyle, éthyle, n-propyle, isopropyle, n-butyle, isobutyle, sec.-butyle, tertiobutyle ou un groupe phényle, benzyle, 1-phényléthyle ou 2-phényléthyle portant chacun le cas échéant dans la partie phényle un substituant fluoro, chloro, bromo, cyano, nitro, méthyle, éthyle, n-propyle, isopropyle, n-butyle, isobutyle, sec.-butyle, tertiobutyle, méthoxy ou éthoxy,
R⁸ est un groupe méthyle, éthyle, n-propyle, isopropyle, n-butyle, isobutyle, sec.-butyle, tertiobutyle ou un groupe phényle, benzyle, 1-phényléthyle ou 2-phényléthyle portant chacun le cas échéant dans la partie phényle un substituant fluoro, chloro, bromo, cyano, nitro, méthyle, éthyle, n-propyle, isopropyle, n-butyle, isobutyle, sec.-butyle, tertiobutyle, méthoxy ou éthoxy, ou bien
R⁷ et R⁸ forment conjointement avec l'atome d'azote auquel ils sont liés un noyau hétérocyclique éventuellement substitué par un radical méthyle,
R⁹ est un groupe méthyle, éthyle, n-propyle, isopropyle, n-butyle, isobutyle, sec.-butyle, tertiobutyle, diméthylamino, diéthylamino, pyrrolidine-1-yle, morpholine-N-yle, pipéridine-1-yle ou un groupe phényle, benzyle, 1-phényléthyle ou 2-phényléthyle dont chacun est éventuellement substitué dans la partie phényle par du fluor, du chlore, du brome, un radical cyano, nitro, méthyle, éthyle, n-propyle, isopropyle, n-butyle, isobutyle, sec.-butyle, tertiobutyle, méthoxy ou éthoxy,
R³ représente l'hydrogène ou un groupe méthyle,
R⁴ est un groupe phényle, naphtyle, furyle, benzofurannyle, pyrrolyle, indolyle, thiényle, benzothiényle, oxazolyle, isoxazolyle, thiazolyle, isothiazolyle, oxadiazolyle, thiadiazolyle, pyridyle, quinolyle, pyrimidyle, pyridazinyle, pyrazinyle, oxirannyle, oxétannyle, tétrahydrofuryle, perhydropyrannyle, pyrrolidinyle, pipéridinyle ou morpholinyle portant chacun le cas échéant un à trois substituants, les substituants possibles étant choisis parmi ceux qui sont énumérés ci-après :
fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertiobutyle, méthoxy, éthoxy, n-propoxy, isopropoxy, méthylthio, éthylthio, n-propylthio, isopropylthio, méthylsufinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximino-éthyle, méthoximino-éthyle, éthoximino-éthyle, triméthylène-(propane-1,3-diyle), tétraméthylène-(butane-1,4-diyle), méthylène-dioxy ou éthylène-dioxy ayant chacun deux liaisons et portant chacun le cas échéant un ou plusieurs substituants fluoro, chloro, méthyle, trifluorométhyle, éthyle, n-propyle ou isopropyle identiques ou différents,
cyclopropyle, cyclobutyle, cyclopentyle ou cyclohexyle.

8. Composés suivant la revendication 5, de formule (I-I) dans laquelle
A représente une liaison simple ou un groupe méthylène, 1,1-éthylène, 1,2-éthylène, 1,1-propylène, 1,2-propylène, 1,3-propylène ou 2,2-propylène,
Q est l'oxygène,
R¹ représente l'un des groupements suivants 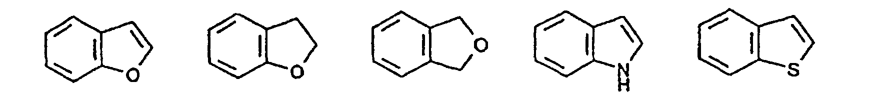 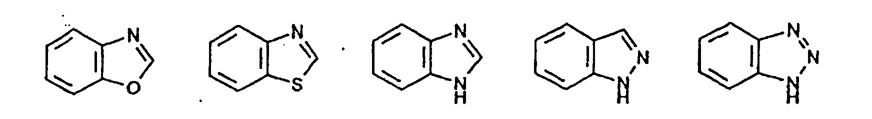 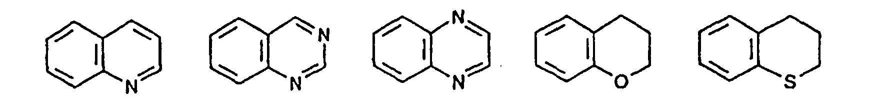 dont chacun est lié par un atome de carbone du noyau benzénique correspondant et dont chacun peut être substitué le cas échéant une à trois fois identiques ou différentes par les substituants énumérés ci-après :
brome, chlore, fluor, nitro, méthylsulfonyle, phényle, phényloxy, benzyloxy, cyclopropyle, cyclohexyle, méthyle, éthyle, n-propyle, isopropyle, n-butyle, isobutyle, sec.-butyle, tertiobutyle, n-pentyle, n-hexyle, n-heptyle, méthoxy, éthoxy, trifluorométhyle, trifluorométhoxy et/ou méthylthio,
R² représente l'un des groupements suivants : 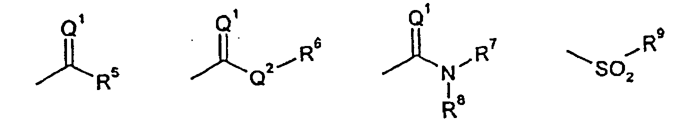 dans lesquels
Q¹ et Q² représentent l'oxygène,
R⁵ est l'hydrogène ou un groupe méthyle, éthyle, n-propyle, isopropyle, n-butyle, isobutyle, sec.-butyle, tertiobutyle ou un groupe phényle, benzyle, 1-phényléthyle ou 2-phényléthyle dont chacun est éventuellement substitué dans la partie phényle par du fluor, du chlore, du brome, un radical cyano, nitro, méthyle, éthyle, n-propyle, isopropyle, n-butyle, isobutyle, sec.-butyle, tertiobutyle, méthoxy ou éthoxy,
R⁶ est un groupe méthyle, éthyle, n-propyle, isopropyle, n-butyle, isobutyle, sec.-butyle ou tertiobutyle ou un groupe phényle, benzyle, 1-phényléthyle ou 2-phényléthyle portant chacun le cas échéant dans la partie phényle un substituant fluoro, chloro, bromo, cyano, nitro, méthyle, éthyle, n-propyle, isopropyle, n-butyle, isobutyle, sec.-butyle, tertiobutyle, méthoxy ou éthoxy,
R⁷ est l'hydrogène, un groupe méthyle, éthyle, n-propyle, isopropyle, n-butyle, isobutyle, sec.-butyle, tertiobutyle ou un groupe phényle, benzyle, 1-phényléthyle ou 2-phényléthyle portant chacun le cas échéant dans la partie phényle un substituant fluoro, chloro, bromo, cyano, nitro, méthyle, éthyle, n-propyle, isopropyle, n-butyle, isobutyle, sec.-butyle, tertiobutyle, méthoxy ou éthoxy,
R⁸ est un groupe méthyle, éthyle, n-propyle, isopropyle, n-butyle, isobutyle, sec.-butyle, tertiobutyle ou un groupe phényle, benzyle, 1-phényléthyle ou 2-phényléthyle portant chacun le cas échéant dans la partie phényle un substituant fluoro, chloro, bromo, cyano, nitro, méthyle, éthyle, n-propyle, isopropyle, n-butyle, isobutyle, sec.-butyle, tertiobutyle, méthoxy ou éthoxy, ou bien
R⁷ et R⁸ forment conjointement avec l'atome d'azote auquel ils sont liés un noyau hétérocyclique éventuellement substitué par un radical méthyle,
R⁹ est un groupe méthyle, éthyle, n-propyle, isopropyle, n-butyle, isobutyle, sec.-butyle, tertiobutyle, diméthylamino, diéthylamino, pyrrolidine-1-yle, morpholine-N-yle, pipéridine-1-yle ou un groupe phényle, benzyle, 1-phényléthyle ou 2-phényléthyle portant chacun dans le cas échéant dans la partie phényle un radical fluoro, chloro, bromo, cyano, nitro, méthyle, éthyle, n-propyle, isopropyle, n-butyle, isobutyle, sec.-butyle, tertiobutyle, méthoxy ou éthoxy;
R³ représente l'hydrogène ou un groupe méthyle,
R⁴ est un groupe cyclohexyle ou un groupe phényle, thiényle, furyle, benzofuryle, benzothiényle, pyridyle, pyrimidinyle, naphtyle, quinolyle, portant chacun le cas échéant un à trois substituants, les substituants possibles étant choisis parmi ceux qui sont énumérés ci-après :
fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertiobutyle, méthoxy, éthoxy, n-propoxy, isopropoxy, méthylthio, éthylthio, n-propylthio, isopropylthio, méthylsufinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximino-éthyle, méthoximino-éthyle ou éthoximino-éthyle ;
triméthylène-(propane-1,3-diyle), tétraméthylène-(butane-1,4-diyle), méthylène-dioxy ou éthylène-dioxy ayant chacun deux liaisons et portant chacun le cas échéant un ou plusieurs substituants, identiques ou différents, fluoro, chloro, méthyle, trifluorométhyle, éthyle, n-propyle ou isopropyle.

9. Composés de formule (I-I) dans laquelle 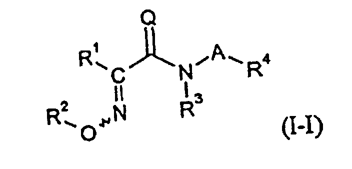 dans laquelle
A représente une liaison simple ou un groupe alkylène ayant 1 à 6 atomes de carbone, portant éventuellement un ou plusieurs substituants identiques ou différents, les substituants possibles étant choisis parmi ceux qui sont énumérés ci-après :
halogène, cyano, nitro, amino, hydroxy, formyle, carboxy, carbamoyle, thiocarbamoyle ;
alkoxy, alkylthio, alkylsulfinyle ou alkylsulfonyle chacun linéaire ou ramifié ayant chacun 1 à 6 atomes de carbone ;
alcényle ou alcényloxy chacun linéaire ou ramifié ayant chacun 2 à 6 atomes de carbone ;
halogénalkoxy, halogénalkylthio, halogénalkylsulfinyle ou halogénalkylsulfonyle chacun linéaire ou ramifié ayant chacun 1 à 6 atomes de carbone et 1 à 13 atomes d'halogènes identiques ou différents ;
halogénalcényle ou halogénalcényloxy chacun linéaire ou ramifié ayant chacun 2 à 6 atomes de carbone et 1 à 13 atomes d'halogènes identiques ou différents ;
alkylamino, dialkylamino, alkylcarbonyle, alkylcarbonyloxy, alkoxycarbonyle, alkylsulfonyloxy, hydroximino-alkyle ou alkoximino-alkyle chacun linéaire ou ramifié, ayant chacun 1 à 6 atomes de carbone dans les parties alkyle individuelles ;
cycloalkyle ayant 3 à 6 atomes de carbone ;
ainsi qu'aryle ou hétérocyclyle portant chacun le cas échéant un ou plusieurs substituants, identiques ou différents,
halogéno, cyano et/ou alkyle linéaire ou ramifié ayant 1 à 4 atomes de carbone
et/ou halogénalkyle linéaire ou ramifié ayant-1 à 4 atomes de carbone et 1 à 9 atomes d'halogènes identiques ou différents
et/ou alkoxy ou alkylthio linéaire ou ramifié ayant 1 à 4 atomes de carbone
et/ou halogénalkoxy ou halogénalkylthio linéaire ou ramifié ayant 1 à 4 atomes de carbone et 1 à 9 atomes d'halogènes identiques ou différents
et/ou alkylène ou dioxyalkylène ayant chacun deux liaisons et chacun 1 à 6 atomes de carbone, chacun portant le cas échéant un ou plusieurs substituants, identiques ou différents, halogéno et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents,
Q représente l'oxygène ou le soufre,
R¹ est un radical tricyclique 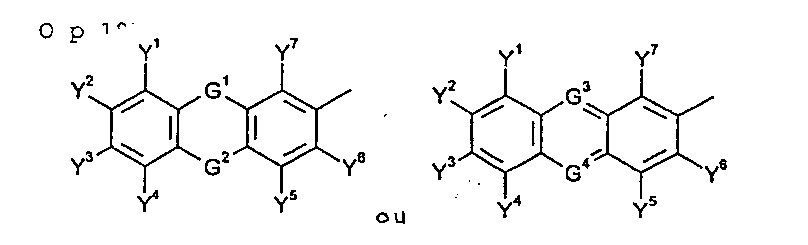 dans lequel
G¹ et G² représentent indépendamment l'un de l'autre une liaison simple, un groupe alcane-diyle, alcène-diyle, l'oxygène, le soufre, un groupe -NH-, -N(alkyl)- ou carbonyle,
G³ et G⁴ représentent indépendamment l'un de l'autre l'azote ou un groupement
Y¹, Y², Y³, Y⁴, Y⁵, Y⁶, Y⁷ et Y⁸ représentent indépendamment les uns des autres l'hydrogène, un halogène, un groupe cyano, nitro ; un groupe alkyle, alkoxy, alkylamino, dialkylamino, alkylthio, alkylsulfinyle ou alkylsulfonyle ayant chacun 1 à 8 atomes de carbone dans les parties alkyle individuelles ;
un groupe halogénalkyle, halogénalkoxy, halogénalkylthio, halogénalkylthio, halogénalkylthio, halogénalkylsulfinyle ou halogénalkylsulfonyle ayant chacun 1 à 6 atomes de carbone et 1 à 13 atomes d'halogènes identiques ou différents, ou bien
un groupe cycloalkyle ayant 3 à 6 atomes de carbone
R² représente l'un des groupements suivants : 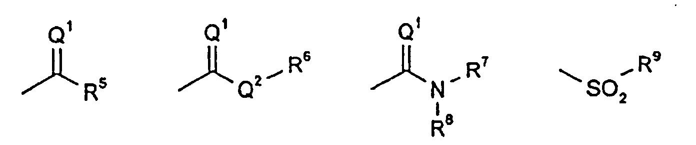 où
Q¹ et Q² représentent indépendamment l'un de l'autre l'oxygène ou le soufre,
R⁵ est l'hydrogène ou un groupe alkyle ayant 1 à 6 atomes de carbone, ou bien un groupe aryle ou un groupe arylalkyle ayant 1 à 4 atomes de carbone dans la partie alkyle, chacun portant éventuellement dans la partie aryle un substituant halogéno, cyano, nitro, alkyle, ou alkoxy ayant chacun 1 à 4 atomes de carbone,
R⁶ est un groupe alkyle ayant 1 à 6 atomes de carbone ou bien un groupe aryle ou un groupe arylalkyle ayant 1 à 4 atomes de carbone dans la partie alkyle, chacun portant éventuellement dans la partie aryle un substituant halogéno, cyano, nitro, alkyle ou alkoxy ayant chacun 1 à 4 atomes de carbone ;
R⁷ représente l'hydrogène, un groupe alkyle ayant 1 à 6 atomes de carbone ou un groupe aryle, ou arylalkyle ayant 1 à 4 atomes de carbone dans la partie alkyle, portant chacun le cas échéant dans la partie aryle un substituant halogéno, cyano, nitro, alkyle ou alkoxy ayant chacun 1 à 4 atomes de carbone,
R⁸ est un groupe alkyle ayant 1 à 6 atomes de carbone ou bien un groupe aryle ou un groupe arylalkyle ayant 1 à 4 atomes de carbone dans la partie alkyle, chacun étant éventuellement substitué dans la partie aryle par un radical halogéno, cyano, nitro, alkyle ou alkoxy ayant 1 à 4 atomes de carbone ou bien
R⁷ et R⁸ forment conjointement avec l'atome d'azote auquel ils sont liés un noyau hétérocyclique éventuellement substitué par un radical méthyle,
R⁹ représente un groupe alkyle ayant 1 à 6 atomes de carbone, un groupe dialkylamino ayant 1 à 4 atomes de carbone dans chacune des parties alkyle individuelles, un groupe hétérocyclyle lié par de l'azote ayant 3 à 7 chaînons ou un groupe aryle ou arylalkyle ayant 1 à 4 atomes de carbone dans la partie alkyle portant chacun le cas échéant dans la partie aryle un substituant halogéno, cyano, nitro, alkyle ou alkoxy ayant chacun 1 à 4 atomes de carbone,
R³ représente l'hydrogène ou un groupe alkyle ayant 1 à 4 atomes de carbone,
R⁴ est un groupe aryle, cycloalkyle ou cycloalcényle ayant 3 à 8 atomes de carbone ou un groupe hétérocyclyle à noyau de 3 à 12 atomes, portant chacun le cas échéant un ou plusieurs substituants identiques ou différents, les substituants possibles étant choisis parmi ceux qui sont énumérés ci-après :
halogéno, cyano, nitro, amino, hydroxy, formyle, carboxy, carbamoyle, thiocarbamoyle ;
alkyle, alkoxy, alkylthio, alkylsulfinyle ou alkylsulfonyle chacun linéaire ou ramifié ayant chacun 1 à 6 atomes de carbone ;
alcényle ou alcényloxy chacun linéaire ou ramifié ayant chacun 2 à 6 atomes de carbone ;
halogénalkyle, halogénalkoxy, halogénalkylthio, halogénalkylsulfinyle ou halogénalkylsulfonyle chacun linéaire ou ramifié ayant chacun 1 à 6 atomes de carbone et 1 à 13 atomes d'halogènes identiques ou différents ;
halogénalcényle ou halogénalcényloxy chacun linéaire ou ramifié ayant chacun 2 à 6 atomes de carbone et 1 à 13 atomes d'halogènes identiques ou différents ;
alkylamino, dialkylamino, alkylcarbonyle, alkylcarbonyloxy, alkoxycarbonyle, alkylsulfonyloxy, hydroximino-alkyle, ou alkoximino-alkyle chacun linéaire ou ramifié ayant chacun 1 à 6 atomes de carbone dans les parties alkyle individuelles,
alkylène ou dioxyalkylène ayant chacun deux liaisons et chacun 1 à 6 atomes de carbone, portant chacun le cas échéant un ou plusieurs substituants, identiques ou différents, halogéno et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents ;
cycloalkyle ayant 3 à 6 atomes de carbone ;
ainsi qu'aryle, aryloxy, arylthio, arylalkyle, arylalkyloxy, arylalkylthio, hétérocyclyle, hétérocyclyloxy, hétérocyclylthio, hétérocyclylalkyle, hétérocyclylalkyloxy ou hétérocyclylalkylthio portant chacun le cas échéant un ou plusieurs substituants, identiques ou différents, halogéno, cyano et/ou alkyle linéaire ou ramifié ayant 1 à 4 atomes de carbone
et/ou halogénalkyle linéaire ou ramifié ayant 1 à 4 atomes de carbone et 1 à 9 atomes d'halogènes identiques ou différents
et/ou alkoxy ou alkylthio linéaire ou ramifié ayant 1 à 4 atomes de carbone
et/ou halogénalkoxy ou halogénalkylthio linéaire ou ramifié ayant 1 à 4 atomes de carbone et 1 à 9 atomes d'halogènes identiques ou différents
et/ou alkylène ou dioxyalkylène ayant chacun deux liaisons et chacun 1 à 6 atomes de carbone portant chacun le cas échéant un ou plusieurs substituants, identiques ou différents, halogéno et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents.

10. Composés de formule (I-I)suivant la revendication 9 dans laquelle
A représente une liaison simple ou un groupe méthylène,1,1-éthylène, 1,2-éthylène, 1,1-, 1,2-, 1,3- ou 2,2-propylène, 1,1-, 1,2-, 1,3-, 1,4-, 2,2- ou 2,3-butylène ou 1,1-, 1,2- ou 1,3-(2-méthylpropylène) portant chacun le cas échéant un ou plusieurs substituants fluoro, chloro, cyano ou méthoxy identiques ou différents,
Q représente l'oxygène ou le soufre,
R¹ représente un groupe tricyclique 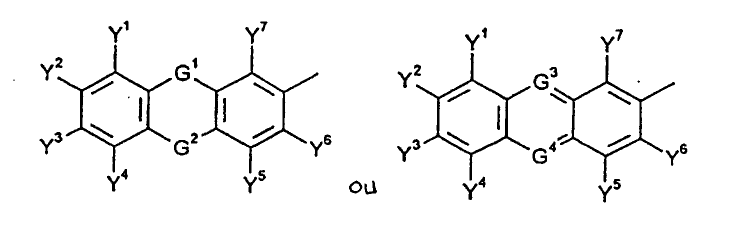 dans lequel
G¹ et G² représentent indépendamment l'un de l'autre une liaison simple, un groupe alcane-diyle, alcène-diyle, l'oxygène, le soufre, un groupe -NH-, -N(alkyl)- ou carbonyle, et
G³ et G⁴ représentent indépendamment l'un de l'autre l'azote ou un groupement 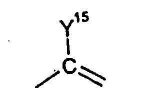
Y¹, Y², Y³, Y⁴, Y⁵, Y⁶, Y⁷ et Y⁸ représentent indépendamment les uns des autres l'hydrogène, le fluor, le chlore, le brome, un groupe cyano, nitro, méthyle, éthyle, n-propyle, isopropyle, n-butyle, isobutyle, sec.-butyle, tertiobutyle, n-pentyle, n-hexyle, n-heptyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, cyclopropyle, cyclobutyle, cyclopentyle ou cyclohexyle,
R² représente l'un des groupements suivants : 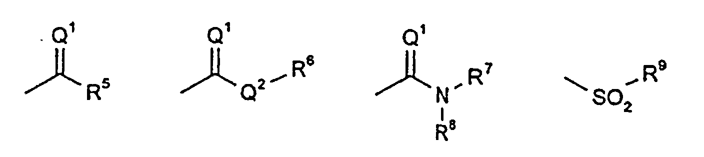 où
Q¹ et Q² représentent indépendamment l'un de l'autre, l'oxygène ou le soufre,
R⁵ est l'hydrogène ou un groupe méthyle, éthyle, n-propyle, isopropyle, n-butyle, isobutyle, sec.-butyle, tertiobutyle ou un groupe phényle, benzyle, 1-phényléthyle ou 2-phényléthyle portant chacun le cas échéant dans la partie phényle un substituant fluoro, chloro, bromo, cyano, nitro, méthyle, éthyle, n-propyle, isopropyle, n-butyle, isobutyle, sec.-butyle, tertiobutyle, méthoxy ou éthoxy,
R⁶ est un groupe méthyle, éthyle, n-propyle, isopropyle, n-butyle, isobutyle, sec.-butyle, tertiobutyle ou un groupe phényle, benzyle, 1-phényléthyle ou 2-phényléthyle portant chacun le cas échéant dans la partie phényle un substituant fluoro, chloro, bromo, cyano, nitro, méthyle, éthyle, n-propyle, isopropyle, n-butyle, isobutyle, sec.-butyle, tertiobutyle, méthoxy, ou éthoxy,
R⁷ représente l'hydrogène, un groupe méthyle, éthyle, n-propyle, isopropyle, n-butyle, isobutyle, sec.-butyle, tertiobutyle, ou un groupe phényle, benzyle, 1-phényléthyle ou 2-phényléthyle portant chacun le cas échéant dans la partie phényle un substituant fluoro, chloro, bromo, cyano, nitro, méthyle, éthyle, n-propyle, isopropyle, n-butyle, isobutyle, sec.-butyle, tertiobutyle, méthoxy ou éthoyx,
R⁸ est un groupe méthyle, éthyle, n-propyle, isopropyle, n-butyle, isobutyle, sec.-butyle, tertiobutyle ou un groupe phényle, benzyle, 1-phényléthyle ou 2-phényléthyle portant chacun le cas échéant dans la partie phényle un substituant fluoro, chloro, bromo, cyano, nitro, méthyle, éthyle, n-propyle, isopropyle, n-butyle, isobutyle, sec.-butyle, tertiobutyle, méthoxy ou éthoxy, ou bien
R⁷ et R⁸ forment conjointement avec l'atome d'azote auquel ils sont liés un noyau hétérocyclique éventuellement substitué par un radical méthyle,
R⁹ est un groupe méthyle, éthyle, n-propyle, isopropyle, n-butyle, isobutyle, sec.-butyle, tertiobutyle, diméthylamino, diéthylamino, pyrrolidine-1-yle, morpholine-N-yle, pipéridine-1-yle ou un groupe phényle, benzyle, 1-phényléthyle ou 2-phényléthyle dont chacun porte le cas échéant dans la partie phényle, un substituant fluoro, chloro, bromo, cyano, nitro, méthyle, éthyle, n-propyle, isopropyle, n-butyle, isobutyle, sec.-butyle, tertiobutyle, méthoxy ou éthoxy,
R³ représente l'hydrogène ou un groupe méthyle ou éthyle,
R⁴ est un groupe cyclopropyle, cyclobutyle, cyclopentyle, cyclohexyle, phényle, naphtyle, furyle, benzofurannyle, pyrrolyle, indolyle, thiényle, benzothiényle, oxazolyle, isoxazolyle, thiazolyle, isothiazolyle, oxadiazolyle, thiadiazolyle, pyridyle, quinolyle, pyrimidyle, pyridazinyle, pyrazinyle, oxyranyle, oxétannyle, tétrahydrofuryle, perhydropyrannyle, pyrrolidinyle, pipéridinyle ou morpholinyle portant chacun le cas échéant un à trois substituants, les substituants possibles étant choisis parmi ceux qui sont énumérés ci-après :
fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertiobutyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy; méthoxycarbonyle, éthoxycarbonyle, méthylsulfonyloxy, éthylsulfonyloxy, hydroximinométhyle, hydroximino-éthyle, méthoximinométhyle ou éthoximino-éthyle, triméthylène-(propane-1,3-diyle), tétraméthylène-(butane-1,4-diyle), méthylène-dioxy ou éthylène-dioxy ayant chacun deux liaisons et portant chacun le cas échéant un ou plusieurs substituants, identiques ou différents, fluoro, chloro, méthyle, trifluorométhyle, éthyle, n-propyle ou isopropyle,
cyclopropyle, cyclobutyle, cyclopentyle ou cyclohexyle.

11. Composés suivant la revendication 9, de formule (I-I) dans laquelle
A est une liaison simple ou un groupe méthylène, 1,1-éthylène, 1,2-éthylène, 1,1-, 1,2-, 1,3- ou 2,2-propylène, 1,1-, 1,2-, 1,3-, 1,4-, 2,2- ou 2,3-butylène ou 1,1-, 1,2- ou 1,3-(2-méthylpropylène) portant chacun le cas échéant un ou plusieurs substituants fluoro, chloro, cyano, ou méthoxy identiques ou différents,
Q représente l'oxygène ou le soufre,
R¹ est un groupe tricyclique 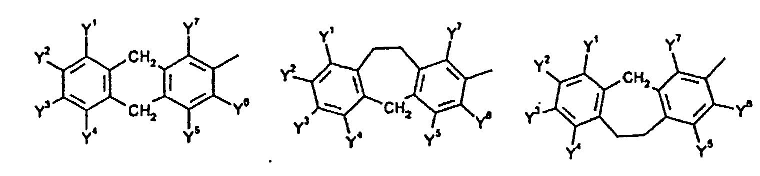 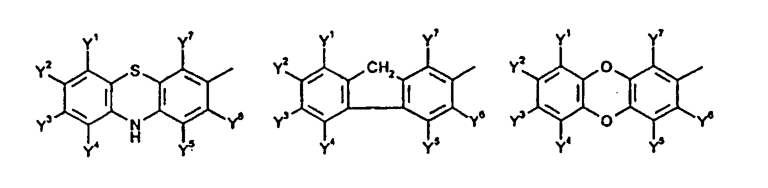 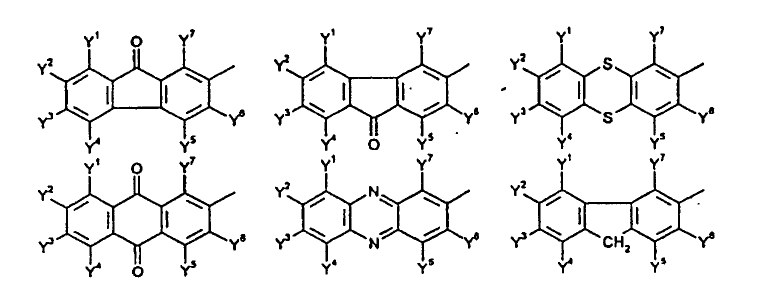 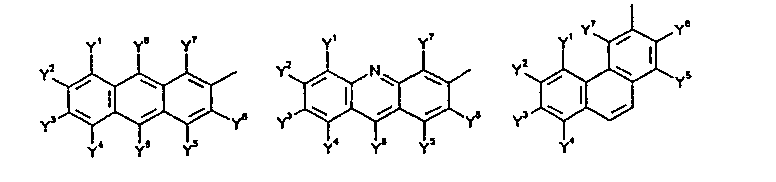 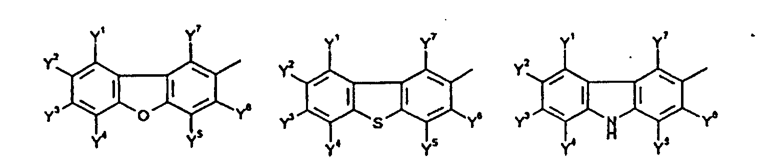 dans lequel
Y¹, Y², Y³, Y⁴, Y⁵, Y⁶, Y⁷ et Y⁸ représentent indépendamment les uns des autres l'hydrogène, le fluor, le chlore, le brome, un groupe cyano, nitro, méthyle, éthyle, n-propyle, isopropyle, n-butyle, isobutyle, sec.-butyle, tertiobutyle, n-pentyle, n-hexyle, n-heptyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, cyclopropyle, cyclobutyle, cyclopentyle ou cyclohexyle,
R² représente l'un des groupements suivants : 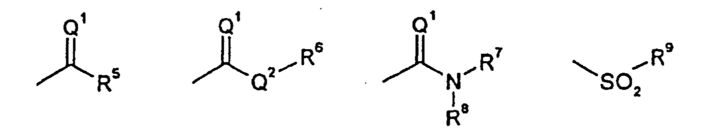 où
Q¹ et Q² représentent indépendamment l'un de l'autre l'oxygène ou le soufre,
R⁵ représente l'hydrogène ou un groupe méthyle, éthyle, n-propyle, isopropyle, n-butyle, isobutyle, sec.-butyle, tertiobutyle, ou bien un groupe phényle, benzyle, 1-phényléthyle ou 2-phényléthyle portant chacun le cas échéant dans la partie phényle un substituant fluoro, chloro, bromo, cyano, nitro, méthyle, éthyle, n-propyle, isopropyle, n-butyle, isobutyle, sec.-butyle, tertiobutyle, méthoxy ou éthoxy,
R⁶ est un groupe méthyle, éthyle, n-propyle, isopropyle, n-butyle, isobutyle, sec.-butyle, tertiobutyle ou un groupe phényle, benzyle, 1-phényléthyle ou 2-phényléthyle portant chacun le cas échéant dans la partie phényle un substituant fluoro, chloro, bromo, cyano, nitro, méthyle, éthyle, n-propyle, isopropyle, n-butyle, isobutyle, sec.-butyle, tertiobutyle, méthoxy ou éthoxy,
R⁷ représente l'hydrogène, un groupe méthyle, éthyle, n-propyle, isopropyle, n-butyle, isobutyle, sec.-butyle ou tertiobutyle ou un groupe phényle, benzyle, 1-phényléthyle ou 2-phényléthyle portant chacun le cas échéant dans la partie phényle un substituant fluoro, chloro, bromo, cyano, nitro, méthyle, éthyle, n-propyle, isopropyle, n-butyle, isobutyle, sec.-butyle, tertiobutyle, méthoxy ou éthoxy,
R⁸ est un groupe méthyle, éthyle, n-propyle, isopropyle, n-butyle, isobutyle, sec.-butyle ou tertiobutyle ou un groupe phényle, benzyle, 1-phényléthyle ou 2-phényléthyle portant chacun le cas échéant dans la partie phényle un substituant fluoro, chloro, bromo, cyano, nitro, méthyle, éthyle, n-propyle, isopropyle, n-butyle, isobutyle, sec.-butyle, tertiobutyle, méthoxy ou éthoxy, ou bien
R⁷ et R⁸ forment conjointement avec l'atome d'azote auquel ils sont liés un noyau hétérocyclique éventuellement substitué par un radical méthyle,
R⁹ est un groupe méthyle, éthyle, n-propyle, isopropyle, n-butyle, isobutyle, sec.-butyle, tertiobutyle, diméthylamino, diéthylamino, pyrrolidinyle-1-yle, morpholine-N-yle, pipéridine-1-yle ou un groupe phényle, benzyle, 1-phényléthyle ou 2-phényléthyle portant chacun le cas échéant dans la partie phényle un substituant fluoro, chloro, bromo, cyano, nitro, méthyle, éthyle, n-propyle, isopropyle, n-butyle, isobutyle, sec.-butyle, tertiobutyle, méthoxy ou éthoxy,
R³ représente l'hydrogène ou le groupe méthyle,
R⁴ est un groupe phényle, naphtyle, furyle, benzofurannyle, pyrrolyle, indolyle, thiényle, benzothiényle, oxazolyle, isoxazolyle, thiazolyle, isothiazolyle, oxadiazolyle, thiadiazolyle, pyridyle, quinolyle, pyrimidynyle, pyridazinyle, pyrazinyle, oxyranyle, oxétannyle, tétrahydrofuryle, perhydropyrannyle, pyrrolidinyle, pipéridinyle ou marpholinyle portant chacun le cas échéant un à trois substituants, les substituants possibles étant choisis parmi ceux qui sont énumérés ci-après :
fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertiobutyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximinoéthyle, méthoximino-éthyle ou éthoximino-éthyle, triméthylène-(1,3-propane-1,3-diyle), tétraméthylène-(butane-1,4-diyle), méthylène-dioxy ou éthylène-dioxy ayant chacun deux liaisons et portant chacun le cas échéant un ou plusieurs substituants, identiques ou différents, fluoro, chloro, méthyle, trifluorométhyle, éthyle, n-propyle ou isopropyle,
cyclopropyle, cyclobutyle, cyclopentyle ou cyclohexyle.

12. Composés suivant la revendication 9, de formule (I-I) dans laquelle
A représente une liaison simple ou un groupe méthylène, 1,1-éthylène, 1,2-éthylène, 1,1-propylène, 1,2-propylène, 1,3-propylène ou 2,2-propylène,
Q représente l'oxygène
R¹ est un groupe tricyclique 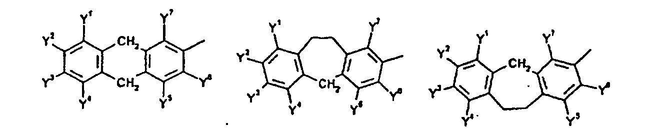 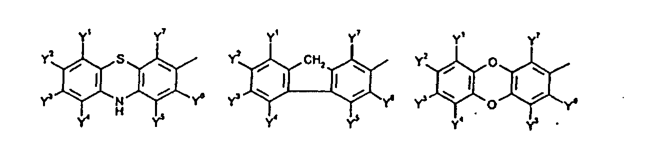 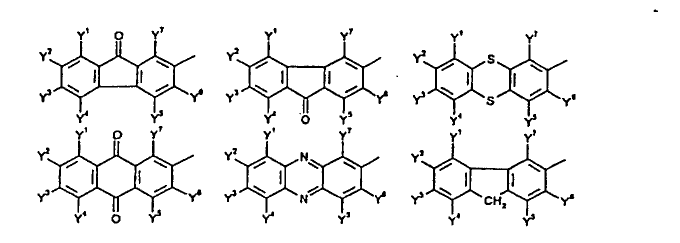 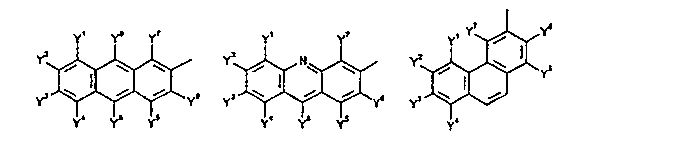 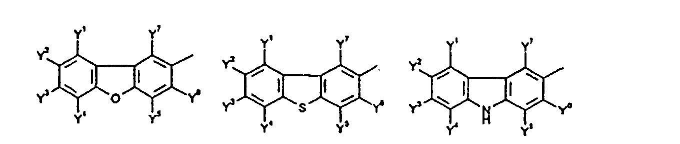 où
Y¹, Y², Y³, Y⁴, Y⁵, Y⁶, Y⁷ et Y⁸ représentent indépendamment les uns des autres l'hydrogène, le fluor, le chlore, le brome, un groupe cyano, nitro, méthyle, éthyle, n-propyle, isopropyle, n-butyle, isobutyle, sec.-butyle, tertiobutyle, n-pentyle, n-hexyle, n-heptyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, - trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, cyclopropyle, cyclobutyle, cyclopentyle ou cyclohexyle,
R² représente l'un des groupements suivants : 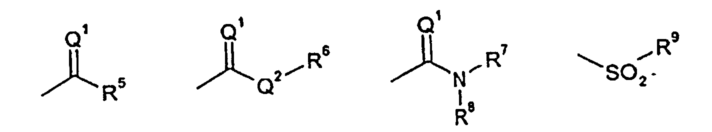 où
Q¹ et Q² représentent l'oxygène,
R⁵ est l'hydrogène ou un groupe méthyle, éthyle, n-propyle, isopropyle, n-butyle, isobutyle, sec.-butyle ou tertiobutyle, ou un groupe phényle, benzyle, 1-phényléthyle ou 2-phényléthyle portant chacun le cas échéant dans la partie phényle un substituant fluoro, chloro, bromo, cyano, nitro, méthyle, éthyle, n-propyle, isopropyle, n-butyle, isobutyle, sec.-butyle, tertiobutyle, méthoxy ou éthoxy,
R⁶ est un groupe méthyle, éthyle, n-propyle, isopropyle, n-butyle, isobutyle, sec.-butyle, tertiobutyle ou un groupe phényle, benzyle, 1-phényléthyle ou 2-phényléthyle portant chacun le cas échéant dans la partie phényle un substituant fluoro, chloro, bromo, cyano, nitro, méthyle, éthyle, n-propyle, isopropyle, n-butyle, isobutyle, sec.-butyle, tertiobutyle, méthoxy ou éthoxy,
R⁷ représente l'hydrogène, un groupe méthyle, éthyle, n-propyle, isopropyle, n-butyle, isobutyle, sec.-butyle, tertiobutyle ou un groupe phényle, benzyle, 1-phényléthyle ou 2-phényléthyle portant chacun le cas échéant dans la partie phényle un substituant fluoro, chloro, bromo, cyano, nitro, méthyle, éthyle, n-propyle, isopropyle, n-butyle, isobutyle, sec.-butyle, tertiobutyle, méthoxy ou éthoxy,
R⁸ est un groupe méthyle, éthyle, n-propyle, isopropyle, n-butyle, isobutyle, sec.-butyle, tertiobutyle ou un groupe phényle, benzyle, 1-phényléthyle ou 2-phényléthyle portant chacun le cas échéant dans la partie phényle un substituant fluoro, chloro, bromo, cyano, nitro, méthyle, éthyle, n-propyle, isopropyle, n-butyle, isobutyle, sec.-butyle, tertiobutyle, méthoxy ou éthoxy, ou bien
R⁷ et R⁸ forment conjointement avec l'atome d'azote auquel ils sont liés un noyau
R⁹ hétérocyclique éventuellement substitué par un radical méthyle, est un groupe méthyle, éthyle, n-propyle, isopropyle, n-butyle, isobutyle, sec.-butyle, tertiobutyle, diméthylamino, diéthylamino, pyrrolidinyle-1-yle, morpholine-N-yle, pipéridine-1-yle ou un groupe phényle, benzyle, 1-phényléthyle ou 2-phényléthyle portant chacun le cas échéant dans la partie phényle un substituant fluoro, chloro, bromo, cyano, nitro, méthyle, éthyle, n-propyle, isopropyle, n-butyle, isobutyle, sec.-butyle, tertiobutyle, méthoxy ou éthoxy,
R³ représente l'hydrogène ou le groupe méthyle,
R⁴ est un groupe cyclohexyle ou bien un groupe phényle, thiényle, furyle, benzofuryle, benzothiényle, pyridyle, pyrimidinyle, naphthyle, quinolyle portant chacun le cas échéant un à trois substituants, les substituants possibles étant choisis parmi ceux qui sont énumérés ci-après :
fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertiobutyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochlorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximino-éthyle, méthoximino-éthyle ou éthoximino-éthyle, triméthylène-(propane-1,3-diyle), tétraméthylène-(butane-1,4-diyle), méthylène-dioxy ou éthylène-dioxy ayant chacun deux liaisons et portant chacun le cas échéant un ou plusieurs substituants fluoro, chloro, méthyle, trifluorométhyle, éthyle, n-propyle ou isopropyle identiques ou différents.

13. Compositions pesticides, **caractérisées par** une teneur en au moins un composé de formule (I) suivant la revendication 1.

14. Procédé pour combattre des parasites, **caractérisé en ce qu'**on fait agir des composés de formule (I) suivant la revendication 1, sur des parasites et/ou sur leur milieu.

15. Utilisations de composés suivant les revendications 1 à 21 pour combattre des parasites.

16. Procédé de préparation de compositions pesticides, **caractérisé en ce qu'**on mélange des composés suivants les revendications 1 à 21 avec des diluants et/ou des agents tensioactifs.
